# EUROPEAN PATENT SPECIFICATION

(11) **EP 2 871 187 B1**
(45) Date of publication and mention of the grant of the patent: **20.09.2017**
(21) Application number: 12880708.8
(22) Date of filing: 04.07.2012
(51) Int. Cl.: C07D 493/12, C07D 493/04, A61K 31/366, A61P 35/00, C07D 311/74, C07D 311/58, C07D 493/14

(54) **NOVEL COMPOUND OR PHARMACEUTICALLY ACCEPTABLE SALT THEREOF, AND PHARMACEUTICAL COMPOSITION CONTAINING SAME AS ACTIVE INGREDIENT**
NEUARTIGE VERBINDUNG ODER PHARMAZEUTISCH UNBEDENKLICHES SALZ DAVON UND PHARMAZEUTISCHE ZUSAMMENSETZUNG DAMIT ALS WIRKSTOFF
NOUVEAU COMPOSÉ OU SEL PHARMACEUTIQUEMENT ACCEPTABLE DE CELUI-CI, ET COMPOSITION PHARMACEUTIQUE LE CONTENANT À TITRE DE PRINCIPE ACTIF

(43) Date of publication of application: 13.05.2015
(73) Proprietor: SNU R&DB Foundation, Seoul 151-742 (KR)
(72) Inventor: SUH, Young-Ger, Seoul 137-070 (KR); CHANG, Dong-Jo, Seoul 137-836 (KR); AN, Hongchan, Seoul 156-766 (KR); KIM, Kyu-Won, Seoul 138-890 (KR); KIM, Young-Myeong, Chuncheon-si Gangwon-do 200-701 (KR); LEE, Ho-Young, Seoul 151-742 (KR)
(74) Representative: Abel & Imray
(86) International application number: PCT/KR2012/005309
(87) International publication number: WO 2014/007412

(56) References cited:
- US-B2- 7 410 998
- SUCHADA CHANTRAPROMMA ET AL: "7a- O -Methyldeguelol", ACTA CRYSTALLOGRAPHICA SECTION E STRUCTURE REPORTS ONLINE, vol. 61, no. 8, 6 July 2005 (2005-07-06), pages o2340-o2342, XP055177308, DOI: 10.1107/S1600536805020325
- YENESEW A ET AL: "7a-O-methyldeguelol, a modified rotenoid with an open ring-C, from the roots of Derris trifoliata", PHYTOCHEMISTRY, PERGAMON PRESS, GB, vol. 66, no. 6, 1 March 2005 (2005-03-01), pages 653-657, XP027726513, ISSN: 0031-9422 [retrieved on 2005-03-01]
- STEFAN J. PASTINE ET AL: "Concise Synthesis of the Chemopreventitive Agent ( )-Deguelin via a Key 6-Endo Hydroarylation", ORGANIC LETTERS, vol. 5, no. 22, 1 October 2003 (2003-10-01), pages 4053-4055, XP055177380, ISSN: 1523-7060, DOI: 10.1021/ol035419j
- PATHAK V P ET AL: "Glabrachalcone, a chromenochalcone from Pongamia glabra seeds", PHYTOCHEMISTRY, PERGAMON PRESS, GB, vol. 22, no. 5, 1 January 1983 (1983-01-01), pages 1303-1304, XP026605625, ISSN: 0031-9422, DOI: 10.1016/0031-9422(83)80254-4 [retrieved on 1983-01-01]
- NICOLAOU K C ET AL: "Natural Product-like Combinatorial Libraries Based on Privileged Structures. 1. General Principles and Solid-Phase Synthesis of Benzopyrans", JOURNAL OF THE AMERICAN CHEMICAL SOCIETY, AMERICAN CHEMICAL SOCIETY, US, vol. 122, no. 41, 1 January 2000 (2000-01-01), pages 9939-9953, XP002348800, ISSN: 0002-7863, DOI: 10.1021/JA002033K
- NICOLAOU K C ET AL: "Combinatorial synthesis of novel and potent inhibitors of NADH:ubiquinone oxidoreductase", CHEMISTRY AND BIOLOGY, CURRENT BIOLOGY, LONDON, GB, vol. 7, no. 12, 1 December 2000 (2000-12-01), pages 979-992, XP002463844, ISSN: 1074-5521, DOI: 10.1016/S1074-5521(00)00047-8
- DATABASE CA [Online] CHEMICAL ABSTRACTS SERVICE, COLUMBUS, OHIO, US; VON GRAFENSTEIN, SUSANNE ET AL: "Identification of Novel Liver X Receptor Activators by Structure-Based Modeling", XP002743350, retrieved from STN Database accession no. 2012:525333
- KIM, W.-Y. ET AL.: 'A novel derivative of the natural agent deguelin for cancer chemoprevention and therapy.' CANCER PREVENTION RESEARCH. vol. 1, 2008, pages 577 - 587, XP055170109
- GARCIA, J. ET AL.: 'Synthesis of deguelin-biotin conjugates and investigation into deguelin's interactions.' BIOORGANIC & MEDICINAL CHEMISTRY. vol. 20, January 2012, pages 672 - 680, XP028442303
- FANG, N. ET AL.: 'Anticancer action of cube insecticide: correlation for rotenoid constituents between inhibition ofNADH: ubiquinone oxidoreductase and induced ornithine decarboxylase activities.' PROC. NATL. ACAD. SCI. USA. vol. 95, 1998, pages 3380 - 3384, XP055170117
- GRANADIOS-COVARRUBIAS, E.H. ET AL.: 'Protected cyanohydrins in the synthesis of rotenoids: (+-)-munduserone and (+-)-cis-12a-hydroxymunduserone.' J. ORG. CHEM. vol. 74, 2009, pages 5097 - 5099, XP055170109
- FANG, Y-T. ET AL.: 'Total synthesis of (+-)-glyfavanone by a rigid quaternary ammonium salt.' JOURNAL OF THE CHINESE CHEMICAL SOCIETY. vol. 54, 2007, pages 817 - 822, XP055170374
- JO DONG HYUN ET AL: "Hypoxia-mediated retinal neovascularization and vascular leakage in diabetic retina is suppressed by HIF-1[alpha] destabilization by SH-1242 and SH-1280, novel hsp90 inhibi", JOURNAL OF MOLECULAR MEDICINE, SPRINGER VERLAG, DE, vol. 92, no. 10, 31 May 2014 (2014-05-31), pages 1083-1092, XP035407201, ISSN: 0946-2716, DOI: 10.1007/S00109-014-1168-8 [retrieved on 2014-05-31]

## Description

### BACKGROUND OF THE INVENTION

### 1. Field of the Invention

The present invention relates to a compound inhibiting Hsp90 and a pharmaceutical composition comprising the same as an active ingredient.

### 2. Description of the Related Art

Molecular chaperone such as heat-shock protein families (HSPs) is a protein that can activate nascent proteins and help refolding or decomposing a damaged protein by regulating the folding of a client protein through its activity of changing the ATP dependent structure. A client protein avoids aggregation by binding with such molecular chaperone. This binding can be a help for the intracellular deposition by membrane translocation of the client protein.

It has been known that the molecular chaperone functions of Hsp90, one of the heat-shock protein families, are necessary for the stabilization and activation of various client proteins involved in cell signaling pathway. The amount of Hsp90 takes 1 ∼ 2% of the total intracellular proteins in the normal condition in the absence of external stimuli, but it becomes double the amount in the presence of external stimuli. Cancer inducible mutation of a client protein requires stronger and intensive Hsp90 functions, resulting in the over-expression of Hsp90. The over-expressed Hsp90 is commonly found in cancers [Bagatell, R.; Whitesell, L. Altered Hsp90 function in cancer: A unique therapeutic opportunity. Mol. Cancer Ther. 2004, 3, 1021-1030].

The Hsp90 client protein including ErbB2, Src, Met tyrosine kinase, MEK 1/2 (mitogen-activated protein kinase kinase), Akt, Raf-1, cyclin-dependent serine kinases, steroid hormone receptor, telomerase, metalloprotein-2 (MMP-2), and HIF-1α (hypoxia-inducible factor-1α) is found in various signaling pathways involved in cell survival, proliferation, invasion, metastasis, and angiogenesis, etc, and is contributed to the malignant phenotype [Eustace, B. K.; Sakurai, T.; Stewart, J. K.; Yimlamai, D.; Unger, C.; Zehetmeier, C.; Lain, B.; Torella, C.; Henning, S. W.; Beste, G.; Scroggins, B. T.; Neckers, L.; Ilag, L. L.; Jay, D. G. Functional proteomic screens reveal an essential extracellular role for hsp90 alpha in cancer cell invasiveness. Nat. Cell Biol. 2004, 6, 507-514].

In particular, HIF-1α, together with HIF-1β, is a subunit that composes HIF (hydroxia-inducible factor) and induces the expression of VEGF (vascular endothelial growth factor), the oxygen-instable transcriptional factor and one of the angiogenesis-regulating proteins, in order to control angiogenesis in the aspect of vascular destruction and vascular dysfunction. Angiogenesis is a process of generating a new blood vessel, which is necessary for the repair, regeneration, and development of blood vessels or metabolically activated tissues.

However, the pathological angiogenesis not only plays an important role in the growth and expansion of cancer via metastasis but also induces hemorrhage, endoleak, and tissue destruction by abnormally fast angiogenesis. So, the pathological angiogenesis causes not just cancer but also various angiogenesis-dependent diseases including diabetic retinopathy and age-related macular degeneration, and is also involved in chronic infectious diseases including psoriasis and rheumatoid arthritis.

Therefore, the inhibition of Hsp90 is expected to be a efficient treatment method for angiogenesis related diseases and thus the Hsp90 inhibitor can be a potential chemotherapeutic agent for angiogenesis related diseases [Eccles, S.; Massey, A.; Raynaud, F.; Sharp, S.; Box, G.; Valenti, M.; Patterson, L.; de Haven Brandon, A.; Gowan, S.; Boxall, F. NVP-AUY922: a heat shock protein 90 inhibitor active against xenograft tumor growth, angiogenesis, and metastasis. Cancer Res. 2008, 68, 2850].

Hsp90 exists mainly as a homodimer, which is composed of N-terminal, intermediate region, and C-terminal. Particularly, N-terminal contains adenine nucleotide-binding pocket in which a specific structural motif known as Bergerat fold is included, by which Hsp90 can harbor ATP-binding site, different from other kinases or Hsp70. This structural specificity provides a potential for the development of a selective Hsp90 inhibitor. In fact, followings are the examples of Hsp90 inhibitors so far.

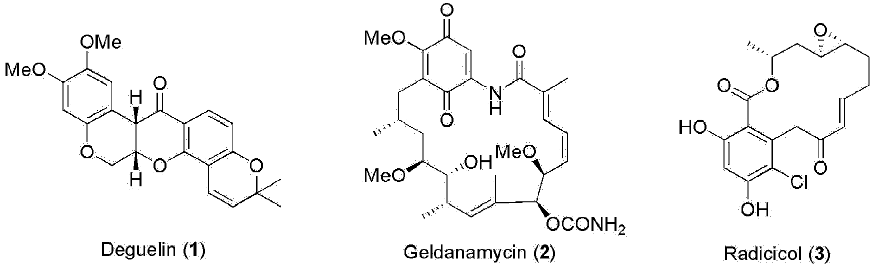

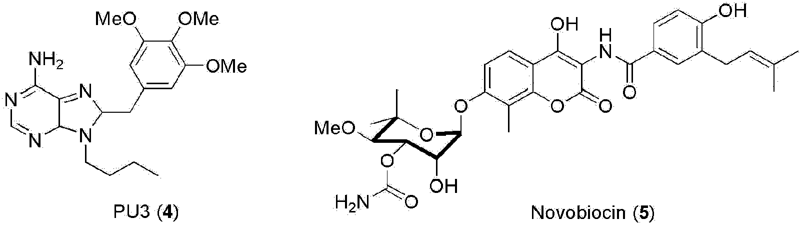

Most of Hsp90 inhibitors, which are exemplified by geldanamycin(2) and its derivative 17-AAG (17-arylamino-17-dimethoxy geldanamycin) having less toxicity, radicicol(3) and the more stable oxime derivative thereof, and the synthetic derivative PU3 (4), are known to interact with ATP-binding pocket in N-terminal of Hsp90. In the meantime, the natural antibiotics novobiocin(5), known as a DNA gyrase inhibitor, displays an efficient Hsp90 inhibiting activity by interacting with ATP-binding pocket in C-terminal of Hsp90. Such Hsp90 inhibitors accelerate the degradation of various cancer inducing Hsp90 client proteins, so that they can bring a significant prevention effect in various cancer cell lines of preclinical models. Some of the Hsp90 inhibitors including 17-AAG are in clinical phase.

Deguelin(1) [Clark, E., A relation between rotenone, deguelin and tephrosin. 1931; Vol. 73, pp 17-18.] is a rotenoid compound isolated from Africa origin *Mundulea sericea,* which is known to have the effect of preventing the development of lung cancer induced by tobacco carcinogens by blocking Akt activation and also to display apoptotic effect and anti-angiogenesis effect in various transformed cell lines and cancer cell lines [Lee, H. Molecular mechanisms of deguelin-induced apoptosis in transformed human bronchial epithelial cells. Biochem. Pharmacol. 2004, 68, 1119-1124].

Even though the potential of deguelin as an anticancer agent or an anti-angiogenesis agent has been confirmed, the toxicity, low solubility, and chemical instability of deguelin draw a limit in its use as a drug. US7410998 discloses chromene derivatives for inhibiting the growth of cancer cells. The present inventors succeeded in synthesizing a compound showing the activity of deguelin but having less toxicity than deguelin and improved physicochemical properties and thereafter confirmed the Hsp90 inhibiting effect, anti-angiogenesis effect, and cytotoxicity of the compound, leading to the completion of this invention.

### SUMMARY OF THE INVENTION

It is an object of the present invention to provide a compound inhibiting Hsp90.

It is described to provide a pharmaceutical composition for treating cancer comprising the compound as an active ingredient.

It is also an object of the present invention to provide a pharmaceutical composition for treating diabetic retinopathy comprising the compound as an active ingredient.

It is further an object of the present invention to provide a pharmaceutical composition for treating rheumatoid arthritis comprising the compound as an active ingredient. The present description provides the compound represented by formula 1 or the pharmaceutically acceptable salt thereof:

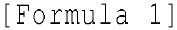

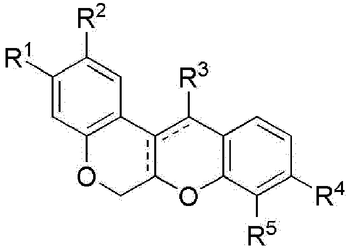

(In formula 1, R¹ ∼ R⁵ are as defined in this description).

The present invention provides the compound represented by formula 2 or the pharmaceutically acceptable salt thereof for use in the prevention or treatment of diabetic retinopathy or rheumatoid arthritis:

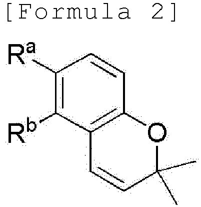

(In formula 2, R^{a} and R^{b} are as defined by the scope of the claims. The present description also provides a pharmaceutical composition for preventing or treating cancer comprising the compound of formula 1 and/or the compound of formula 2 or the pharmaceutically acceptable salt thereof as an active ingredient.

The present invention also provides a pharmaceutical composition for preventing or treating diabetic retinopathy comprising the compound of formula 2 or the pharmaceutically acceptable salt thereof as an active ingredient.

In addition, the present invention provides a pharmaceutical composition for preventing or treating rheumatoid arthritis comprising the compound of formula 2 or the pharmaceutically acceptable salt thereof as an active ingredient.

### ADVANTAGEOUS EFFECT

The compounds represented by formula 2 of the present invention suppress the expression of Hsp90 so that they can inhibit the accumulation of HIF-1α, the Hsp90 client protein, and also efficiently inhibit the activation of VEGF. In addition, these compounds display low toxicity, so that they can be effectively used as an active ingredient of an anti-cancer agent, a diabetic retinopathy treating agent, and an anti-arthritic agent.

### BRIEF DESCRIPTION OF THE DRAWINGS

The application of the preferred embodiments of the present invention is best understood with reference to the accompanying drawings, wherein:
Figure 1 is a diagram illustrating the result of Western blotting performed to measure the activity of the compounds of the present invention to inhibit the accumulation of HIF-1α.
Figure 2 is a photograph illustrating the effect of the compound 53 of the present invention on angiogenesis.
Figure 3 is a photograph illustrating the effect of the compound 69 of the present invention on angiogenesis.
Figure 4 is a photograph illustrating the effect of the compound 72 of the present invention on angiogenesis.
Figure 5 is a graph illustrating the inhibitory effect of the compound of the present invention on the proliferation of vascular endothelial cells.
Figure 6 is a diagram illustrating the inhibitory effect of the compound of the present invention on the migration of vascular endothelial cells.
Figure 7 is a graph illustrating the inhibitory effect of the compound of the present invention on the migration of vascular endothelial cells.
Figure 8 is a diagram illustrating the inhibitory effect of the compound of the present invention on the tube formation of vascular endothelial cells.
Figure 9 is a graph illustrating the cell survival rate (%) obtained by MTT assay performed to investigate the cytotoxicity of the compound of the present invention.

### DESCRIPTION OF THE PREFERRED EMBODIMENTS

Hereinafter, the present invention is described in detail.

The present description provides the compound represented by formula 1 or the pharmaceutically acceptable salt thereof:

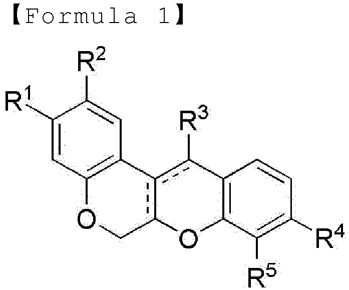

In formula 1,
R¹ and R² are independently -H, -OH, C₁₋₄ straight or branched alkyl, or C₁₋₄ alkoxy;
R³ is -H, -OH, =O, -OR⁶, or =N-O-R⁷;
R⁴ is -H, -OH, C₁₋₄ alkoxy, acetate, benzyloxy, or phenylmethoxy;
R⁵ is C₁₋₆ straight or branched alkyl or alkenyl;
Or R⁴ and R⁵ can form 5-8 atom saturated or unsaturated heterocycle containing one or more oxygen (O) atoms along with carbon atoms which are attached to the same, wherein the heterocycle can be substituted with one or more substituents independently selected from the group consisting of - OH and C₁₋₄ straight or branched alkyl;
R⁶ is C₁₋₆ straight or branched alkyl or alkenyl, C₅₋₈ arylalkyl, 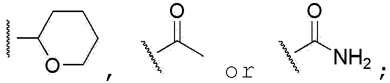 R⁷ is -H, C₁₋₄ straight or branched alkyl, or C₅₋₈ arylalkyl; 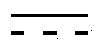 is single bond or double bond; and

The compound of formula 1 is not the compound in which R¹ and R² are -OMe, R³ is =O, and the ring formed by R⁴ and R⁵ is

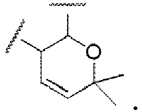

Preferably,
R¹ and R² are independently -OH or -OMe;
R³ is -H, -OH, =O, -OR⁶ or =N-O-R⁷;
R⁴ is -OH, C₁₋₃ alkoxy, acetate or phenylmethoxy;
R⁵ is C₁₋₄ straight or branched alkyl or alkenyl;

Or R⁴ and R⁵ can form 5-6 atom saturated or unsaturated heterocycle containing one or more oxygen (O) atoms along with carbon atoms which are attached to the same, wherein the heterocycle can be substituted with -OH or C₁₋₂ alkyl;
R⁶ is C₁₋₃ straight or branched alkyl or alkenyl, C₅₋₆ arylalkyl, 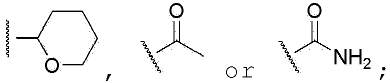 and
R⁷ is -H, C₁₋₃ straight or branched alkyl, or C₅₋₆ arylalkyl.

More preferably,
R³ is -H, -OH, =O, methoxy, ethoxy, propoxy, 2-prophenoxy, benzyloxy, 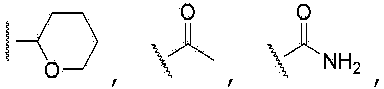 =N-OH, =N-OMe or =N-OBn,
R⁴ is -OH, methoxy, 2-prophenoxy, benzyloxy or acetate,
R⁵ is

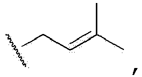

Or R⁴ and R⁵ can form 6 atom heterocycle containing one or more oxygen (O) atoms, wherein the heterocycle can be substituted with -OH or C₁₋₂ alkyl. The heterocycle formed by R⁴ and R⁵ is

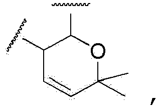

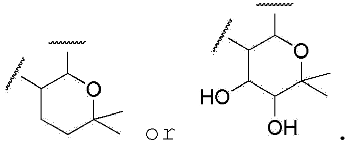

Specific examples of the compound represented by formula 1 of the present invention are as follows:
(6a*S*,12a*S*)-9-hydroxy-2,3-dimethoxy-8-(3-methyl-2-butenyl)-6a,12a-dihydrochromeno[3,4-*b*]chromen-12(6*H*)-one,
(7a*S*,13a*S*)-9-hydroxy-13,13a-dihydro-10-methoxy-3,3-dimethyl-3*H*-chromeno[3,4-*b*]pyrano[2,3-*h*]chromen-7(7a*H*)-one,
(7a*S*,13a*S*)-10-hydroxy-13,13a-dihydro-9-methoxy-3,3-dimethyl-3*H*-chromeno[3,4-*b*]pyrano[2,3-*h*]chromen-7(7a*H*)-one,
(7a*S*,13a*S*)-13,13a-dihydro-9,10-dihydroxy-3,3-dimethyl-3*H*-chromeno[3,4-*b*]pyrano[2,3-*h*]chromen-7(7a*H*)-one,
9,10-dimethoxy-3,3-dimethyl-3*H*-chromeno[3,4-*b*]pyrano[2, 3-*h*]chromen-7-(13*H*)-one,
(7a*S*,13a*S*)-9,10-dimethoxy-3,3-dimethyl-7,7a,13,13a-tetrahydro-3*H*-chromeno[3,4-*b*]pyrano[2,3-*h*]chromene,
(7*S*,7a*R*,3a*S*)-9,10-dimethoxy-3,3-dimethyl-7-ethoxy-7,7a,13,13a-tetrahydro-3*H*-chromeno[3,4-*b*]pyrano[2,3-*h*]chromene,
(7*S*,7a*R*,3a*S*)-9,10-dimethoxy-3,3-dimethyl-7-propoxy-7,7a,13,13a-tetrahydro-3*H*-chromeno[3,4-*b*]pyrano[2,3-*h*]chromene,
(7*S*,7a*R*,3a*S*)-7-benzyloxy-9,10-dimethoxy-3,3-dimethyl-7,7a,13,13a-tetrahydro-3*H*-chromeno[3,4-*b*]pyrano[2,3-*h*]chromene,
(7*S*,7a*S*,13a*S*)-9,10-dimethoxy-3,3-dimethyl-7-(tetrahydro-2*H*-pyran-2-yloxy)-7,7a,13,13a-tetrahydro-3*H*-chromeno[3,4-*b*]pyrano[2,3-*h*]chromene,
(7*S*,7a*S*,13a*S*)-9,10-dimethoxy-3,3-dimethyl-7,7a,13,13a-tetrahydro-3*H*-chromeno[3,4-*b*]pyrano[2,3,*h*]chromen-7-yl acetate,
(13a*S*)-9,10-dimethoxy-3,3-dimethyl-13,13a-dihydro-3*H*-chromeno[3,4-*b*]pyrano[2,3-*h*]chromene,
(7a*R*,13a*S*)-9,10-dimethoxy-3,3-dimethyl-13,13a-dihydro-3*H*-chromeno[3,4-*b*]pyrano[2,3-*h*]chromen-7(7a*H*)-one oxime,
(7a*R*,13a*S*)-9,10-dimethoxy-3,3-dimethyl-13,13a-dihydro-3*H*-chromeno[3,4-*b*]pyrano[2,3-*h*]chromen-7(7a*H*)-one *O*-methyloxime,
(7a*R*,13a*S*)-9,10-dimethoxy-3,3-dimethyl-13,13a-dihydro-3*H*-chromeno[3,4-*b*]pyrano[2,3-*h*]chromen-7(7a*H*)-one *O*-benzyloxime,
(7a*S*,13a*S*)-1,2-dihydroxy-9,10-dimethoxy-3,3-dimethyl-2,3,13,13a-tetrahydro-1*H*-chromeno[3,4-*b*]pyrano[2,3-*h*]chromen-7(7a*H*)-one,
2,3,9-trimethoxy-8-(3-methyl-but-2-enyl)-6a,12a-dihydro-6*H*-chromeno[3,4-*b*]chromen-12-one,
9-aryloxy-2,3-dimethoxy-8-(3-methyl-but-2-enyl)-6a,12a-dihydro-6*H*-chromeno[3,4-*b*]chromen-12-one,
9-benzyloxy-2,3-dimethoxy-8-(3-methyl-but-2-enyl)-6a,12a-dihydro-6*H*-chromeno[3,4-*b*]chromen-12-one,
acetic acid 2,3-dimethoxy-8-(3-methyl-but-2-enyl)-12-oxo-6,6a,12,12a-tetrahydrochromeno[3,4-*b*]chromen-9-yl ester, and
(7*S*,7a*R*,3a*S*)-9,10-dimethoxy-3,3-dimethyl-7-(prop-2-en-oxy)-7,7a,13,13a-tetrahydro-3H-chromeno[3,4-*b*]pyrano[2,3-*h*]chromene.

The present invention provides the compound represented by formula 2 or the pharmaceutically acceptable salt thereof for use in the prevention or treatment of diabetic retinopathy or rheumatoid arthritis:

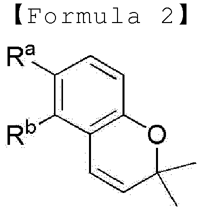

In formula 2 the definitions correspond to the ones given in the claims.
R^{a} is 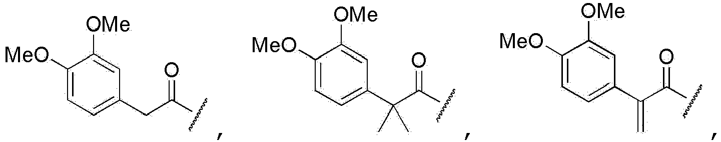 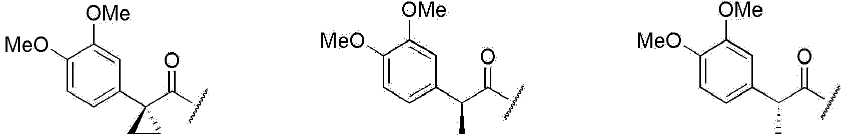 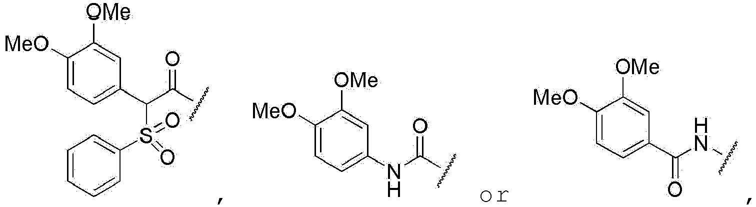 R^{b} is -H, -OH or methoxy.

Specific examples of the compound represented by formula 2 of the present invention are as follows: (if encompassed by the scope of the claims).
(3*S*)-3-(3,4-dimethoxyphenyl)-8,8-dimethyl-2,3-dihydro-4*H*,8*H*-pyrano[2,3-*f*]chromen-4-one,
(6,7-dimethoxychroman-4-yl)(2,2-dimethyl-2*H-*chromen-6-yl)methanone,
2-(3,4-dimethoxyphenyl)-1-(2,2-dimethyl-2*H-*chromen-6-yl)ethanone,
2-(3,4-dimethoxyphenyl)-1-(5-methoxy-2,2-dimethyl-2*H*-chromen-6-yl)ethanone,
2-(3,4-dimethoxyphenyl-1-(5-methoxy-2,2-dimethyl-2*H*-chromen-6-yl)propan-1-one
2-(3,4-dimethoxyphenyl)-1-(5-methoxy-2,2-dimethyl-2*H*-chromen-6-yl)-2-methylpropan-1-one,
2-(3,4-dimethoxyphenyl)-1-(5-methoxy-2,2-dimethyl-2*H*-chromen-6-yl)prop-2-en-1-one,
1-(3,4-dimethoxyphenyl)cyclopropyl)(5-methoxy-2,2-dimethyl-2*H*-chromen-6-yl)methanone,
(*S*)-2-(3,4-dimethoxyphenyl)-1-(5-methoxy-2,2-dimethyl-2*H*-chromen-6-yl)propan-1-one,
(*R*)-2-(3,4-dimethoxyphenyl)-1-(5-methoxy-2,2-dimethyl-2*H*-chromen-6-yl)propan-1-one,
3-(3,4-dimethoxyphenyl)-8,8-dimethyl-4*H*, 8*H-*pyrano[2,3-*f*]chromen-4-one,
6,7-dimethoxy-2*H*-chromen-4-yl(2,2-dimethyl-2*H-*chromen-6-yl)methanone,
6,7-dimethoxy-2,2-dimethyl-2*H*-chromen-4-yl)(4-methoxy-2,2-dimethyl-2*H*-chromen-6-yl)methanone,
6,7-dimethoxy-2,2-dimethyl-2*H*-chromen-4-yl)(2,2-dimethyl-2*H*-chromen-6-yl)methanone,
6,7-dimethoxy-2*H*-chromen-4-yl)(4-methoxy-2,2-dimethyl-2*H*-chromen-6-yl)methanone,
2-(3,4-dimethoxyphenyl)-1-(5-hydroxy-2,2-dimethyl-2*H*-chromen-6-yl)-2-(phenylsulfonyl)ethanone,
(3,4-dimethoxyphenyl)(2,2-dimethyl-2*H*-chromen-6-yl)methanone,
(*E*)-1-(3,4-dimethoxyphenyl)-3-(2,2-dimethyl-2*H-*chromen-6-yl)prop-2-en-1-one,
(*E*)-3-(3,4-dimethoxyphenyl)-1-(5-hydroxy-2,2-dimethyl-2*H*-chromen-6-yl)prop-2-en-1-one,
(*E*)-3-(3,4-dimethoxyphenyl)-1-(2,2-dimethyl-2*H-*chromen-6-yl)prop-2-en-1-one,
(*E*)-1-(5-hydroxy-2,2-dimethyl-2*H*-chromen-6-yl)-3-(2,4,5-trimethoxyphenyl)prop-2-en-1-one,
(*E*)-3-(3,4-dimethoxyphenyl)-1-(5-methoxy-2,2-dimethyl-2*H*-chromen-6-yl)prop-2-en-1-one,
(*E*)-1-(5-methoxy-2,2-dimethyl-2*H*-chromen-6-yl)-3-(2,4,5-trimethoxyphenyl)prop-2-en-1-one,
2-(3,4-dimethoxyphenyl)-8,8-dimethyl-4*H*,8*H-*pyrano[2,3-*f*]chromen-4-one,
2-(3,4-dimethoxyphenyl)-*N*-(5-methoxy-2,2-dimethyl-2*H*-chromen-6-yl)acet-amide,
*N*-(3,4-dimethoxybenzyl)-2,2-dimethyl-2*H*-chromen-6-carboxamide,
*N*-(3,4-dimethoxyphenyl)-2,2-dimethyl-2*H*-chromen-6-carboxamide,
*N*-(2,2-dimethyl-2*H*-chromen-6-yl)-3,4-dimethoxybenzamide,
(*R*)-2-(3,4-dimethoxyphenyl)-1-(2,2-dimethyl-2*H-*chromen-6-yl)propan-1-one,
(*S*)-2-(3,4-dimethoxyphenyl)-1-(2,2-dimethyl-2*H-*chromen-6-yl)propan-1-one.

Specific examples of the compounds represented by formula 2 of the present invention are presented in Table 1.

### (Only examples covered by the scope of the claims form part of the invention)

**[Table 1]**

| Compound | Structure |
|---|---|
| Deguelin | 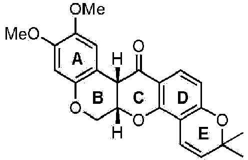 |
| Example 1 (Compound 8) | 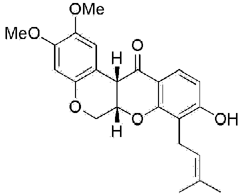 |
| Example 2 (Compound 9) | 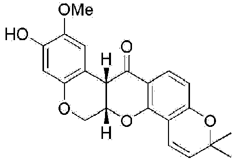 |
| Example 3 (Compound 10) | 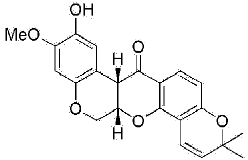 |
| Example 4 (Compound 11) | 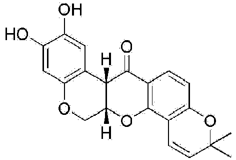 |
| Example 5 (Compound 12) | 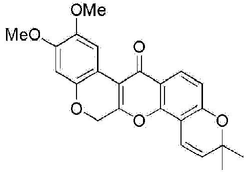 |
| Example 7 (Compound 13) | 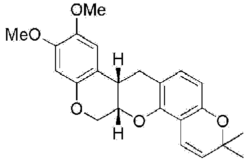 |
| Example 9 (Compound 16) | 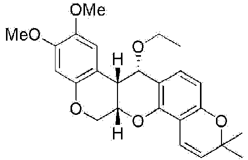 |
| Example 10 (Compound 17) | 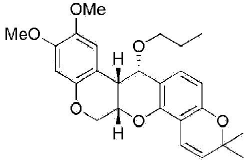 |
| Example 11 (Compound 18) | 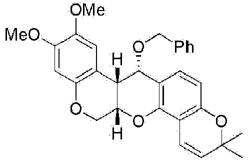 |
| Example 12 (Compound 19) | 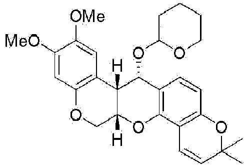 |
| Example 13 (Compound 20) | 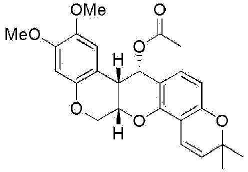 |
| Example 15 (Compound 22) | 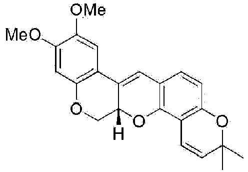 |
| Example 16 (Compound 23) | 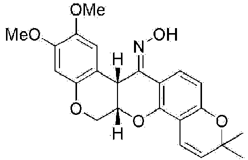 |
| Example 17 (Compound 24) | 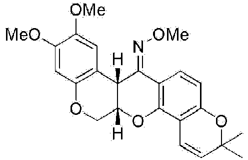 |
| Example 18 (Compound 25) | 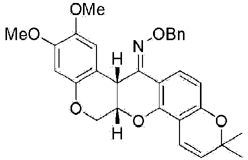 |
| Example 20 (Compound 27) | 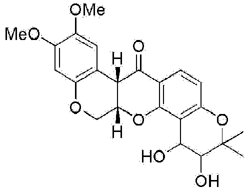 |
| Example 21 (Compound 28) | 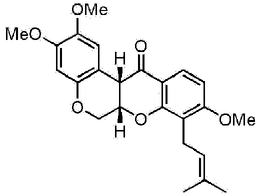 |
| Example 22 (Compound 29) | 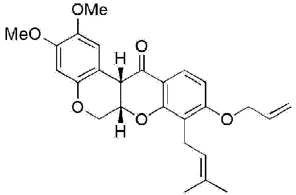 |
| Example 23 (Compound 30) | 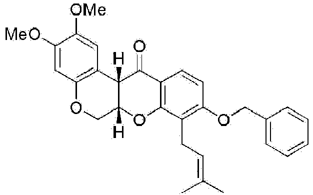 |
| Example 24 (Compound 31) | 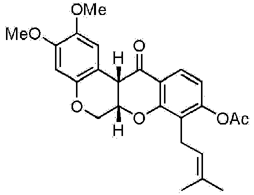 |
| Example 25 (Compound 37) | 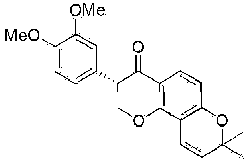 |
| Example 26 (Compound 45) | 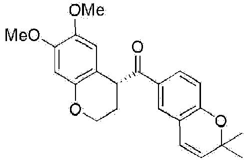 |
| Example 27 (Compound 53) | 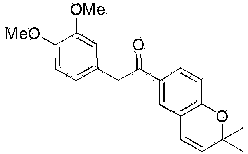 |
| Example 28 (Compound 54) | 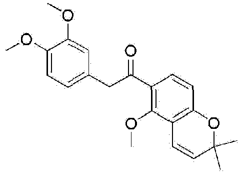 |
| Example 29 (Compound 56) | 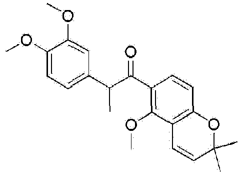 |
| Example 30 (Compound 57) | 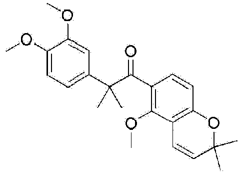 |
| Example 31 (Compound 58) | 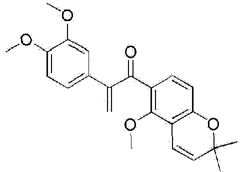 |
| Example 32 (Compound 59) | 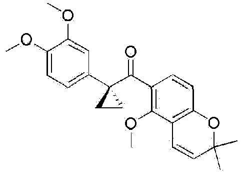 |
| Example 33 (Compound 69) | 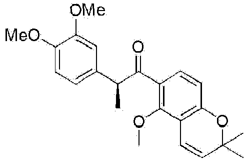 |
| Example 34 (Compound 72) | 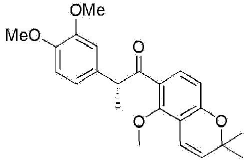 |
| Example 35 (Compound 80) | 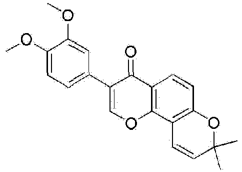 |
| Example 36 (Compound 81) | 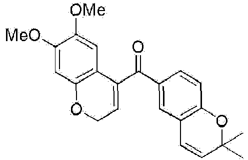 |
| Example 37 (Compound 82) | 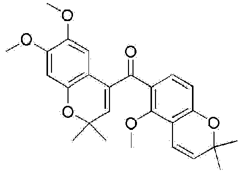 |
| Example 38 (Compound 83) | 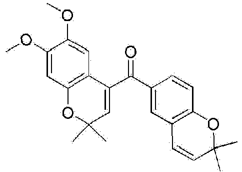 |
| Example 39 (Compound 84) | 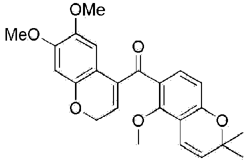 |
| Example 40 (Compound 86) | 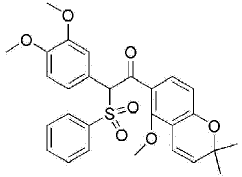 |
| Example 41 (Compound 87) | 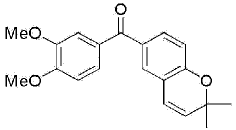 |
| Example 42 (Compound 88) | 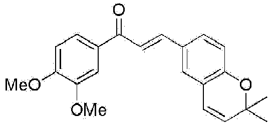 |
| Example 43 (Compound 89) | 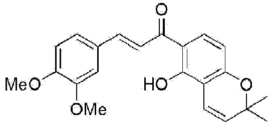 |
| Example 44 (Compound 90) | 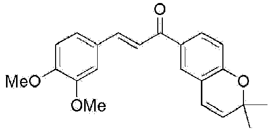 |
| Example 45 (Compound 91) | 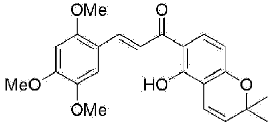 |
| Example 46 (Compound 92) | 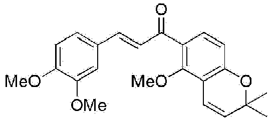 |
| Example 47 (Compound 93) | 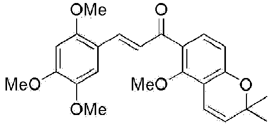 |
| Example 48 (Compound 94) | 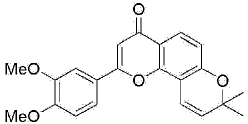 |
| Example 49 (Compound 95) | 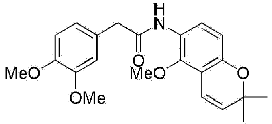 |
| Example 50 (Compound 96) | 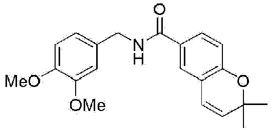 |
| Example 51 (Compound 97) | 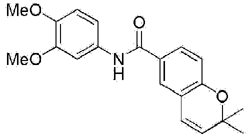 |
| Example 52 (Compound 98) | 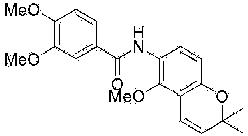 |
| Example 53 (Compound 99) | 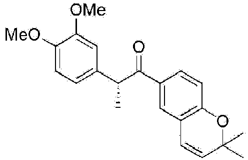 |
| Example 54 (Compound 100) | 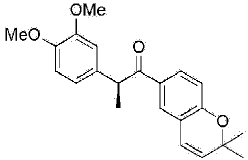 |
| Example 55 (Compound 101) | 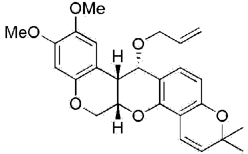 |

The present invention not only includes the compounds represented by formula 2 or the pharmaceutically acceptable salt but also includes every possible solvate, hydrate, constructed from the same by the conventional method.

The compounds represented by formula 2 of the present invention can be used as a form of a pharmaceutically acceptable salt, in which the salt is preferably acid addition salt formed by pharmaceutically acceptable free acids. The acid addition salt can be obtained from inorganic acids such as hydrochloric acid, nitric acid, phosphoric acid, sulfuric acid, hydrobromic acid, hydroiodic acid, nitrous acid and phosphorous acid, or non-toxic organic acids such as aliphatic mono/dicarboxylate, phenyl-substituted alkanoate, hydroxy alkanoate, alkandioate, aromatic acids and aliphatic/aromatic sulfonic acids. The pharmaceutically non-toxic salts are exemplified by sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, cabacate, fumarate, maliate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, hydroxybutylate, glycolate, malate, tartrate, methanesulfonate, propanesulfonate, naphthalene-1-sulfonate, naphthalene-2-sulfonate and mandelate.

The acid addition salt in this invention can be prepared by the conventional method known to those in the art. For example, the compounds represented by formula 2 of the present invention are dissolved in acid aqueous solution. Then, the salt is obtained by precipitating the solution by using water-miscible organic solvent such as methanol, ethanol, acetone, or acetonitrile. The salt can be obtained by another way. The equal amount of each compound represented by formula 2 of the present invention and acid or alcohol in water are heated, followed by evaporating/drying the mixture to give the acid addition salt or suction-filtering the precipitated salt to give the same.

A pharmaceutically acceptable metal salt can be prepared by using a base. Alkali metal or alkali earth metal salt is obtained by the following processes: dissolving the compound in excessive alkali metal hydroxide or alkali earth metal hydroxide solution; filtering non-soluble compound salt; evaporating the remaining solution and drying thereof. At this time, the metal salt is preferably prepared in the pharmaceutically suitable form of sodium, potassium, or calcium salt. And the corresponding silver salt is prepared by the reaction of alkali metal or alkali earth metal salt with proper silver salt (ex; silver nitrate).

The compound of the present invention can be produced by the method informed in the following references [(a) Caboni, P.; Sherer, T.; Zhang, N.; Taylor, G.; Na, H.; Greenamyre, J.; Casida, J. Rotenone, deguelin, their metabolites, and the rat model of Parkinson's disease. Chem. Res. Toxicol 2004, 17, 1540-1548; (b) Anzeveno, P. Rotenoid interconversion. Synthesis of deguelin from rotenone. J. Org. Chem. 1979, 44, 2578-2580], but not always limited thereto. Besides, any informed method or even uninformed method can be used as long as it can synthesize the compound of the present invention.

The compound synthesized by the method of the present invention proceeds to high performance liquid chromatography (HPLC) for the separation/purification and then the molecular structure thereof can be identified by nuclear magnetic resonance (NMR).

The present description also provides a pharmaceutical composition for preventing or treating cancer comprising the compound of formula 1 and/or the compound of formula 2 or the pharmaceutically acceptable salt thereof as an active ingredient.

The compound represented by formula 2 or the pharmaceutically acceptable salt thereof of the present invention suppresses the expression of Hsp90, by which it can inhibit the accumulation of HIF-1α, the Hsp90 client protein, and the activity of VEGF. The inhibition of HIF-1α activity indicates all the activity to inhibit the accumulation of HIF-1α or the expression of HIF-1α target gene.

In this invention, the inhibitory effect of the compound represented by formula 1 and/or formula 2 on the accumulation of HIF-1α was investigated. As a result, the compound of the invention was confirmed to inhibit HIF-1α accumulation dose-dependently without affecting the generation of tublin, the comparative control, under hypoxic condition.

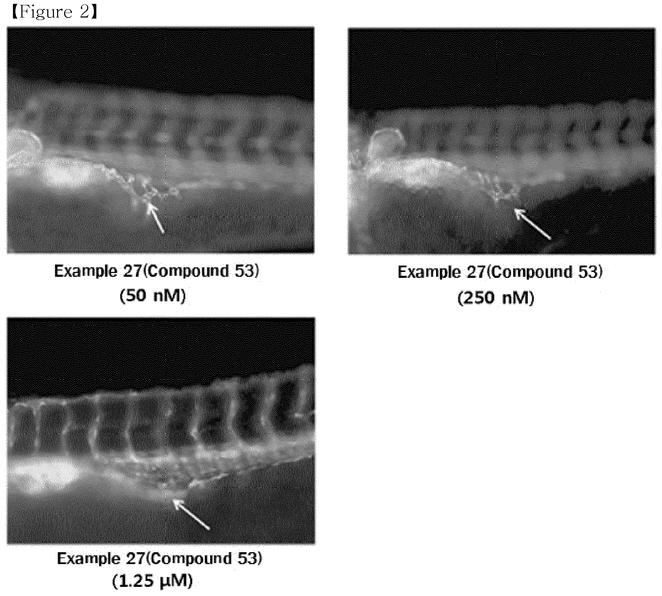

The compound represented by formula 2 of the present invention was also confirmed to inhibit the expression of VEGF (vascular endothelial growth factor A) dose-dependently that has been known to be an important factor for cancer growth and metastasis, among the HIF-1α target genes (see Figures 2 ∼ 4).

Therefore, the compound represented by formula 2 of the present invention can be used as an anticancer agent treating various cancers including colorectal cancer, liver cancer, stomach cancer, breast cancer, colon cancer, bone cancer, pancreatic cancer, head/neck cancer, uterine cancer, ovarian cancer, rectal cancer, esophageal cancer, small bowel cancer, anal cancer, colon cancer, fallopian tube carcinoma, endometrial carcinoma, uterine cervical carcinoma, vaginal carcinoma, Hodgkin's disease, prostate cancer, bladder cancer, kidney cancer, ureter cancer, renal cell carcinoma, renal pelvic carcinoma, and central nervous system tumor since the compound can suppress the expression of Hsp90 and accordingly inhibit the accumulation of HIF-1α, the Hsp90 client protein, and the activity of VEGF.

In addition, the present invention provides a pharmaceutical composition having the therapeutic effect on diabetic retinopathy or arthritis by inhibiting Hsp90 comprising the compound of formula 1 and/or the compound of formula 2 or the pharmaceutically acceptable salt thereof as an active ingredient.

Hsp90 can be a target of the study to develop a therapeutic agent for the disease particularly whose aggravation is related to the activation of angiogenesis. Angiogenesis factors like VEGF induced by HIF-1α, the Hsp90 client protein, activated under hypoxic condition are involved in the progress of such diseases as diabetic retinopathy and rheumatoid arthritis. Thus, the composition capable of inhibiting HIF-1α activated under hypoxic condition in disease tissue can be used as a therapeutic agent for diabetic retinopathy or arthritis.

The compound of formula 2 of the present invention was confirmed to be excellent in inhibiting angiogenesis in Zebra fish embryo (see Figures 2 ∼ 4).

Therefore, the compound of the present invention can selectively inhibit the expression of VEGF (vascular endothelial growth factor A), the angiogenesis factor, so that it can be effectively used as an active ingredient of a pharmaceutical composition to treat diabetic retinopathy or arthritis aggravated with the increase of VEGF expression induced by HIF-1α under hypoxic condition.

The present description also provides a treatment method for cancer, diabetic retinopathy, or rheumatoid arthritis containing the step of administering the compound represented by formula 1 and/or formula 2 of the invention or the pharmaceutically acceptable salt thereof to a subject with cancer, diabetic retinopathy, or rheumatoid arthritis.

The pharmaceutical composition of the present invention can be administered orally or parenterally and be used in general forms of pharmaceutical formulation, but not always limited thereto. The formulations for oral administration are exemplified by tablets, pills, hard/soft capsules, solutions, suspensions, emulsions, syrups, granules, and elixirs, etc. These formulations can include diluents (for example, lactose, dextrose, sucrose, mannitol, sorbitol, cellulose, and/or glycine) and lubricants (for example, silica, talc, stearate and its magnesium or calcium salt, and/or polyethylene glycol) in addition to the active ingredient. Tablets can include binding agents such as magnesium aluminum silicate, starch paste, gelatin, methylcellulose, sodium carboxymethylcellulose and/or polyvinylpyrolidone, and if necessary disintegrating agents such as starch, agarose, alginic acid or its sodium salt or azeotropic mixtures and/or absorbents, coloring agents, flavors, and sweeteners can be additionally included thereto.

The pharmaceutical composition comprising the compound represented by formula 2 of the present invention can be administered by parenterally and the parenteral administration includes subcutaneous injection, intravenous injection, intramuscular injection and intrathoracic injection. To prepare the composition as a formulation for parenteral administration, the compound represented by formula 1 and/or formula 2 or the pharmaceutically acceptable salt thereof of the present invention are mixed with a stabilizer or a buffering agent to produce a solution or suspension, which is then formulated as ampoules or vials. The composition herein can be sterilized and additionally contains preservatives, stabilizers, wettable powders or emulsifiers, salt and/or buffers for the regulation of osmotic pressure, and other therapeutically useful materials, and the composition can be formulated by the conventional mixing, granulating or coating method. The effective dosage of the pharmaceutical composition comprising the compound represented by formula 1 and/or formula 2 as an active ingredient of the present invention is 0.1 ∼ 500 mg/kg(weight) per day, preferably 0.5 ∼ 100 mg/kg(weight) per day for a mammal including human, which can be administered orally or parenterally several times a day or preferably once a day or a couple of times a day.

The pharmaceutical composition of the present invention can be administered alone or treated together with surgical operation, radiotherapy, hormone therapy, chemo-therapy and biological regulators.

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Manufacturing Example 1: Preparation of Methyl 2-(3,4-dimethoxyphenyl)acetate (32)

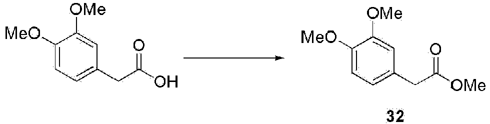

carboxylic acid (1 equivalent) and anhydrous DMF (catalytic amount) were dissolved in anhydrous CH₂Cl₂ (0.1 M). After lowering the temperature of the mixture to 0°C, oxalic acid (3 equivalent) was added drop by drop to the mixture. After stirring the mixture at room temperature for 30 minutes, anhydrous methanol (50 equivalent) was added drop by drop, followed by stirring again for 10 minutes. Then, the mixture was treated with water. The water layer was extracted with CH₂Cl₂ and the organic layer was dried over MgSO₄, followed by filtering. After concentrating the mixture under reduced pressure, the residue was purified by flash column chromatography (EtOAc:n-hexane=1:3) to give the compound 32.
¹H NMR (CDCl₃, 300 MHz) δ 6.79 (s, 3H), 3.86 (s, 3H), 3.84 (s, 3H), 3.67 (s, 3H), 3.55 (s, 2H).

### Manufacturing Example 2: Preparation of Methyl 2-(3,4-dimethoxyphenyl)-3-(4-methoxybenzyloxy)propanoate (33)

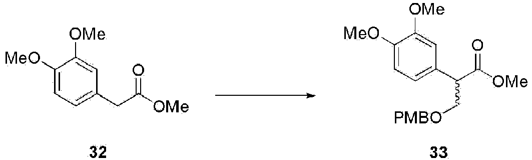

The compound 32 (500 mg, 2.38 mmol) prepared in Manufacturing Example 1 and paraformaldehyde paraformaldehyde (76 mg, 2.50 mmol) were dissolved in anhydrous DMSO (5.0 mL), which was then treated with sodium methoxide (6.8 mg, 0.12 mmol). The mixture was stirred at room temperature for 24 hours, which was poured into ice water (10 mL), followed by stirring. The mixture was neutralized with 2 N-HCl solution, which was poured into water, followed by extraction with EtOAc (×3). The organic layer was washed with saturated brine once and dried over MgSO₄, followed by filtering and concentrating under reduced pressure. Then, the residue was purified by silica gel resolution chromatography (EtOAc:*n*-hexane=1:1) to give the aldol product as a yellow solid (yield: 58%, 331 mg).
¹H NMR (CDCl₃, 300 MHz) δ6.74 (m, 3H), 4.05 (m, 1H), 3.81 (s, 3H), 3.79 (s, 3H), 3.73 (m, 2H), 3.65 (s, 3H).

*p*-methoxybenzyl 2,2,2-trichloroacetamidate (84 mg, 0.30 mmol) and CSA (catalytic amount) were added to anhydrous CH₂Cl₂ solution (1.0 mL) containing the aldol product (36 mg, 0.15 mmol) prepared above at room temperature. The reaction mixture was stirred under argon atmosphere overnight, followed by filtering. The resultant filter cake was washed with CH₂Cl₂. The filtrate was extracted with saturated NaHCO₃ solution and brine. The organic layer was dried over MgSO₄, filtered, and then evaporated. Then, the residue was purified by flash column chromatography (EtOAc:n-hexane=1:3) to give the compound 33 of Manufacturing Example 2 as a white solid (yield: 100%, 54 mg).
¹H NMR (CDCl₃, 500 MHz) δ7.19 (d, 2H, *J =* 8.5 Hz), 6.84 (d, 2H, *J* = 8.5 Hz), 6.78 (m, 3H), 4.46 (AB quartet, 2H, *J* = 33.4, 11.7 Hz), 3.98 (m, 1H), 3.83 (s, 6H), 3.80 (m, 2H), 3.77 (s, 3H), 3.67 (s, 3H).

### Manufacturing Example 3: Preparation of 2-(3,4-Dimethoxyphenyl)-3-(4-methoxybenzyloxy)propanal (34)

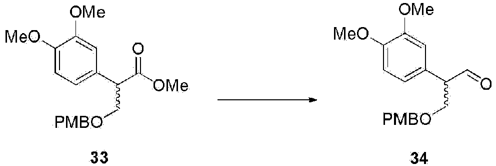

The compound 33 (20 mg, 0.055 mmol) prepared in Manufacturing Example 2 was dissolved in anhydrous THF (1.0 mL), which was treated with methanol (0.06 mmol, 1.1 equivalent) and then cooled down to -78°C. DIBAL-H (0.16 mL, 0.16 mmol) was slowly added thereto. While monitoring the reaction mixture with TLC, the mixture was stirred at -78°C until the reaction was completed. Rochelle aqueous solution was carefully added thereto for 15 minutes. The mixture of the two layers was stirred strongly at 0°C for 1 hour, which was added to water. The water layer was extracted with EtOAc (×2) and the organic layer was extracted with anhydrous NaSO₄, filtered and concentrated under reduced pressure. Then, the obtained residue was purified by flash column chromatography (EtOAc:n-hexane=1:4 ∼ 1:1) to give the primary alcohol (yield: 88%, 16 mg).
¹H NMR (CDCl₃, 500 MHz) δ 7.21 (d, 2H, *J* = 8.5 Hz), 6.85 (d, 2H, *J* = 8.5 Hz), 6.77 (d, 1H, *J* = 8.6 Hz), 6.73 (m, 2H), 4.46 (s, 2H), 3.93 (m, 1H), 3.83 (s. 6H), 3.81 (m, 1H), 3.78 (s, 3H), 3.72 (m, 2H), 3.10 (quin, 1H, *J* = 6.5 Hz); HRMS (FAB) Calcd for C₁₉H₂₄O₅(M+H⁺): :332.1624, Found: 332.1628.

Dess-Martin periodinane (2.0 equivalent) was added to CH₂Cl₂ (0.03 M) solution containing the primary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:3) to give the compound 34 of Manufacturing Example 3 (yield: 71%, 11 mg).
¹H NMR (CDCl₃, 300MHz)δ9.66 (d, 1H, *J* = 1.65 Hz), 7.14 (d, 1H, *J* = 8.4 Hz), 6.78 (m, 3H), 6.66 (m. 2H), 4.40 (d, 2H, *J* = 3.5 Hz), 3.99 (dd, 1H, *J* = 8.6, 6.9 Hz), 3.80 (s, 3H), 3.77 (s, 3H), 3.73 (s, 3H), 3.70 (m, 2H).

### Manufacturing Example 4: Preparation of (S)-2-(3,4-Dimethoxyphenyl)-1-(5-methoxy-2,2-dimethyl-2H-chromen-6-yl)-3-(4-methoxybenzyl oxy)propan-1-one (35)

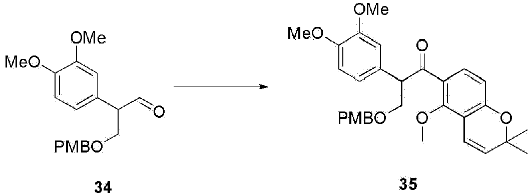

n-BuLi (1.4 equivalent) was added to anhydrous THF solution containing aryl bromide (1.5 equivalent) drop by drop at -78°C, which was stirred at -78°C to generate aryl anions. The mixture was stirred at - 78°C for 20 minutes, to which aldehyde (1.0 equivalent) was added, followed by stirring for 30 minutes with raising the reaction temperature to room temperature. The reaction mixture was treated with saturated NH₄Cl aqueous solution, followed by extraction with EtOAc. The extract was washed with brine and then dried over MgSO₄. The residue obtained after evaporating the solvent was purified by flash column chromatography (EtOAc:n-hexane=1:2) to give the secondary alcohol (yield: 72%, 34 mg).
HRMS (FAB) Calcd for C₃₁H₃₆O₇ (M+H⁺) : 543.2359, Found: 543.2365.

Dess-Martin periodinane (3.0 equivalent) was added to CH₂Cl₂ (0.03 M) solution containing the secondary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:3) to give the compound 35 of Manufacturing Example 4 (yield: 84%, 26 mg).
¹H NMR (CDCl₃, 300 MHz) δ7.34 (d, 1H, *J* = 8.6 Hz), 7.11 (d, 2H, *J* = 8.6 Hz), 6.73 (m, 5H), 6.50 (d, 1H, *J* = 10.0 Hz), 6.45 (d, 1H, *J* = 8.2 Hz), 5.57 (d, 1H, *J* = 9. 9 Hz), 4.83 (dd, 1H, *J* = 8. 9, 5. 1 Hz), 4.39 (q, 2H, *J* = 11.5 Hz), 4.12 (t, 1H, *J* = 9.1 Hz), 3.75 (s, 6H), 3.71 (s, 3H), 3.57 (dd, 1H, *J* = 9.1, 5.1 Hz), 3.52 (s, 3H), 1.34 (s, 6H);
HRMS (FAB) Calcd for C₃₁H₃₅O₇ (M+H⁺): 519.2383, Found: 519.2373.

### Manufacturing Example 5: Preparation of (S)-2-(3,4-Dimethoxyphenyl)-3-hydroxy-1-(5-hydroxy-2,2-dimethyl-2H-chromen-6-yl)propan-1-one (36)

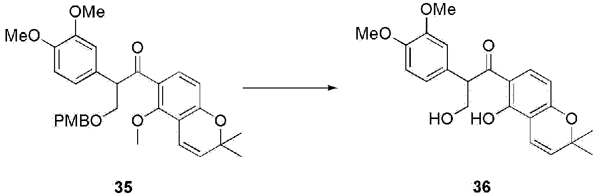

The compound 35 (23 mg, 0.044 mmol) prepared in Manufacturing Example 4 and CH₂Cl₂ (1.0 mL) were loaded in a 10 mL round-bottom flask, which was dried by heating. The mixture was cooled down to -78°C, to which boron trichloride (0.16 mL, 0.16 mmol, 1.0 M in CH₂Cl₂ solution) was added. After stirred for 1 hour, the reaction was terminated with saturated NH₄Cl aqueous solution. Then, the reaction mixture was extracted with CH₂Cl₂, dried over MgSO₄, and concentrated under reduced pressure. The residue was purified by flash column chromatography (EtOAc:n-hexane=1:2) to give the compound 36 of Manufacturing Example 5 as a light-yellow solid (yield: 75%, 12 mg).
¹H NMR (CDCl₃, 300 MHz) δ 7.40 (d, 1H, *J* = 8.7 Hz), 6.76 (m, 3H), 6.53 (d, 1H, *J* = 11.1 Hz), 6.49 (d, 1H, *J* = 10.5 Hz), 5.63 (d, 1H, *J* = 9.9 Hz), 4.75 (dd, 1H, *J* = 8.7, 4.8 Hz), 3.81 (s, 3H), 3.80 (s, 3H), 3.77 (m, 2H), 1.39 (s, 3H), 1.38 (s, 3H);
HRMS (FAB) Calcd for C₂₂H₂₄O₆ (M⁺): 384.1573, Found: 384.1570.

### Manufacturing Example 6: Preparation of methyl 2-(2-bromo-4,5-dimethoxyphenyl)acetate (38)

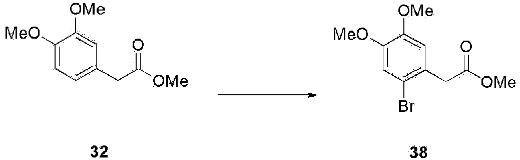

*N*-bromosuccinimide (449 mg, 2.50 mmol) was added to anhydrous THF (12.0 mL) solution containing the compound 32 (500 mg, 2.38 mmol) prepared in Manufacturing Example 1. The reaction mixture was stirred at -78°C for 30 minutes. After raising the reaction temperature to room temperature, the mixture was filtered and concentrated under reduced pressure. The obtained residue was purified by flash column chromatography (EtOAc:n-hexane=1:3) to give the compound 38 of Manufacturing Example 6 as a light-yellow solid (yield: 96%, 660 mg).
¹H NMR (CDCl₃, 300 MHz) δ 6.96 (s, 1H), 6.72 (s, 1H), 3.79 (s, 6H), 3.66 (s, 2H), 3.65 (s, 3H).

### Manufacturing Example 7: Preparation of methyl 2-(2-bromo-4,5-dimethoxyphenyl)pent-4-enoate (39)

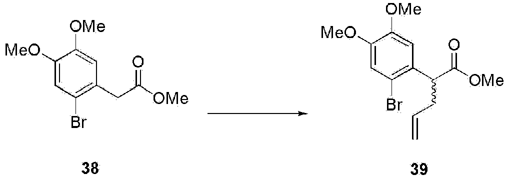

The compound 38 (150 mg, 0.52 mmol) obtained in Manufacturing Example 6 was dissolved in anhydrous THF (6.0 mL) at -78°C, to which LHMDS (0.63 mL, 0.93 mmol, 1.0 M in THF solution) was added drop by drop. After stirred for 20 minutes, the mixture was added with aryl bromide (0.044 mL, 0.52 mmol) drop by drop, followed by stirring again for 1 hour with raising the temperature to -40°C. The mixture was treated with saturated NH₄Cl aqueous solution (5.0 mL), to which water (5.0 mL) was added. The water layer was extracted with EtOAc (2×15 mL) and the organic layer was dried over MgSO₄, filtered, and concentrated under reduced pressure. The obtained residue was purified by flash column chromatography (EtOAc:n-hexane=1:8) to give the compound 39 of Manufacturing Example 7 (yield: 53%, 90 mg).
¹H NMR (CDCl₃, 300 MHz) δ 6.94 (s, 1H), 6.81 (s, 1H), 5.68 (m, 1H), 4.98 (m, 2H), 4.11 (dd, 1H, *J* = 8.2, 7.2 Hz), 3.78 (s, 6H), 3.61 (s, 3H), 2.69 (m, 1H), 2.42 (m, 1H).

### Manufacturing Example 8: Preparation of 1-(1-(Benzyloxy)pent-4-en-2-yl)-2-bromo-4,5-dimethoxybenzene (40)

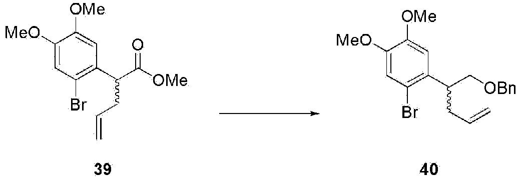

The compound 39 (84 mg, 0.25 mmol) prepared in Manufacturing Example 7 was dissolved in anhydrous THF (2.0 mL), and the mixture was cooled down to 0°C. Lithium aluminum hydride (10 mg, 0.25 mmol) was added thereto. The mixture was stirred at room temperature for 1 hour until the reaction was completed. Then, the reaction mixture was cooled down to 0°C again, to which NaHCO₃ (saturated aqueous solution) was carefully added for 30 minutes. The mixture of two phases was stirred vigorously at 0°C for 1 hour, to which water was added. The water layer was extracted with EtOAc (×2) and the organic layer was dried over MgSO₄, filtered, and concentrated under reduced pressure. The obtained residue was purified by flash column chromatography (EtOAc:n-hexane=1:6) to give the primary alcohol (yield: 88%).
¹H NMR (CDCl₃, 500 MHz) δ 7.01 (s, 1H), 6.73 (s, 1H), 5.73 (m, 1H), 5.03 (d, 1H, *J* = 17.1 Hz), 4.97 (d, 1H, *J* = 10.1 Hz), 3.84 (s, 3H), 3.83 (s, 3H), 3.77 (d, 2H, *J* = 5.7 Hz), 3.41 (quin, 1H, *J* = 7.1 Hz), 2.50 (quin, 1H, *J* = 7.1 Hz), 2.37 (quin, 1H, *J* = 7.1 Hz).

Sodium hydride (64 mg, 1.59 mmol, 60% in mineral oil) was added to anhydrous THF (7.0 mL) solution containing the primary alcohol (400 mg, 1.33 mmol) obtained above at 0°C, followed by stirring at room temperature for 30 minutes. Tetrabutyl ammonium bromide (23 mg, 0.066 mmol) and benzyl bromide (0.19 ml, 0.59 mmol) were added thereto, and the mixture was stirred at room temperature overnight. The mixture was treated with saturated NH₄Cl aqueous solution (5.0 mL), followed by extraction with EtOAc (10 mL×2). The extract was washed with brine, dried over MgSO₄, filtered, and concentrated under reduced pressure. The obtained residue was purified by flash column chromatography (EtOAc:n-hexane=1:8) to give the compound 40 of Manufacturing Example 8 as a colorless solid (yield: 86%, 448 mg).
¹H NMR (CDCl₃, 300 MHz) δ7.23 (m, 5H), 6.94 (s, 1H), 6.71 (s, 1H), 5.65 (m, 1H), 4.93 (m, 2H), 4.43 (s, 2H), 3.78 (s, 3H), 3.72 (s, 3H), 3.53 (m, 3H), 2.53 (m, 1H), 2.30 (m, 1H);
HRMS (FAB) Calcd for C₂₀H₂₃BrO₃ (M⁺): 390.0831, Found: 390.0839.

### Manufacturing Example 9: Preparation of 4-(Benzyloxy)-3-(2-bromo-4,5-dimethoxyphenyl)butan-l-ol (41)

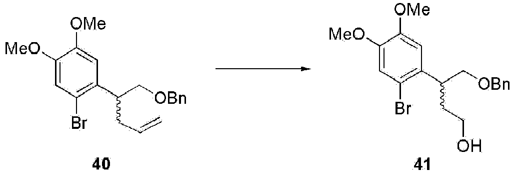

NMO (233 mg, 1.93 mmol) was added to the compound 40 (252 mg, 0.64 mmol) prepared in Manufacturing Example 8, which was dissolved in acetone:water (4:1, 25 mL) solution at 0°C. OsO₄ (0.32 mL, 0.032 mmol, 0.1 M in toluene solution) added thereto, followed by stirring at room temperature for 6 hours. The reaction mixture was extracted with EtOAc (3×15 mL). The extract was washed with saturated sodium sulfite solution and dried over MgSO₄. The solvent was removed under reduced pressure and the resultant non-purified product was dissolved in acetone:water (4:1, 25 mL) solution, followed by stirring at room temperature. NaIO₄ (413 mg, 1.93 mmol) was slowly added thereto. The mixture was stirred at room temperature for 30 minutes and then extracted with EtOAc (3×15 mL). The extract was washed with saturated sodium thiosulfate solution. The solvent was eliminated under reduced pressure and as a result, non-purified aldehyde was obtained. The non-purified aldehyde was used for the next step without being through purification process.

Sodium borohydride (49 mg, 1.29 mmol) was added to methanol (6.0 mL) containing the non-purified aldehyde (0.64 mmol) obtained above at -100°C. The temperature was maintained for 1 hour. The mixture was cooled down with saturated NH₄Cl aqueous solution, followed by extraction with EtOAc. The extract was dried over MgSO₄ and concentrated under reduced pressure. The obtained residue was purified by flash column chromatography (EtOAc:n-hexane=1:1) to give the compound 41 of Manufacturing Example 9 as a colorless solid (yield: 73%, 184 mg).
¹H NMR (CDCl₃, 300 MHz) δ 7.23 (m, 5H), 6.94 (s, 1H), 6.69 (s, 1H), 4.47 (s, 2H), 3.78 (s, 3H), 3.73 (s, 3H), 3.54 (m, 4H), 2.01 (m, 2H), 1.81 (m, 1H);
HRMS (FAB) Calcd for C₁₉H₂₃BrO₄ (M⁺): 394.0780, Found: 394.0774.

### Manufacturing Example 10: Preparation of 4-(Benzyloxymethyl)-6,7-dimethoxychroman (42)

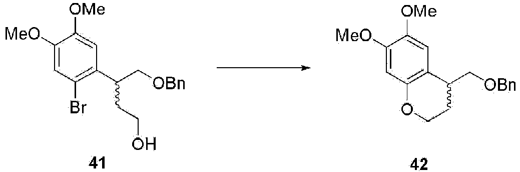

The compound 41 (379 mg, 0.96 mmol) obtained in Manufacturing Example 9 and sodium *t*-butoxide (124 mg, 1.25 mmol) were loaded in the heat dried 2-neck round-bottom flask containing toluene (10 mL) containing Pd₂(dba)₃ (13 mg, 0.0143 mmol) and 2-(di-*t-*butylphosphino)biphenyl (7.0 mg, 0.024 mmol) under argon atmosphere. For the reaction, the flask was heated at 50 ∼ 55°C overnight and then the temperature was cooled down to room temperature. The mixture was diluted with EtOAc (10 mL) and filtered using celite. The solvent was eliminated under reduced pressure and the obtained dark-yellow non-purified product was purified by flash column chromatography (EtOAc:n-hexane=1:6) to give the compound 42 of Manufacturing Example 10 as a light-yellow solid (yield: 75%, 226 mg).
¹H NMR (CDCl₃, 300 MHz) δ 7.24 (m, 5H), 6.61 (s, 1H), 6.30 (s, 1H), 4.48 (q, 2H, *J* = 11.9 Hz), 4.03 (m, 2H), 3.73 (s, 3H), 3.70 (s, 3H), 3.61 (m, 1H), 3.49 (m, 1H), 2.98 (m, 1H), 1.95 (m, 2H);
HRMS (FAB) Calcd for C₁₉H₂₂O₄ (M⁺): 314.1518, Found: 314.1518.

### Manufacturing Example 11: Preparation of 6,7-Dimethoxychroman-4-carbaldehyde (43)

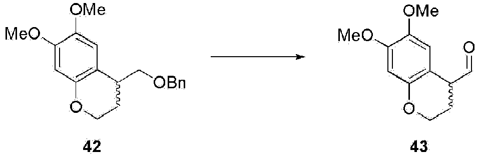

The methanol mixture (5 mL) containing the compound 42 (191 mg, 0.61 mmol) obtained in Manufacturing Example 10 and 20% Pd(OH)₂/C (38 mg) was stirred at room temperature for 5 hours under hydrogen atmosphere. The mixture was filtered using celite, and the filtrate was washed with methanol (10 mL). The methanol was evaporated and the obtained residue was purified by flash column chromatography (EtOAc:n-hexane=1:1) to give the primary alcohol (yield: 100%, 158 mg).
¹H NMR (CDCl₃, 300 MHz) δ6.61 (s, 1H), 6.33 (s, 1H), 4.08 (m, 2H), 3.78 (m, 2H), 3.74 (s, 6H), 2.85 (m, 1H), 1.98 (m, 2H); LRMS (FAB) *m*/*z* 225 (M+H⁺).

Dess-Martin periodinane (3.0 equivalent) was added to CH₂Cl₂ (0.03 M) solution containing the primary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:2) to give the compound 43 of Manufacturing Example 11 (yield: 60%, 15 mg).
¹H NMR (CDCl₃, 300 MHz) δ9.67 (d, 1H, *J* = 1.8 Hz), 6.58 (s, 1H), 6.42 (s, 1H), 4.13 (m, 3H), 3.81 (s, 6H), 2.32 (m, 1H), 2.06 (m, 1H).

### Manufacturing Example 12: Preparation of (6,7-Dimethoxychroman-4-yl)(2,2-dimethyl-2H-chromen-6-yl)methanol (44)

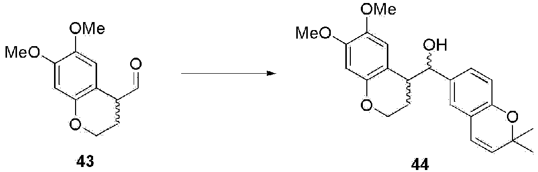

n-BuLi (1.4 equivalent) was added to anhydrous THF solution containing 6-bromo-2,2-dimethyl-2*H-*chromene (1.5 equivalent) drop by drop at -78°C, which was stirred at -78°C to generate aryl anions. After stirred at -78°C for 20 minutes, the mixture was added with the compound 43 (1.0 equivalent) obtained in Manufacturing Example 11. The mixture was stirred again for 30 more minutes to increase the reaction temperature to room temperature. The reaction mixture was treated with saturated NH₄Cl aqueous solution, followed by extraction with EtOAc. The extract was washed with brine and then dried over MgSO₄. The residue obtained after evaporating the solvent was purified by flash column chromatography (EtOAc:n-hexane=1:4) to give the compound 44 of Manufacturing Example 12 (yield: 75%, 18 mg).
HRMS (FAB) Calcd for C₂₃H₂₆O₅ (M+H⁺) : 382.1780, Found: 382.1793.

### Manufacturing Example 13: Preparation of 2-(3,4-Dimethoxyphenyl) acetaldehyde (52)

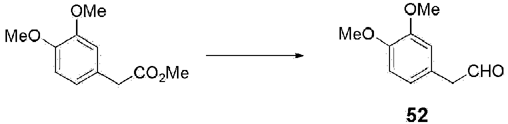

Anhydrous diethyl ether solution containing methyl 3',4'-dimethoxyphenyl acetate (1.94 g, 9.24 mmol) was stirred, during which DIBAL-H (11.1 mL, 1 M in THF solution) was added thereto drop by drop with maintaining the temperature at -78°C. The mixture was stirred at the same temperature for 1 hour. Rochelle aqueous solution was carefully added thereto for 15 minutes. The mixture of the two phases was stirred vigorously at 0°C for 1 hour, which was added to water. The water layer was extracted with EtOAc (×2) and the organic layer was dried over MgSO₄, filtered and concentrated under reduced pressure. Then, the obtained residue was purified by flash column chromatography (EtOAc:n-hexane=1:2) to give the compound 52 of Manufacturing Example 13 (yield: 68%, 1.13 g).
¹H NMR (CDCl₃, 300 MHz) δ 9.66 (t, 1H, *J* = 2.5 Hz), 6.80 (d, 1H, *J* = 8.0 Hz), 6.68 (m, 1H), 6.64 (d, 1H, *J* = 1.8 Hz), 3.81 (s. 6H), 3.56 (d, 2H, *J* = 2.5 Hz).

### Manufacturing Example 14: Preparation of 2-(3,4-Dimethoxyphenyl)propanal (55)

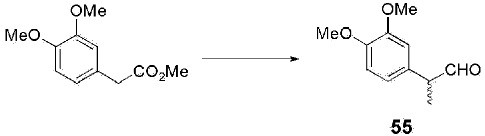

LDA (18.1 mL, 36.21 mmol, 2 M in THF solution) was added drop by drop to anhydrous THF solution (5 mL) containing methyl 3',4'-dimethoxyphenyl acetate (4.76 g, 22.63 mmol) under argon atmosphere at -78°C, followed by stirring for 30 minutes. Then, methyl iodide (2.82 mL, 45.26 mmol) was added thereto, followed by stirring for 1 hour. 2 N-HCl aqueous solution was added to the above reaction mixture, leading to acidization, and then the mixture was extracted with EtOAc. The organic layer was washed with brine, dried over MgSO₄, and concentrated under reduced pressure. The obtained residue was purified by silica gel column chromatography (EtOAc:*n-*hexane=1:4) to give 2-(3,4-dimethoxyphenyl)propanoate as a colorless oil (yield: 79%, 4.01 g).
¹H NMR (CDCl₃, 500 MHz) δ 6.80 (m, 3H), 3.85 (s, 3H), 3.83 (s, 3H), 3.64 (q, 1H, *J*=7.1 Hz), 3.63 (s, 3H), 1.46 (d, 3H, *J* = 7.1 Hz);
¹³C NMR (CDCl₃, 125 MHz) δ 175.7, 148.9, 148.1, 133.0, 119.5, 111.2, 110.6, 55.8, 55.8, 51.9, 44.9, 18.6.

Anhydrous THF solution containing the 2-(3,4-dimethoxyphenyl)propanoate obtained above was stirred, during which DIBAL-H (16.0 mL, 1 M in THF solution) was added drop by drop with maintaining the temperature at -78°C. The mixture was stirred again for 1 hour at the same temperature. Rochelle aqueous solution was carefully added thereto for 15 minutes. The mixture of the two phases was stirred vigorously at 0°C for 1 hour, which was added to water. The water layer was extracted with EtOAc (×2) and the organic layer was dried over MgSO₄, filtered and concentrated under reduced pressure. Then, the obtained residue was purified by flash column chromatography (EtOAc:n-hexane=1:2) to give the compound 55 of Manufacturing Example 14 (yield: 61%, 1.58 g).
¹H NMR (CDCl₃, 300 MHz) δ 9.63 (d, 1H, *J* = 1.5 Hz), 6.86 (d, 1H, *J* = 8.2 Hz), 6.74 (m, 1H), 6.66 (d, 1H, *J* = 2.0 Hz), 3.85 (s. 6H), 3.56 (q, 1H, *J* = 6.9 Hz), 1.40 (d, 3H, *J =* 6.9 Hz).

### Manufacturing Example 15: Preparation of 2-(3,4-Dimethoxyphenyl)propanoic acid (64)

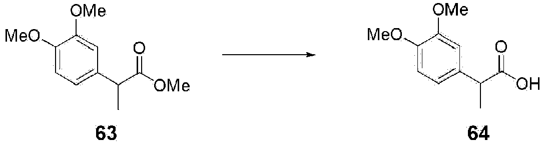

Monohydrate (230 mg, 5.35 mmol) was added to THF/water solution (2/1, 9.0 mL) containing the compound 63 (400mg, 1.78 mmol) at room temperature, followed by stirring for 5 hours. Upon completion of the reaction, the reaction mixture was extracted with EtOAc (×2). The organic layer was alkalization with 2 N-NaOH aqueous solution, and the water layer was acidization with 2 N-HCl aqueous solution. The water layer was extracted with EtOAc (×2) and the organic layer was washed with brine, dried over MgSO₄, filtered and concentrated under reduced pressure. The obtained product was used in Manufacturing Examples 16 and 17.
¹H NMR (CDCl₃, 300 MHz) δ 6.83 (m, 3H), 3.85 (s, 3H), 3.84 (s, 3H), 3.66 (q, 1H, *J*=7.3 Hz), 1.48 (d, 3H, J=7.3 Hz).

### Manufacturing Example 16∼17: Preparation of (S)-2-(3,4-Dimethoxyphenyl)-N-((R)-2-hydroxy-1-phenylethyl) propaneamide (65) and (R)-2-(3,4-Dimethoxyphenyl)-N-((R)-2-hydroxy-1-phenylethyl)propanamide (66)

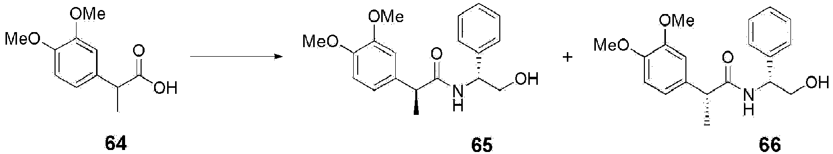

*N*-(3-dimethylaminopropyl)-*N*'-ethylcarbodimide hydrochloride (EDCI) (287 mg, 1.47 mmol), (*R*)-(-)-2-phenylglycinol (207 mg, 1.47 mmol) and HOBt (230 mg, 1.47 mmol) were added to CH₂Cl₂ solution (10 mL) containing the compound 64 (282 mg, 1.34 mmol) prepared in Manufacturing Example 15. Finally, diisopropylethylamine (0.26 mL, 1.47 mmol) was added thereto drop by drop at 0°C. The reaction mixture was stirred at room temperature overnight. The reaction was terminated with saturated NH₄Cl aqueous solution and the mixture was diluted with CH₂Cl₂. The organic layer was washed with water, dried over MgSO₄, filtered, and concentrated under reduced pressure. Then, the obtained residue was purified by flash column chromatography (EtOAc: CH₂Cl₂:*n*-hexane=3:2:1) to give the compound 65 (yield: 42%, 187 mg) of Manufacturing Example 16 and the compound 66 (yield: 42%, 186 mg) of Manufacturing Example 17 as light-yellow solids. and (yield: 61%, 1.58 g).
(S)-Diastereomer (65): ¹H NMR (CDCl₃, 300 MHz) δ 7.26 (m, 3H), 7.03 (m, 2H), 6.81 (m, 2H), 6.73 (s, 1H), 6.05 (m, 1H), 5.02 (m, 1H), 3.86 (s, 3H), 3.80 (m, 2H), 3.78 (s, 3H), 3.58 (q, 1H, *J* = 7.1 Hz) 1.48 (d, 3H, *J* = 7.1 Hz);
(*R*)-Diastereomer (66) : ¹H-NMR (CDCl₃, 300 MHz) δ 7.23 (m, 3H), 7.10 (m, 2H), 6.78 (m, 3H), 5.97 (m, 1H), 4.96 (m, 1H), 3.82 (s, 3H), 3.80 (s, 3H), 3.72 (d, 2H, *J* = 5.0 Hz), 3.50 (q, 1H, *J* = 7.1 Hz) 1.46 (d, 3H, *J* = 7.1 Hz).

### Manufacturing Example 18: Preparation of (S)-2-(3,4-Dimethoxyphenyl) propanoic acid (67)

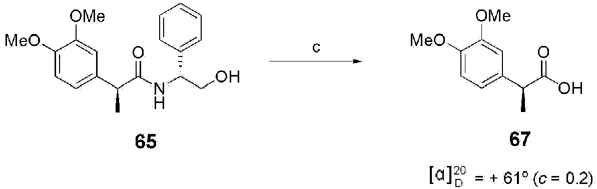

Sulfonic acid aqueous solution (5.0 mL, 4.0 M in water) was added to 1,4-dioxane solution (5.0 mL) containing the compound 65 (186 mg, 0.56 mmol) prepared in Manufacturing Example 16 slowly at 0°C. The reaction mixture was refluxed for 2 hours, cooled down at room temperature, and diluted with EtOAc. The organic layer was washed with water, dried over MgSO₄, filtered, and concentrated under reduced pressure to give the compound 67 as a brown oil (yield: 100%, 117 mg).
¹H-NMR (CDCl₃, 300 MHz) δ 6.83 (m, 3H), 3.86 (s, 3H), 3.84 (s, 3H), 3.67 (q, 1H, *J* = 7.1 Hz), 1.47 (d, 3H, J=7.1 Hz).

### Manufacturing Example 19: Preparation of (S)-2-(3,4-Dimethoxyphenyl) propanal (68)

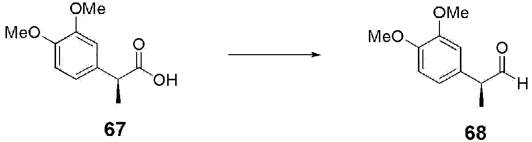

Anhydrous diethyl ether solution (8.0 mL) containing the compound 67 (115 mg, 0.55 mmol) prepared in Manufacturing Example 17 was cooled down at 0°C, to which BH₃·SMe₂ complex (1.5 mL, 3.00 mmol) was added drop by drop. The mixture was stirred for 1 hour at 0°C, and then stirred again for 3 more hours at room temperature. The reaction was terminated by adding water drop by drop at 0°C. When hydrogen gas bubbles were no more observed, 2 N-NaOH aqueous solution was added thereto drop by drop. The water layer was extracted with ether (x2) and the ether layer was washed with brine, dried over MgSO₄, filtered, and concentrated under reduced pressure. Then, the obtained residue was purified by flash column chromatography (EtOAc:n-hexane=1:2) to give the primary alcohol as a light-yellow solid (yield: 85%, 104 mg).
¹H-NMR (CDCl₃, 500 MHz) δ 6.78 (m, 3H), 3.87 (s, 3H), 3.85 (s, 3H), 3.66 (m, 2H), 2.88 (sex, 1H, *J* = 6.9 Hz), 1.24 (d, 3H, *J* = 6.9 Hz).

Dess-Martin periodinane (3.0 equivalent) was added to CH₂Cl₂ (0.03 M) solution containing the primary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:3) to give the compound 68 of Manufacturing Example 19 (yield: 81%, 21 mg).
¹H-NMR (CDCl₃, 300 MHz) δ9.63 (d, 1H, *J* = 1.5 Hz), 6.86 (d, 1H, *J* = 8.2 Hz), 6.74 (dd, 1H, *J* = 8.2, 2.0 Hz), 6.66 (d, 1H, *J* = 2.0 Hz), 3.86 (s, 6H), 3.55 (q, 1H, *J* = 7.1 Hz), 1.40 (d, 3H, *J* = 7.1 Hz).

### Manufacturing Example 20: Preparation of (R)-2-(3,4-Dimethoxyphenyl) propanoic acid (70)

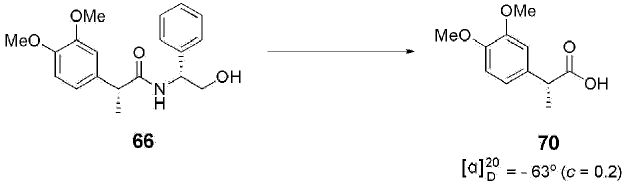

Sulfonic acid aqueous solution (5.6 mL, 4.0 M in water) was added to 1,4-dioxane solution (5.6 mL) containing the compound 66 (217 mg, 0.66 mmol) prepared in Manufacturing Example 17 slowly at 0°C. The reaction mixture was refluxed for 2 hours, cooled down at room temperature, and diluted with EtOAc. The organic layer was washed with water, dried over MgSO₄, filtered, and concentrated under reduced pressure to give the compound 70 as a brown oil (yield: 100%, 138 mg).
¹H-NMR (CDCl₃, 300 MHz) δ 6.83 (m, 3H), 3.86 (s, 3H), 3.84 (s, 3H), 3.67 (q, 1H, *J* = 7.1 Hz), 1.47 (d, 3H, J=7.1 Hz).

### Manufacturing Example 21: Preparation of (R)-2-(3,4-Dimethoxyphenyl) propanal (71)

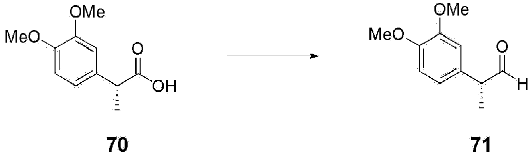

Anhydrous diethyl ether solution (10.0 mL) containing the compound 70 (115 mg, 0.55 mmol) prepared in Manufacturing Example 20 was cooled down at 0°C, to which BH₃·SMe₂ complex (1.8 mL, 3.61 mmol) was added drop by drop. The mixture was stirred for 1 hour at 0°C, and then stirred again for 3 more hours at room temperature. The reaction was terminated by adding water drop by drop at 0°C. When hydrogen gas bubbles were no more observed, 2 N-NaOH aqueous solution was added thereto drop by drop. The water layer was extracted with ether (x2) and the ether layer was washed with brine, dried over MgSO₄, filtered, and concentrated under reduced pressure. Then, the obtained residue was purified by flash column chromatography (EtOAc:n-hexane=1:2) to give the primary alcohol as a light-yellow solid (yield: 81%, 104 mg).
¹H-NMR (CDCl₃, 500 MHz) δ 6.78 (m, 3H), 3.87 (s, 3H), 3.85 (s, 3H), 3.66 (m, 2H), 2.88 (sex, 1H, *J* = 6.9 Hz), 1.24 (d, 3H, *J* = 6.9 Hz).

Dess-Martin periodinane (3.0 equivalent) was added to CH₂Cl₂ (0.03 M) solution containing the primary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:3) to give the compound 71 of Manufacturing Example 21 (yield: 79%, 16 mg).
¹H-NMR (CDCl₃, 300 MHz) δ 9.63 (d, 1H, *J* = 1.5 Hz), 6.86 (d, 1H, *J* = 8.2 Hz), 6.74 (dd, 1H, *J* = 8.2, 2.0 Hz), 6.66 (d, 1H, *J* = 2.0 Hz), 3.86 (s, 6H), 3.55 (q, 1H, *J* = 7.1 Hz), 1.40 (d, 3H, *J* = 7.1 Hz).

### Manufacturing Example 22: Preparation of 4-Nitrobenzene-1,3-diol (46)

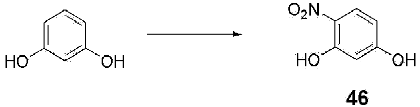

Resorcinol (5.0g, 44.96 mmol) was dissolved in chloroform:acetic acid (2:1, 270 mL) mixed solution, to which acetic acid solution (70 mL) containing nitric acid (3.6 mL) was slowly added, followed by stirring for 1 hour. The reaction was terminated by adding water (100 mL), followed by extraction with CH₂Cl₂ (100 mL×3) and drying over MgSO₄. The residue obtained after filtering under reduced pressure was purified by flash column chromatography (EtOAc:n-hexane=1:4 ∼ EtOAc:*n*-hexane:CH₂Cl₂=1:4:2) to give the compound 46 as a yellow solid (yield: 52%, 3.6 g).
¹H NMR (CD₃OD, 300 MHz) δ 7.99 (d, 1H, *J* = 9.1 Hz), 6.43 (m, 2H).

### Manufacturing Example 23: Preparation of 5-(2-Methylbut-3-yn-2-yloxy)-2-nitrophenol (47)

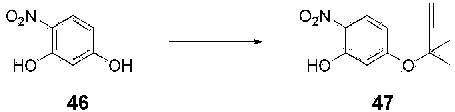

DBU (0.63 mL, 4.19 mmol) was added to acetonitrile solution (18 mL) containing 2-methyl-3-butin-2-ol (0.36 mL, 3.70 mmol) at 0°C, to which trifluoroacetanhydride (0.58 mL, 4.19 mmol) was added drop by drop for 30 minutes. The obtained yellow solution was stirred at 0°C for 40 minutes. Acetonitrile solution (18 mL) containing the compound 46 (500 mg, 3.22 mmol) prepared in Manufacturing Example 22 in another flak was treated with DBU (0.63 mL, 4.19 mmol) at 0°C, to which CuCl₂ (8.65 mg, 0.064 mmol) was added. The yellow solution prepared above (2-methyl-3-butin-2-yl trifluoroacetate) was added to this mixture drop by drop for 40 minutes at 0°C. The reaction mixture was stirred at 0°C overnight. The resultant residue obtained by concentration under reduced pressure was poured in water. The water layer was extracted with ethyl acetate and the organic layer was washed with 1 N-HCI, 1 N-KOH, and brine stepwise. The organic layer was then dried over MgSO₄ and concentrated under reduced pressure to give the compound 47 (Yield: 45%, 320 mg).
¹H NMR (CDCl₃, 300 MHz) δ 7.90 (d, 1H, *J* = 9.4 Hz), 6.70 (d, 1H, *J* = 10.0 Hz), 6.38 (d, 1H, *J* = 9.4 Hz), 5.63 (d, 1H, *J* = 10.0 Hz), 1.45 (s, 6H);
HRMS (FAB) Calcd for C₁₁H₁₂NO₄ (M+H⁺): 206.1181, Found: 206.1186.

### Manufacturing Example 24: Preparation of 2,2-Dimethyl-6-nitro-2H-chromen-5-ol (48)

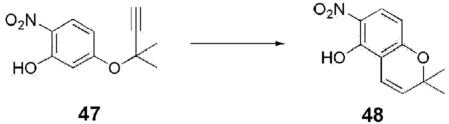

While monitoring N,N-diethylaniline solution (28 mL) containing *O*-alkylated phenol (310 mg, 1.40 mmol) with TLC, the solution was heated at 130°C until the reaction was completed under nitrogen atmosphere. The reaction mixture was poured in ice water, followed by extraction with EtOAc. The organic layer was washed with 2 *N*-HCl aqueous solution, 2 *N*-sodium hydroxide aqueous solution, and water stepwise, dried over MgSO₄, and concentrated under reduced pressure. Then, the obtained residue was purified by flash column chromatography (EtOAc:n-hexane=1:15) to give the compound 48 of Manufacturing Example 24 as a light-yellow solid (yield: 91%, 281 mg).
¹H NMR (CDCl₃, 300 MHz) δ 7.90 (d, 1H, *J* = 9.4 Hz), 6.70 (d, 1H, *J* = 10.0 Hz), 6.38 (d, 1H, *J* = 9.4 Hz), 5.63 (d, 1H, *J* = 10.0 Hz), 1.45 (s, 6H);
HRMS (FAB) Calcd for C₁₁H₁₂NO₄ (M+H⁺) : 206.1181, Found: 206.1186.

### Manufacturing Example 25: Preparation of 5-Methoxy-2,2-dimethyl-6-nitro-2H-chromene (49)

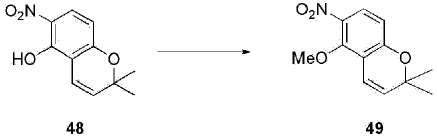

Anhydrous acetone (7.0 mL) containing the compound 48 (130 mg, 0.59 mmol) obtained in Manufacturing Example 24, potassium carbonate (244 mg, 1.76 mmol) and iodomethane (0.11 mL, 1.76) was heated at 55°C overnight. The mixture was concentrated and then treated with water, followed by extraction with EtOAc (×3). The extract was washed with water, dried over MgSO₄, and concentrated under reduced pressure to give the compound 49.
¹H NMR (CDCl₃, 300 MHz) δ 7.77 (d, 1H, *J* = 8.9 Hz), 6.59 (m, 2H), 5.72 (d, 1H, *J* = 10.0 Hz), 3.89 (s, 3H), 1.44 (s, 6H) ;
HRMS (FAB) Calcd for C₁₂H₁₄NO₄ (M+H⁺): 236.0923, Found: 236.0924.

### Manufacturing Example 26: Preparation of 5-Methoxy-2,2-dimethyl-2H-chromen-6-amine (50)

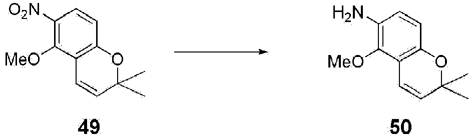

Boiling ethanol (2.0 mL) containing the compound 49 (136 mg, 0.663 mmol) prepared in Manufacturing Example 25 and tin(II) chloride dihydrate (740 mg, 3.21 mmol) was stirred for 1 hour under nitrogen atmosphere. Ethanol was eliminated by vacuum evaporation and the residue was extracted with ethyl acetate (×3). The organic layer was treated with 2 N-NaOH solution (10 mL) and then treated with water (2×10 mL). The organic layer was washed with brine, dried over MgSO₄, and concentrated under reduced pressure. Then, the obtained residue was purified by flash column chromatography (EtOAc:n-hexane=1:15) to give the compound 50 of Manufacturing Example 26 as a yellow solid (yield: 79%, 100 mg).
¹H NMR (CDCl₃, 300 MHz) δ 6.54 (m, 2H), 6.43 (d, 1H, *J* = 8.4 Hz), 5.63 (d, 1H, *J* = 9.9 Hz), 3.78 (s, 3H), 3.46 (br, 2H), 1.37 (s, 6H);
HRMS (FAB) Calcd for C₁₂H₁₅NO₂ (M⁺): 205.1103, Found: 205.1104.

### Manufacturing Example 27: Preparation of 6-Bromo-5-methoxy-2,2-dimethyl-2H-chromene (51)

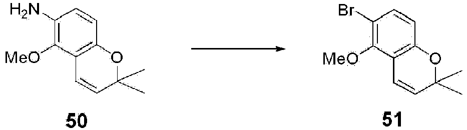

Water (5.0 mL) containing the compound 50 (260 mg, 1.27 mmol) obtained in Manufacturing Example 26 was cooled down to 0°C, to which HBr (1.6 mL, 48% in water) was slowly added. The reaction mixture was stirred vigorously for 10 minutes at 0°C. The slurry containing NaNO₂ and HBr was slowly added to water (1.0 mL) containing sodium nitrite. The temperature of the reaction mixture was maintained under 5°C. Water (5.0 mL) containing Cu(I)Br (190 mg, 1.30 mmol) was loaded in a separatory funnel, which was then heated at 60°C. Aniline solution was added thereto drop by drop, during which the temperature was maintained at 60°C. Upon completion of the reaction, the reaction mixture was cooled down to room temperature, followed by extraction with EtOAc (x2). The organic layer was washed with brine, dried over MgSO₄, and concentrated under reduced pressure. Then, the obtained residue was purified by flash column chromatography (EtOAc:n-hexane=1:12) to give the compound 51 of Manufacturing Example 27 as a light-yellow oil (yield: 90%, 365 mg).
¹H NMR (CDCl₃, 500 MHz) δ 7.16 (d, 1H, *J* = 8.6 Hz), 6.52 (d, 1H, *J* = 10.0 Hz), 6.42 (d, 1H, *J* = 8.6 Hz), 5.59 (d, 1H, *J* = 10.0 Hz), 3.74 (s, 3H), 1.35 (s, 6H) ;
¹³C NMR (CDCl₃, 125 MHz) δ153.3, 152.7, 132.1, 131.2, 116.9, 116.6, 113.9, 107.3, 76.1, 61.5, 30.9, 27.7.

### Manufacturing Example 28: Preparation of 1,2-Dimethoxy-4-(phenylsulfonylmethyl)benzene (105)

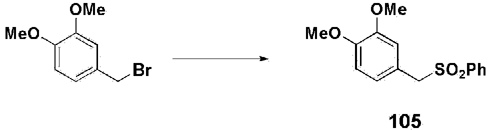

4-(bromoethyl)-1,2-dimethoxybenzene (100 mg, 0.43 mmol) and benzenesulfonic acid sodium salt dihydrate (87 mg, 0.52 mmol) were added to anhydrous DMF solution (1.5 mL). The mixture was stirred at 80°C for 12 hours and then cooled down at room temperature. Upon completion of the conventional synthesis using water and EtOAc, the solvent was evaporated. Then, the obtained residue was purified by flash column chromatography (EtOAc:n-hexane:CH₂Cl₂=1:1:1) to give the compound 105 of Manufacturing Example 28 as a colorless oil (yield: 93%, 118 mg).
¹H NMR (CDCl₃, 300 MHz) δ7.56 (m, 3H), 7.39 (m, 1H), 6.67 (d, 1H, *J* = 8.2 Hz), 6.55(dd, 2H, *J* = 8.1, 2.0 Hz), 6.47(d, 1H, *J* = 2.0 Hz), 4.18(s, 2H), 3.79(s, 3H), 3.65 (s, 3H).

### Manufacturing Example 29: Preparation of 5-Hydroxy-2,2-dimethyl-2H-chromene-6-carbaldehyde (110)

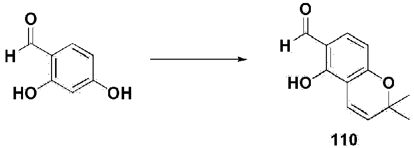

Pyridine (0.01 M) solution containing 3-methyl-but-2-enyl (2.0 equivalent) was added to anhydrous acetone solution (0.01 M) containing 2,4-dihydroxyaldehyde (1 equivalent) drop by droop at 120°C for 5.5 hours, followed by heating for 18 hours. After cooling down at room temperature, the solvent was eliminated under reduced pressure using a rotary evaporator. Pyridine was eliminated by azeotropic distillation along with toluene. The obtained non-purified product was purified by flash column chromatography (EtOAc:n-hexane=1:8) to give the compound 119 as a colorless solid (yield: 32%, 1.12 g).
¹H-NMR (CDCl₃, 300 MHz) δ11.61 (s, 1H), 9.62 (s, 1H), 7.25 (d, 1H, *J* = 8.6 Hz), 6.39 (d, 1H, *J* = 8.6 Hz), 5.58 (d, 1H, *J* = 10.0 Hz), 1.42 (s, 6H).

### Manufacturing Example 30: Preparation of 5-(Methoxymethoxy)-2,2-dimethyl-2H-chromene-6-carbaldehyde (108)

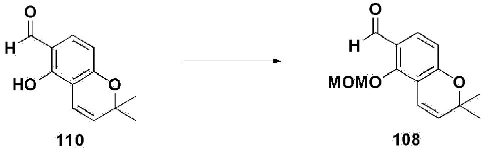

Chloromethylmethyl ether (0.11 mL, 1.47 mmol) was added to acetone solution (5 mL) containing the compound 110 (200 mg, 0.98 mmol) obtained in Manufacturing Example 29 and potassium carbonate (406 mg, 2.94 mmol) at room temperature. The mixture was refluxed for 3 hours, followed by concentration under reduced pressure. The resultant non-purified residue was extracted with EtOAc and the organic layer was washed with brine. The extract was dried over anhydrous MgSO₄ and concentrated under reduced pressure. The obtained non-purified residue was purified by flash column chromatography (EtOAc:n-hexane=1:10) to give the compound 108 of Manufacturing Example 30 as a light-yellow solid (yield: 100%, 231 mg).
¹H NMR (CDCl₃, 300 MHz) δ 10.07(s, 1H), 7.59 (d, *J* = 8.6 Hz, 1H), 6.61 (d, *J* = 8.6 Hz, 1H), 6.53 (d, *J* = 10.0 Hz, 1H), 5.63(d, *J* = 10.0 Hz, 1H), 5.02(s, 2H), 3.52(s, 3H).

### Manufacturing Example 31: Preparation of 2-(3,4-Dimethoxyphenyl)-1-(5-hydroxy-2,2-dimethyl-2H-chromen-6-yl)-2-(phenylsulfonyl)ethanone (106)

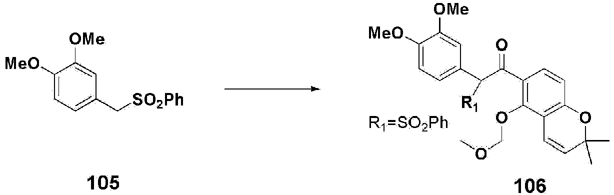

*n*-BuLi (0.26 mL, 0.444 mmol, 1.6 M in hexane) was added to anhydrous THF solution (5.0 mL) containing the compound 105 (119 mg, 0.407 mmol) prepared in Manufacturing Example 28 at -78°C, which was stirred at the same temperature for 1 hour. Anhydrous THF (3.0 mL) containing the compound 108 (121 mg, 0.448 mmol) obtained in Manufacturing Example 30 was added slowly to the above reaction mixture at -78°C. One hour later, water was added to the reaction mixture to terminate the reaction, followed by extraction with EtOAc (×3). The extract was washed with brine, dried over anhydrous MgSO₄, and concentrated under reduced pressure. The obtained intermediate residue (secondary alcohol) was used for the next step without being through purification process.

Dess-Martin periodinane (286 mg, 0.660 mmol) was added to anhydrous CH₂Cl₂ (5.0 mL) solution containing the secondary alcohol (183 mg, 0.339 mmol) obtained above, followed by stirring for 1 hour. The reaction mixture was treated with saturated sodium carbonate solution:saturated sodium thiosulfate solution (1:1, 4 mL), followed by stirring for 30 minutes. The reaction mixture was poured in water, followed by extraction with CH₂Cl₂ (×3). The organic layer was dried over MgSO₄, filtered and concentrated under reduced pressure. Then, the obtained residue was purified by flash column chromatography (EtOAc:n-hexane=1:2) to give the compound 106 of Manufacturing Example 31 (yield: 63%, 137 mg).
¹H NMR (CDCl₃, 300 MHz) δ 7.95 (dd, 1H, *J* = 20.0, 7.9 Hz), 7.58 (m, 3H), 7.34 (m, 3H), 6.81 (dd, 1H, *J* = 8.4, 2.2 Hz), 6.74 (d, 1H, *J* = 2.2 Hz), 6.70 (d, 1H, *J* = 8.4 Hz), 6.60 (d, 1H, *J* = 10.0 Hz), 5.85 (s, 1H), 5.50(d, 1H, *J* = 10.0 Hz), 3.78 (s, 3H), 3.63 (s, 3H), 1.37 (s, 3H), 1.33(s, 3H).

### Manufacturing Example 32: Preparation of 2-(3,4-Dimethoxyphenyl)-1-(5-hydroxy-2,2-dimethyl-2H-chromen-6-yl)ethanone (107)

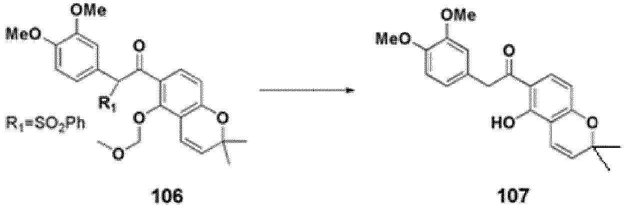

SmI₂ (0.81 mL, 0.081 mmol, 0.1 M in THF) was added to anhydrous THF (1.0 mL) containing the compound 106 (10 mg, 0.0202 mmol) prepared in Manufacturing Example 31 at -20°C. The reaction mixture was stirred at - 20°C for 10 minutes and then the reaction was terminated with saturated ammonium chloride aqueous solution (2.0 mL). The mixture was filtered by celite pad, followed by extraction with EtOAc (4.0 mL×3). The organic layer was dried over 20% sodium thiosulfate saturated aqueous solution, filtered and concentrated under reduced pressure. Then, the obtained residue was purified by flash column chromatography (EtOAc:n-hexane=1:3) to give the compound 107 of Manufacturing Example 32 as a light-yellow solid (yield: 87%, 77 mg).
¹H NMR (CDCl₃, 300 MHz) δ 12.88 (s, 1H), 7.58 (d, 1H, *J* = 8.8 Hz), 7.74 (s, 1H), 6.71 (s, 1H), 6.63 (d, 1H, *J* = 10.0 Hz), 6.25 (d, 1H, *J* = 8.8 Hz), 5.50 (d, 1H, *J* = 10.0 Hz), 4.15 (s, 2H), 3.79 (s, 6H), 1.37 (s, 6H).

### Manufacturing Example 33: Preparation of 1,2-Dimethoxy-4-(prop-2-ynyloxy)benzene (115)

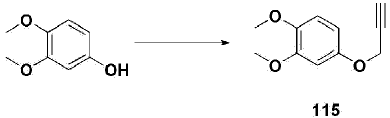

Propargyl bromide (225 mg, 1.51 mmol) and potassium carbonate (211 mg, 1.51 mmol) were added to DMF (0.2 M) solution containing 3,4-dimethoxyphenol, which was stirred at room temperature for 12 hours. The reaction was terminated with saturated ammonium chloride aqueous solution, followed by extraction with diethyl ether. The organic layer was washed with water (×2) and brine, dried over anhydrous MgSO₄, and concentrated under reduced pressure. Then, the obtained residue was purified by flash column chromatography (n-hexane : CH₂Cl₂=1:1) to give the compound 115 of Manufacturing Example 33 as a light-yellow oil (yield: 100%, 241 mg).
¹H NMR (CDCl₃, 300 MHz) δ 6.79 (d, 1H, *J* = 8.8 Hz), 6. 60 (d, 1H, *J* = 2.7 Hz), 6.49 (dd, 1H, *J* = 8.8, 2.7 Hz), 4.65 (d, 2H, *J* = 2.4 Hz), 3.86(s, 3H), 3.84 (s, 3H), 2.52 (t, 1H, *J* = 2.4 Hz).

### Manufacturing Example 34: Preparation of 4-(3-Bromoprop-2-ynyloxy)-1,2-dimethoxybenzene (116)

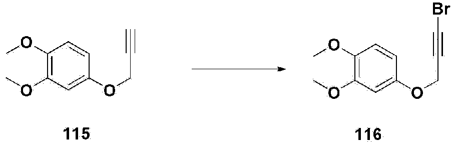

Cold bromine (1.12 mmol, 0.03 mL) was added to saturated sodium hydroxide aqueous solution (0.5 mL, 5.0 M), to which dimethoxyethane (1.0 mL) containing the compound 115 (108 mg, 0.56 mmol) obtained in Manufacturing Example 33 was added. Next, the flask was filled with nitrogen gas, which was stirred vigorously at room temperature for 5 hours. Ice water was poured in the reaction mixture, followed by extraction with n-hexane (5.0 mL). The extract was washed with water, dried over anhydrous sodium sulfate, and concentrated under reduced pressure. Then, the obtained residue was purified by flash column chromatography (EtOAc:n-hexane=1:5) to give the compound 116 of Manufacturing Example 34 as a light-yellow oil (yield: 86%, 130 mg).
¹H NMR (CDCl₃, 300 MHz) δ 6.79 (d, 1H, *J* = 8.6 Hz, 1H), 6.58 (d, 1H, *J* = 2.9 Hz), 6.47 (dd, 1H, *J* = 8.6, 2.9 Hz), 4.66 (s, 2H), 3.86 (s, 3H), 3.84(s, 3H).

### Manufacturing Example 35: Preparation of 4-Bromo-6,7-dimethoxy-2H-chromene (118)

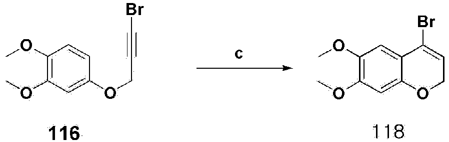

The compound 116 (130 mg, 0.48 mmol) prepared in Manufacturing Example 34 was added to *N*,*N-*diethylaniline (7.0 mL). While monitoring with TLC, the reaction mixture was heated at 210°C until the reaction was completed under nitrogen atmosphere. The reaction mixture was poured in ice water, followed by extraction with EtOAc (10 mL). The organic layer was washed with 2 *N*-HCl aqueous solution, 2 *N*-NaOH aqueous solution, and water stepwise, dried over anhydrous sodium sulfate, and concentrated under reduced pressure. Then, the obtained residue was purified by flash column chromatography (EtOAc:n-hexane=1:8) to give the compound 118 of Manufacturing Example 35 as a light-yellow solid (yield: 77%, 100 mg).
¹H NMR (CDCl₃, 300 MHz) δ 6.73 (s, 1H), 6.42 (s, 1H), 6.01 (t, 1H, *J* = 4.2 Hz), 4.72 (d, 2H, *J* = 4.2 Hz), 3.88 (s, 3H), 3.86 (s, 3H).

### Manufacturing Example 36: Preparation of 4-Bromo-6,7-dimethoxy-2,2-dimethyl-2H-chromene (119)

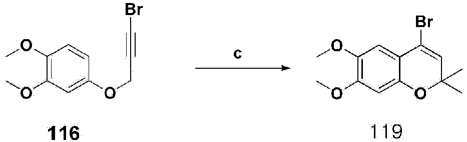

Anhydrous trichloroethylene solution (1.5 mL) containing 2,2-dimethylchromanone (100 mg, 0.41 mmol) was added to anhydrous trichloroethylene solution (1.5 mL) containing anhydrous DMF (0.05 mL, 0.62 mmol) and phosphorous oxibromide (188 mg, 0.62 mmol) at 0°C. While monitoring with TLC, the reaction mixture was heated at 60°C until the reaction was completed. By concentrating the reaction mixture under reduced pressure, an orange gum was obtained. The gum was extracted with CH₂Cl₂ (×5), and the extract was washed with brine. The organic layer was dried over anhydrous sodium sulfate, and concentrated under reduced pressure. Then, the obtained residue was purified by flash column chromatography (EtOAc:n-hexane=1:5) to give the compound 119 of Manufacturing Example 36 as a yellow solid (yield: 91%, 9 mg).
¹H NMR (CDCl₃, 300 MHz) δ 6.95 (s, 1H), 6.38 (s, 1H), 5.82 (s, 1H), 3.85 (s, 3H), 3.83 (s, 3H), 1.41(s, 6H).

### Manufacturing Example 37: Preparation of 2,2-dimethyl-2H-chromene-6-carbaldehyde (120)

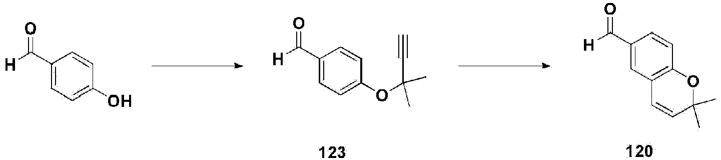

DMF (1.0 M) suspension containing phenol (1 equivalent), potassium carbonate (2 equivalent), potassium iodide (1.7 equivalent) and copper(I) iodide (0.02 equivalent) was prepared at room temperature, to which 3-chloro-3-methyl-1-butin (1.8 equivalent) was added. The reaction mixture was heated at 65°C for 2 hours and then cooled down at room temperature. Diethyl ether was added to the mixture, which was washed with 10% sodium hydroxide (x3) and brine. The extract was dried over anhydrous MgSO₄, and concentrated under reduced pressure. Then, the obtained residue was purified by silica gel column chromatography (EtOAc:n-hexane) to give o-alkylated phenol.

While monitoring *N*,*N*-diethylaniline solution (0.05 M) containing O-alkylated phenol (1 equivalent) with TLC, the solution was heated at 190°C until the reaction was completed under nitrogen atmosphere. The reaction mixture was poured in ice water, followed by extraction with EtOAc. The organic layer was washed with 2 *N*-HCl aqueous solution, 2 *N*-NaOH aqueous solution, and water stepwise, dried over anhydrous sodium sulfate, and concentrated under reduced pressure. Then, the obtained non-purified residue was purified by flash column chromatography (EtOAc:n-hexane) to give the compound 120 (yield: 46%, 326 mg).
¹H NMR (CDCl₃, 300 MHz) δ 9.80 (s, 1H), 7.15 (dd, 1H, *J* = 8.2, 2.0 Hz), 7.49 (d, 1H, *J* = 2.0 Hz), 6.84 (d, 1H, *J* = 8.4 Hz), 6.35 (d, 1H, *J* = 9.8 Hz), 5.67 (d, 1H, *J* = 9.8 Hz), 1.45 (s, 6H);
HRMS (FAB) Czalcd for C₁₂H₁₃rO₂(M+H⁺): 189.0916, Found: 189.0916.

### Manufacturing Example 38: Preparation of 5-(Methoxy)-2,2-dimethyl-2H-chromene-6-carbaldehyde (121)

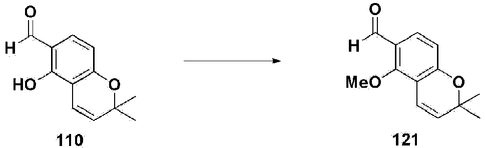

Iodomethane (0.11 mL, 1.47 mmol) was added to acetone solution (5 mL) containing the compound 110 (200 mg, 0.98 mmol) prepared in Manufacturing Example 29 and potassium carbonate (406 mg, 2.94 mmol) at room temperature. The mixture was refluxed for 3 hours, and then concentrated under reduced pressure. The obtained non-purified residue was extracted with EtOAc. The organic layer was washed with brine. The extract was dried over anhydrous MgSO₄, and concentrated under reduced pressure. Then, the obtained residue was purified by flash column chromatography (EtOAc:n-hexane=1:10) to give the compound 121 as a light-yellow solid (yield: 91%, 231 mg).
¹H NMR (CDCl₃, 300 MHz) δ 10.07 (s, 1H), 7.59 (d, *J* = 8.6 Hz, 1H), 6.61 (d, *J* = 8.6 Hz, 1H), 6.53 (d, *J* = 10.0 Hz, 1H), 5.63(d, *J* = 10.0 Hz, 1H), 5.02(s, 2H), 3.52(s, 3H).

### Manufacturing Example 39: Preparation of 6-Bromo-2,2-dimethyl-2H-chromene (131)

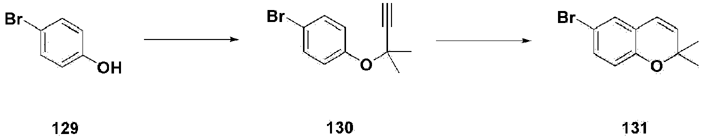

DBU (1.3 equivalent) was injected in acetonitrile (0.02 M)solution containing 2-methyl-3-butin-2-ol (1.15 equivalent) by using a syringe at 0°C for 30 minutes. The resultant yellow solution was stirred at 0°C for 40 minutes. In another flask, 4-bromophenol (1 equivalent) was added to acetonitrile (0.03 M) at 0°C, which was then treated with DBU (1.3 equivalent). CuC1₂·H₂0 (0.02 equivalent) was added thereto. The yellow solution prepared above (2-methyl-3-butin-2-yl trifluoroacetate) was added to the mixture at 0°C drop by drop by using a cannula for 40 minutes. The reaction mixture was stirred at 0°C for 5 hours and then concentrated under reduced pressure. The obtained residue was poured in water. The water layer was extracted with hexane, and the organic layer was washed with 1 *N*-HCI, 1 *N*-KOH (×2), and brine stepwise. The organic layer was dried over anhydrous MgSO₄ and concentrated under reduced pressure to give *o-*alkylated phenol (1.0 equivalent). This product was used for the next step.

While monitoring *N*,*N*-diethylaniline solution (0.05 M) containing *O*-alkylated phenol (1.0 equivalent) with TLC, the solution was heated at 190°C until the reaction was completed under nitrogen atmosphere. The reaction mixture was poured in ice water, followed by extraction with EtOAc. The organic layer was washed with 2 *N*-HCl aqueous solution, 2 *N-*NaOH aqueous solution, and water stepwise, dried over anhydrous sodium sulfate, and concentrated under reduced pressure. Then, the obtained non-purified residue was purified by flash column chromatography (EtOAc:n-hexane=1:20) to give the compound 131 as a light-yellow oil (yield: 68%, 464 mg).
¹H NMR (CDCl₃, 300 MHz) δ 7.15 (d, 1H, *J* = 8.4 Hz), 7.06 (d, 1H, *J* = 2.3 Hz), 6.63 (d, 1H, *J* = 8.4 Hz), 6.23 (d, 1H, *J* = 9.9 Hz), 5.62 (d, 1H, *J* = 9.9 Hz), 1.39 (s, 6H).

### Manufacturing Example 40: Preparation of (3,4-Dimethoxyphenyl)(2,2-dimethyl-2H-chromen-6-yl)methanol (132)

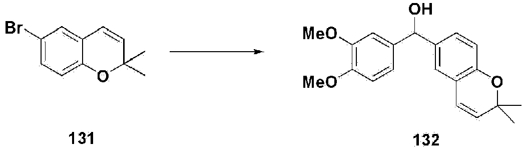

n-BuLi (0.17 mL, 0.28 mmol, 1.6 M in n-hexane solution) was added to anhydrous THF solution (3.0 mL) containing the compound 131 (60 mg, 0.25 mmol) obtained in Manufacturing Example 39 at -78°C, which was stirred for 30 minutes. Then, anhydrous THF (1.0 mL) containing 3,4-dimethoxybenzaldehyde (83 mg, 0.50 mmol) was added to the mixture drop by drop, followed by stirring for 30 minutes, during which the temperature was maintained at -78°C. Saturated ammonium chloride aqueous solution was added thereto to terminate the reaction, followed by extraction with EtOAc (×2). The extract was washed with brine. The organic layer was dried over anhydrous MgSO₄, and concentrated under reduced pressure. Then, the obtained residue was purified by flash column chromatography (EtOAc:n-hexane=1:4) to give the compound 132 as a white solid (yield: 66%, 108 mg).
¹H NMR (CDCl₃, 300 MHz) δ 7.06 (d, 1H, *J* = 8.2 Hz), 7.85 (m, 4H), 6.71 (d, 1H, *J* = 8.2 Hz), 6.26 (d, 1H, *J* = 9.8 Hz), 5.70 (s, 1H), 5.58 (d, 1H, *J* = 9.8 Hz), 3.84 (s, 3H), 3.83 (s, 3H), 2.09 (d, 1H, J = 3.2 Hz), 1.39 (s, 6H).

### Manufacturing Example 41: Preparation of 1-(5-hydroxy-2,2-dimethyl-2H-chromen-6-yl)ethanone (112)

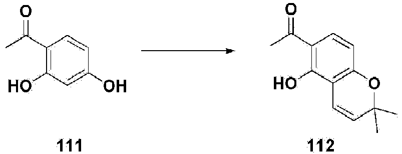

n-BuLi (1.4 equivalent) was added to anhydrous THF solution containing aryl bromide (1.5 equivalent) drop by drop at -78°C, which was stirred at -78°C to generate aryl anions. The mixture was stirred at - 78°C for 20 minutes, to which 2',4'-dihydroxyacetophenone (1.0 equivalent) was added, followed by stirring for 30 minutes with raising the reaction temperature to room temperature. The reaction mixture was treated with saturated NH₄Cl aqueous solution, followed by extraction with EtOAc. The extract was washed with brine and then dried over MgSO₄. The residue obtained after evaporating the solvent was purified by flash column chromatography (EtOAc:n-hexane=1:15 ∼ 1:10) to give the compound 112 as a light-yellow solid (yield: 32%, 460 mg).
¹H-NMR (CDCl₃, 300 MHz) δ 12.94 (s, 1H), 7.50 (d, 1H, *J* = 8.7 Hz), 6.79 (d, 1H, *J* = 10.0 Hz), 6.31 (d, 1H, *J* = 8.7 Hz), 5.56 (d, 1H, *J* = 8.7 Hz), 1.42 (s, 6H).

### Manufacturing Example 42: Preparation of 1-(2,2-Dimethyl-2H-chromen-6-yl)ethanone (134)

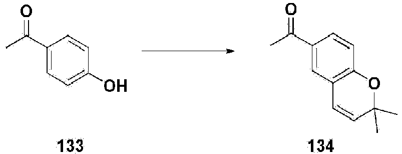

DMF (1.0 M) suspension containing compound 133 (1 equivalent), potassium carbonate (2 equivalent), potassium iodide (1.7 equivalent) and copper(I) iodide (0.02 equivalent) was prepared at room temperature, to which 3-chloro-3-methyl-1-butin (1.8 equivalent) was added. The reaction mixture was heated at 65°C for 2 hours and then cooled down at room temperature. Diethyl ether was added to the mixture, which was washed with 10% sodium hydroxide (x3) and brine. The extract was dried over anhydrous MgSO₄, and concentrated under reduced pressure. Then, the obtained residue was purified by silica gel column chromatography to give o-alkylated phenol.

While monitoring N,N-diethylaniline solution (0.05 M) containing o-alkylated phenol (1 equivalent) with TLC, the solution was heated at 190°C until the reaction was completed under nitrogen atmosphere. The reaction mixture was poured in ice water, followed by extraction with EtOAc. The organic layer was washed with 2 *N*-HCl aqueous solution, 2 *N*-NaOH aqueous solution, and water stepwise, dried over anhydrous sodium sulfate, and concentrated under reduced pressure. Then, the obtained non-purified residue was purified by flash column chromatography (EtOAc:n-hexane=1:15) to give the compound 134 as a colorless oil (yield: 65%, 248 mg).
¹H NMR (CDCl₃, 300 MHz) δ 7.32 (dd, 1H, *J* = 8.4, 2.1 Hz), 6.60 (d, 1H, *J* = 2.4 Hz), 6.77 (d, 1H, *J* = 8.4 Hz), 6.34 (d, 1H, *J* = 9.9 Hz), 5.65 (d, 1H, *J* = 9.9 Hz), 2.51 (s, 3H), 1.43(s, 6H); LRMS (FAB) *m*/*z* 203 (M+H⁺).

### Manufacturing Example 43: Preparation of (7S,7aR,13aS)-9,10-Dimethoxy-3,3-dimethyl-7,7a,13,13a-tetrahydro-3H-chromeno[3,4-b]pyrano[2,3-h]chromen-7-ol (14)

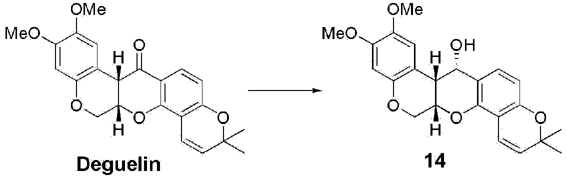

NaBH₄ (43 mg, 1.13 mmol) was added to methanol (10.0 mL) containing deguelin (300 mg, 0.76 mmol) dissolved therein at 0°C, followed by stirring for 30 minutes. The mixture was cooled down with water. The mixture was extracted with diethyl ether. The organic layer was washed with brine, dried over MgSO₄, and concentrated. Then, the obtained residue was purified by flash column chromatography (EtOAc:n-hexane=1:1) to give the compound 14 of Manufacturing Example 43 as a white solid (yield: 100%, 300 mg).
¹H-NMR (CDCl₃, 400 MHz) δ 6.99 (d, 1H, *J* = 8.2 Hz), 6.68 (s, 1H), 6.64 (d, 1H, *J* = 10.0 Hz), 6.47 (s, 1H), 6.41 (d, 1H, *J* = 8.2 Hz), 5.56 (d, 1H, *J* = 10.0 Hz), 4.80 (m, 2H), 4.57 (d, 1H, *J* = 10.0 Hz), 4.21 (m, 1H), 3.84 (s, 3H), 3.82 (s, 3H), 3.36 (t, 1H, *J* = 4.9 Hz), 1.41 (s, 3H), 1.39 (s, 3H);
¹³C-NMR (CDCl₃, 100 MHz) δ 154.3, 149.6, 149.4, 147.9, 143.8, 130.0, 129.1, 116.4, 113.6, 111.3, 109.8, 109.5, 108.7, 100.7, 76.0, 69.1, 66.3, 65.0, 56.5, 55.8, 37.9, 27.8, 27.7;
HRMS (FAB) Calcd for C₂₃H₂₄O₆ (M+H⁺) : 396.1560, Found: 396.1562.

### (Only examples covered by the scope of the claims form part of the invention).

### Example 1: Preparation of (6aS,12aS)-9-Hydroxy-2,3-dimethoxy-8-(3-methyl-2-butenyl)-6a,12a-dihydrochromeno [3,4-b]chromen-12(6H)-one (8)

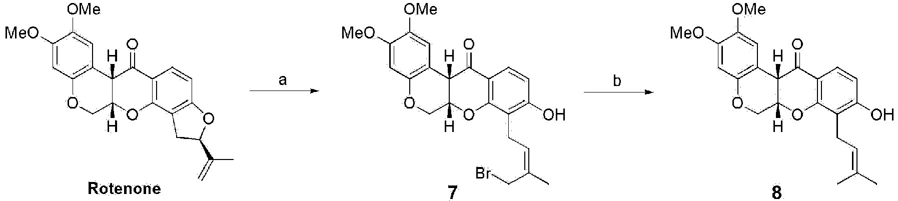

BBr₃ (0.53 mL, 0.53 mmol, 1.0 M in CH₂Cl₂ solution) was added to anhydrous CH₂Cl₂ solution (5.0 mL) containing rotenone (Sigma-Aldrich, 200 mg, 0.50 mmol) dissolved therein at -10°C under argon atmosphere. The reaction mixture was well mixed for 5 minutes. Methanol (1.0 mL) was added to the mixture and the solvent was eliminated under reduced pressure. The mixture was filtered and the non-purified residue (compound 7) obtained thereby was used for the next step without being additionally purified.

Compound 7 and NaBH₃CN (69 mg, 1.1 mmol) were added to hexamethylphosphoamide (HMPA) solution, which was then heated at 70°C for 3 hours with stirring. The mixture was poured in water. The extract of the mixture was washed with brine, dried over MgSO₄, and concentrated under reduced pressure. Then, the obtained residue was purified by flash column chromatography (EtOAc:n-hexane=1:2) to give the compound 8 of Example 1 as a light-yellow solid (yield: 50%, 55 mg).
¹H-NMR (CDCl₃, 500 MHz) δ 7.68 (d, 1H, *J* = 8.7 Hz), 6.76 (s, 1H), 6.50 (d, 1H, *J* = 8.7 Hz), 6.41 (s, 1H), 5.19 (m, 1H), 4.87 (t, 1H, *J* = 2.97 Hz), 4.60 (dd, 1H, *J* = 11.9, 3.1 Hz), 4.14 (d, 1H, *J* = 11.9 Hz), 3.80 (m, 1H), 3.75 (s, 3H), 3.70(s, 3H), 3.34 (m 2H), 1.74 (s, 3H), 1.65 (s, 3H);
¹³C-NMR (CDCl₃, 125 MHz) δ190.4, 162.1, 160.1, 149.3, 147.5, 143.6, 134.0, 126.9, 121.1, 114.8, 112.5, 110.7, 110.5, 108.5, 104.7, 100.8,72.0, 66.2, 56.2, 55.7, 44.1, 25.7, 22.0, 17.7;
HRMS (FAB) Calcd for C₂₃H₂₄O₆ (M⁺): 396.1573, Found: 396.1575.

### Example 2∼4: Preparation of (7aS,13aS)-9-Hydroxy-13,13a-dihydro-10-methoxy-3,3-dimethyl-3H-chromeno[3,4-b]pyrano[2,3-h]chromen-7(7aH)-one (9);

### (7aS,13aS)-10-Hydroxy-13,13a-dihydro-9-methoxy-3,3-dimethyl-3H-chromeno[3,4-b]pyrano[2,3 -h]chromen-7(7aH)-one (10); and

### (7aS,13aS)-13,13a-Dihydro-9,10-dihydroxy-3,3-dimethyl-3H-chromeno[3,4-b]pyrano[2,3-h]chromen-7(7aH)-one (11)

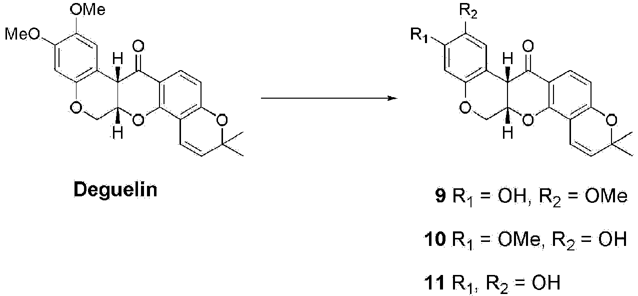

BBr₃ (0.25 mL, 0.25 mmol, 1.0 M in CH₂Cl₂ solution) was added to anhydrous CH₂Cl₂ solution (7.0 mL) containing deguelin (100 mg, 0.25 mmol) of Comparative Example 1 dissolved therein at -78°C under argon atmosphere, followed by stirring for 1 hour. The mixture stood until the temperature thereof reached 0°C. The mixture was additionally stirred for 30 more minutes and then cooled down with water, followed by extraction with CH₂Cl₂. The extract was washed with brine. The organic layer was dried over MgSO₄, filtered, and concentrated under reduced pressure. Then, the obtained residue was purified by flash column chromatography (EtOAc:n-hexane:CH₂Cl₂=1:3:1 ∼ 1:2:1) to give the compound 9 of Example 2 as a light-yellow solid (yield: 16%, 16 mg).
¹H-NMR (acetone-d₆, 400 MHz) δ 7.64 (d, 1H, *J* = 8.7 Hz), 7.60 (s, 1H), 6.67 (s, 1H), 6.59 (d, 1H, *J* = 10.1 Hz), 6.40 (d, 1H, *J* = 8.7 Hz), 6.30 (s, 1H), 5.66 (d, 1H, *J* = 10.1 Hz), 5.06 (m, 1H), 4.57 (dd, 1H, *J* = 12.2, 2.9 Hz), 4.22 (d, 1H, *J* = 12.2 Hz), 3.85 (d, 1H, *J* = 4.0 Hz), 3.62 (s, 3H), 1.39 (s, 3H), 1.30 (s, 3H);
¹³C-NMR (acetone-d₆, 100 MHz) δ190.2, 160.8, 158.2, 149.6, 148.6, 143.7, 130.4, 129.4, 116.6, 114.3, 112.2, 112.1, 110.3, 105.8, 105.2, 78.8, 74.0, 67.3, 57.3, 45.4, 29.0, 28.6;
HRMS (FAB) Calcd for C₂₂H₂₁O₆ (M+H⁺) : 381.1321, Found: 381.1327.

The compound 10 of Example 3 was also obtained as a light-yellow solid (yield: 14%, 14 mg).
¹H-NMR (acetone-d₆, 300 MHz) δ7.64 (d, 1H, *J* = 8.6 Hz), 7.07 (s, 1H), 6.60 (s, 1H), 6.59 (d, 1H, *J* = 10.1 Hz), 6.39 (d, 1H, *J* = 8.6 Hz), 6.38 (s, 1H), 5.67 (d, 1H, *J* = 10.1 Hz), 5.06 (m, 1H), 4.59 (dd, 1H, *J* = 12.2, 2.9 Hz), 4.23 (d, 1H, *J* = 12.2 Hz), 3.82 (d, 1H, *J* = 4.0 Hz), 3.71 (s, 3H), 1.39 (s, 3H), 1.30 (s, 3H);
¹³C-NMR (acetone-d₆, 75 MHz) δ190.0, 160.8, 158.2, 149.1, 148.2, 142.1, 130.4, 129.4, 116.6, 114.5, 114.3, 112.1, 110.3, 107.2, 102.1, 78.8, 73.9, 67.4, 56.6, 45.4, 29.0, 28.6;
HRMS (FAB) Calcd for C₂₂H₂₁O₆ (M+H⁺): 381.1338, Found: 381.1335.

Further, the compound 11 of Example 4 was prepared as a light-yellow solid (yield: 33%, 32 mg).
¹H-NMR (acetone-d₆, 400 MHz) δ7.75 (s, 1H), 7.63 (d, 1H, *J* = 8.7 Hz), 7.38 (s, 1H), 6.59 (s, 1H), 6.58 (d, 1H, *J* = 10.1 Hz), 6.39 (d, 1H, *J* = 8.7 Hz), 6.29 (s, 1H), 5.65 (d, 1H, *J* = 10.1 Hz), 5.03 (m, 1H), 4.55 (dd, 1H, *J* = 12.2, 2.9 Hz), 4.20 (d, 1H, *J* = 12.2 Hz), 3.79 (d, 1H, *J* = 4.0 Hz), 1.38 (s, 3H), 1.30 (s, 3H);
¹³C-NMR (acetone-d₆, 100 MHz) δ190.1, 160.8, 158.2, 148.4, 146.9, 140.7, 130.4, 129.3, 116.6, 114.9, 114.2, 112.1, 110.3, 106.2, 105.1, 78.8, 74.0, 67.2, 45.4, 29.0, 28.6;
HRMS (FAB) Calcd for C₂₁H₁₉O₆ (M+H⁺) : 367.1182, Found: 367.1179.

### Example 5: Preparation of 9,10-Dimethoxy-3,3-dimethyl-3H-chromeno[3,4-b]pyrano[2,3-h]chromen-7-(13H)-one (12)

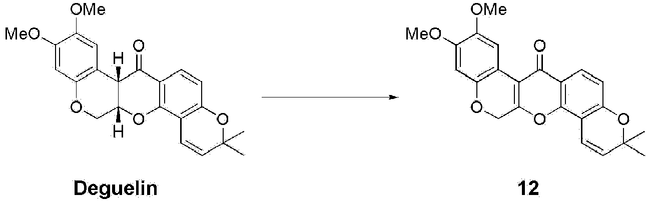

Sodium acetate (21 mg, 0.25 mmol) and I₂ (290 mg, 1.14 mmol) were added to ethyl alcohol (2.0 mL) containing deguelin (50 mg, 0.13 mmol) of Comparative Example 1 dissolved therein at room temperature. The mixture was refluxed for 12 hours, cooled down at room temperature, and extracted with EtOAc. The organic layer of the extract was washed with brine, dried over MgSO₄, and concentrated under reduced pressure. Then, the obtained residue was crystallized in EtOAc:n-hexane=1:1 solution to give the compound 12 of Example 5 as a light-yellow solid (yield: 35%, 17 mg).
¹H-NMR (CDCl₃, 500 MHz) δ 8.42 (s, 1H), 8.01 (d, 1H, *J* = 8.7 Hz), 6.84 (d, 1H, *J* = 8.7 Hz), 6.73 (d, 1H, *J* = 10.0 Hz), 6.52 (s, 1H), 5.70 (d, 1H, *J* = 8.7 Hz), 4.99 (s, 2H), 3.93 (s, 3H), 3.84 (s, 3H), 1.47 (s, 6H).;
¹³C-NMR (CDCl₃, 125 MHz) δ 174.2, 157.1, 156.1, 151.0, 148.9, 146.2, 144.0, 130.5, 126.4, 118.4, 115.3, 114.6, 111.7, 110.5, 109.9, 109.0, 100.3, 77.7, 64.8, 56.2, 55.8, 28.1, 28.1;
HRMS (FAB) Calcd for C₂₃H₂₀O₆ (M⁺) : 392.1260, Found: 392.1263.

### Example 7: Preparation of (7aS,13aS)-9,10-Dimethoxy-3,3-dimethyl-7,7a,13,13a-tetrahydro-3H-chromeno[3,4-b]pyrano[2,3-h]chromene (13)

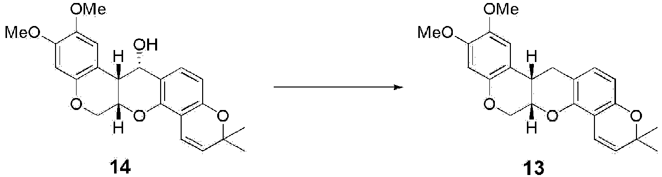

Sodium hydride (16 mg, 0.40 mmol) was added to anhydrous THF solution (2.0 mL) containing the compound 14 (40 mg, 0.10 mmol) prepared in Manufacturing Example 43 dissolved therein at 0°C under argon atmosphere, followed by stirring for 30 minutes. Carbon disulfide (0.063 mL, 1.00 mmol) was added thereto at 0°C. Iodomethane (0.064 mL, 1.00 mmol) was added to the mixture at 0°C and the temperature of the reaction mixture was raised to room temperature. After confirming the termination of the reaction by TLC, the mixture was cooled down with methanol (1.0 mL). The solvent was eliminated under reduced pressure, followed by extraction with EtOAc. The extract was washed with brine. The organic layer of the extract was dried over MgSO₄, filtered, and concentrated. The obtained non-purified residue was purified by flash column chromatography (EtOAc:n-hexane=1:6) to give the intermediate compound (yield: 71%, 35 mg). The intermediate compound (21 mg, 0.043 mmol) was dissolved in anhydrous toluene (1.0 mL), to which tri-n-butyltin hydride (0.024 mL, 0.086 mmol) and AIBN (catalytic amount) were added. While monitoring with TLC, the reaction mixture was refluxed until the reaction was completed. The solvent was eliminated under reduced pressure. Then, the obtained residue was purified by flash column chromatography (EtOAc:n-hexane=1:6) to give the compound 13 of Example 7 as a light-yellow oil (yield: 69%, 11 mg).
¹H-NMR (CDCl₃, 300 MHz) δ 6.75 (d, 1H, *J* = 8.2 Hz), 6.63 (d, 1H, *J* = 10.0 Hz), 6.62 (s, 1H), 6.38 (s, 1H), 6.31 (d, 1H, *J* = 8.2 Hz), 5.52 (d, 1H, *J* = 10.0 Hz), 5.53 (d, 1H, *J* = 10.0 Hz), 4.67 (q, 1H, *J* = 4.7 Hz), 4.24 (d, 1H, *J* = 5.4 Hz), 3.79 (s, 6H), 3.26 (m, 1H), 2.99 (m, 2H), 1.38 (s, 3H), 1.37 (s, 3H);
¹³C-NMR (CDCl₃, 75 MHz) δ 152.2, 148.9, 148.7, 147.7, 143.5, 128.9, 128.5, 116.7, 113.0, 111.4, 111.2, 109.7, 108.8, 100.6, 75.6, 69.7, 65.6, 56.6, 55.8, 31.6, 29.4, 27.8, 27.6;
HRMS (FAB) Calcd for C₂₃H₂₄O₅ (M⁺) : 380.1624, Found: 380.1631.

### Example 9: Preparation of (7S,7aR,3aS)-9,10-Dimethoxy-3,3-dimethyl-7-ethoxy-7,7a,13,13a-tetrahydro-3H-chromeno[3,4-b] pyrano[2,3-h]chromene (16)

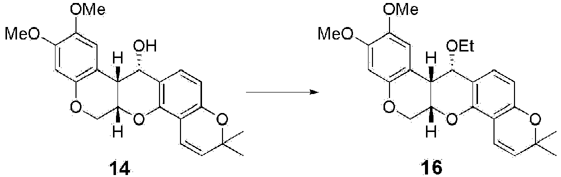

Iodoethane (1.5 equivalent) was added to anhydrous THF solution containing the compound 14 (1 equivalent) prepared in Example 7 at 0°C, to which t-BuOK solution (1 M in THF solution, 1 equivalent) was added drop by drop at 0°C. While monitoring with TLC, the reaction mixture was stirred at 0°C until the reaction was completed. The mixture was cooled down with saturated NH₄Cl aqueous solution, followed by extraction with EtOAc. The organic layer of the extract was dried over MgSO₄, filtered, and concentrated under reduced pressure. Then, the obtained residue was purified by flash column chromatography (EtOAc:n-hexane=1:3) to give the compound 16 of Example 9 as a colorless solid (yield: 71%, 23 mg).
¹H-NMR (CDCl₃, 300 MHz) δ 6.95 (d, 1H, *J* = 8.0 Hz), 6.84 (s, 1H), 6.63 (d, 1H, *J* = 9.9 Hz), 6.39 (s, 1H), 6.35 (d, 1H, *J* = 8.3 Hz), 5.53 (d, 1H, *J* = 9.9 Hz), 4.75 (m, 1H), 4.58 (t, 1H, *J* = 9.7 Hz), 4.53 (d, 1H, *J* = 3.8 Hz), 4.20 (dd, 1H, *J* = 9.9, 4.2 Hz), 3.80 (s, 3H), 3.80 (s, 3H), 3.42 (m, 2H), 3.29 (m, 1H), 1.39 (s, 3H), 1.38 (s, 3H), 1.00 (t, 3H, *J* = 6.8 Hz) ;
¹³C-NMR (CDCl₃, 75 MHz) δ 153.9, 149.1, 148.9, 148.4, 143.3, 128.9, 128.6, 116.6, 114.4, 111.7, 110.1, 109.8, 108.5, 100.3, 75.8, 75.0, 70.1, 66.0, 64.9, 56.5, 55.7, 36.8, 29.6, 27.8, 27.8;
HRMS (FAB) Calcd for C₂₅H₂₈O₆ (M⁺) : 424.1886, Found: 424.1894.

### Example 10 : Preparation of (7S,7aR,3aS)-9,10-Dimethoxy-3,3-dimethyl-7-propoxy-7,7a,13,13a-tetrahydro-3H-chromeno[3,4-b]pyrano[2,3-h]chromene (17)

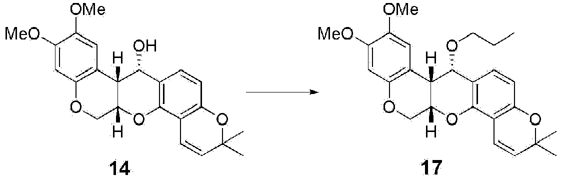

Iodopropane (1.5 equivalent) was added to anhydrous THF solution containing the compound 14 (1 equivalent) prepared in Example 7 at 0°C, to which t-BuOK solution (1 M in THF solution, 1 equivalent) was added drop by drop at 0°C. While monitoring with TLC, the reaction mixture was stirred at 0°C until the reaction was completed. The mixture was cooled down with saturated NH₄Cl aqueous solution, followed by extraction with EtOAc. The organic layer of the extract was dried over MgSO₄, filtered, and concentrated under reduced pressure. Then, the obtained residue was purified by flash column chromatography (EtOAc:n-hexane=1:5) to give the compound 17 of Example 10 as a colorless solid (yield: 68%, 15 mg).
¹H-NMR (CDCl₃, 500 MHz) δ 6.94 (d, 1H, *J* = 8.2 Hz), 6.83 (s, 1H), 6.63 (d, 1H, *J* = 9.9 Hz) 6.39 (s, 1H), 6.34 (d, 1H, *J* = 8.2 Hz), 5.53 (d, 1H, *J* = 9.9 Hz), 4.77 (m, 1H), 4.57 (t, 1H, *J* = 9.9 Hz), 4.50 (d, 1H, *J* = 3.7 Hz), 4.21 (dd, 1H, *J* = 9. 9, 4.2 Hz), 3.80 (s, 6H), 3.39 (m, 2H), 3.18 (m, 1H), 1.39 (s, 8H), 0.69 (t, 3H, *J* = 7.3 Hz);
¹³C-NMR (CDCl₃, 125 MHz) δ 153.9, 149.1, 148.9, 148.3, 143.3, 128.9, 128.7, 116.6, 114.3, 111.7, 110.2, 109.8, 108.4, 100.3, 75.8, 75.3, 71.3, 70.1, 65.9, 56.5, 55.8, 36.8, 29.6, 27.9, 27.8, 22.9;
HRMS (FAB) Calcd for C₂₆H₃₁O₆ (M+H⁺) : 439.2121, Found: 439.2120.

### Example 11: Preparation of (7S,7aR,3aS)-7-Benzyloxy-9,10-dimethoxy-3,3-dimethyl-7,7a,13,13a-tetrahydro-3H-chromeno[3,4-b]pyrano[2,3-h]chromene (18)

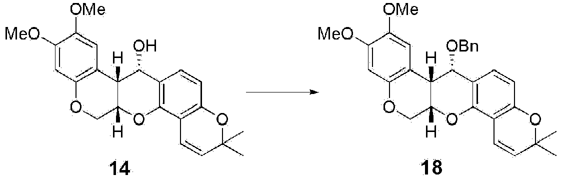

Benzylbromide (1.5 equivalent) was added to anhydrous THF solution containing the compound 14 (1 equivalent) prepared in Example 7 at 0°C, to which t-BuOK solution (1 M in THF solution, 1 equivalent) was added drop by drop at 0°C. While monitoring with TLC, the reaction mixture was stirred at 0°C until the reaction was completed. The mixture was cooled down with saturated NH₄Cl aqueous solution, followed by extraction with EtOAc. The organic layer of the extract was dried over MgSO₄, filtered, and concentrated under reduced pressure. Then, the obtained residue was purified by flash column chromatography (EtOAc:n-hexane=1:6) to give the compound 18 of Example 11 as a colorless solid (yield: 88%, 22 mg).
¹H-NMR (CDCl₃, 500 MHz) δ 7.22 (m, 3H), 7.00 (m, 2H), 6.87 (d, 1H, *J* = 8.2 Hz), 6.66 (d, 1H, *J* = 9.9 Hz), 6.62 (s, 1H), 6.45 (s, 1H), 6.37 (d, 1H, *J* = 8.2 Hz), 5.56 (d, 1H, *J* = 9.9 Hz), 4.81 (m, 1H), 4.62 (t, 1H, *J* = 9.9 Hz), 4.54 (d, 1H, *J* = 3.2 Hz), 4.48 (AB quartet, 2H, *J* = 85.0, 12.5 Hz), 4.25 (dd, 1H, *J* = 14.2, 4.6 Hz), 3.84(s, 3H), 3.72 (s, 3H), 3.36 (m, 1H), 1.42 (s, 3H), 1.40 (s, 3H);
¹³C-NMR (CDCl₃, 125 MHz) δ 154.2, 149.1, 148.9, 148.7, 143.4, 137.9, 129.2, 129.0, 128.1, 127.6, 127.4, 127.2, 116.5, 113.8, 111.3, 110.1, 110.0, 108.4, 100.4, 75.9, 72.8, 69.9, 69.8, 65.8, 64.9, 56.2, 55.8, 37.3, 27.9, 27.8;
HRMS (FAB) Calcd for C₃₀H₃₀O₆ (M⁺) : 486.2042, Found: 486.2050.

### Example 12 : Preparation of (7S,7aS,13aS)-9,10-Dimethoxy-3,3-dimethyl-7-(tetrahydro-2H-pyran-2-yloxy)-7,7a,13,13a-tetrahydro-3H-chromeno[3,4-b]pyrano[2,3-h]chromene (19)

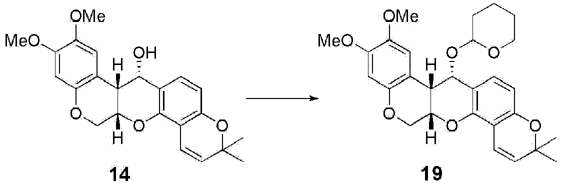

to *p*-toluenesulfonate (5.8 mg, 0.023 mmol) was added to CH₂Cl₂ (1.0 mL) containing the compound 14 (30 mg, 0.076 mmol) prepared in Example 7 and DHP (13 mg, 0.15 mmol) dissolved therein, followed by stirring at room temperature for 1 hour. The reaction mixture was cooled down with water (0.5 mL), followed by extraction with EtOAc. The organic layer of the extract was dried over MgSO₄, filtered, and concentrated under reduced pressure. Then, the obtained residue was purified by flash column chromatography (EtOAc:n-hexane=1:4) to give the compound 19 of Example 12 as a colorless oil (yield: 50%, diastereomer mixture, 18 mg).
¹H-NMR (CDCl₃, 500 MHz) δ 7.06 (d, 1H, *J* = 8.2 Hz), 6.91 (d, 1H, *J* = 8.2 Hz), 6.84 (s, 1H), 6.72 (s, 1H), 6.63 (t, 1H, *J* = 9.9 Hz), 6.42 (s, 1H), 6.69 (s, 1H), 6.37 (d, 1H, *J* = 8.4 Hz), 6.33 (d, 1H, *J* = 8.2 Hz), 5.54 (t, 1H, *J* = 9.3 Hz), 4.85 (m, 4H), 4.75 (m, 1H), 4.63 (t, 1H, *J* = 10.0 Hz), 4.55 (t, 1H, *J* = 10.0 Hz), 4.33 (m, 1H), 4.21 (m, 2H), 3.81 (s, 3H), 3.80 (s, 3H), 3.79 (s, 3H), 3.78 (s, 3H), 3.70 (m, 1H), 3.40 (m, 3H), 3.29 (m, 1H), 3.11 (m, 1H), 1.71-1.19 (m, 12H), 1.40 (s, 12H);
¹³C-NMR (CDCl₃, 125 MHz) δ 154.2, 153.7, 149.2, 149.1, 149.0, 148.9, 148.7, 148.7, 148.2, 148.2, 143.3, 143.2, 116.5, 116.4, 113.4, 113.1, 111.8, 111.7, 110.2, 110.0, 109.6, 109.6, 109.3, 109.3, 109.1, 108.2, 100.3, 100.2, 99.1, 93.2, 75.9, 75.8, 72.4, 69.8, 69.4, 69.1, 65.8, 65.2, 61.6, 60.4, 56.5, 56.5, 55.8, 55.8, 37.2, 36.8, 30.3, 30.2, 28.0, 27.9, 27.8, 27.7, 25.4, 25.3, 18.7, 18.1;
HRMS (FAB) Calcd for C₂₈H₃₂O₇ (M⁺): 480.2148, Found: 480.2155.

### Example 13 : Preparation of (7S,7aS,13aS)-9,10-Dimethoxy-3,3-dimethyl-7,7a,13,13a-tetrahydro-3H-chromeno[3,4-b]pyrano[2,3,h]chromen-7-yl acetate (20)

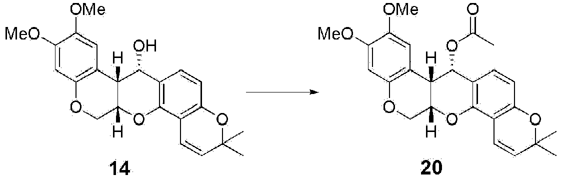

Et₃N (0.012 mL, 0.086 mmol) and acetic anhydride (0.06 mL, 0.64 mmol) were added to CH₂Cl₂ solution (1.0 mL) containing the compound 14 (30 mg, 0.076 mmol) prepared in Example 7 and DMAP (catalytic amount) dissolved therein at 0°C under argon atmosphere. The reaction mixture was stirred at room temperature for 10 minutes. The reaction mixture was cooled down with saturated NH₄Cl aqueous solution, followed by extraction with CH₂Cl₂. The organic layer of the extract was washed with brine, dried over MgSO₄, and concentrated. Then, the obtained residue was purified by flash column chromatography (EtOAc:n-hexane=1:5) to give the compound 20 of Example 13 as a white solid (yield: 82%, 27 mg).
¹H-NMR (CDCl₃, 500 MHz) δ 7.01 (d, 1H, *J* = 8.3 Hz), 6.63 (s, 1H), 6.63 (d, 1H, *J* = 9.9 Hz), 6.38 (s, 1H), 6.38 (d, 1H, *J* = 7.5 Hz), 6.24 (d, 1H, *J* = 4.4 Hz), 5.56 (d, 1H, *J* = 9.9 Hz), 4.86 (m, 1H), 4.43 (t, 1H, *J* = 10.2 Hz), 4.24 (m, , 1H), 3.81 (s, 6H), 3.49 (m, 1H), 1.71 (s, 3H), 1.40 (s, 6H);
¹³C-NMR (CDCl₃, 125 MHz) δ170.0, 154.6, 149.3, 148.6, 148.5, 143.4, 130.5, 129.1, 116.2, 111.7, 110.9, 109.7, 109.6, 108.6, 100.1, 76.1, 69.0, 66.7, 64.5, 56.4, 55.8, 36.4, 27.9, 27.8, 20.8;
HRMS (FAB) Calcd for C₂₅H₂₆O₇ (M⁺) : 438.1679, Found: 438.1681.

### Example 15: Preparation of (13aS)-9,10-Dimethoxy-3,3-dimethyl-13,13a-dihydro-3H-chromeno[3,4-b]pyrano[2,3-h]chromene (22)

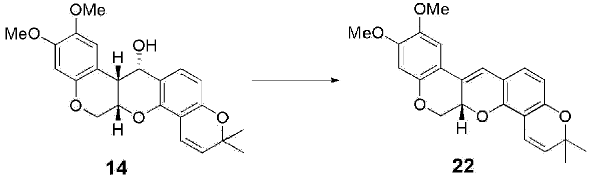

Acetic acid solution (1.0 mL) containing the compound 14 (3 mg, 7.6 µmol) prepared in Example 7 dissolved therein was stirred at 100 °C for 2 hours, which was then treated with water (2.0 mL). The mixture was extracted with diethyl ether, and the water layer was extracted again with diethyl ether. The organic layer was washed with brine, dried over MgSO₄, filtered, and concentrated under reduced pressure. Then, the obtained residue was purified by flash column chromatography (EtOAc:n-hexane=1:7) to give the compound 22 of Example 15 as a colorless oil (yield: 70%, 2 mg).
¹H-NMR (CDCl₃, 400 MHz) δ 6.98 (s, 1H), 6.81 (d, 1H, *J* = 8.2 Hz), 6.60 (d, 1H, *J* = 10.0 Hz), 6.55 (s, 1H), 6.40 (s, 1H), 6.36 (d, 1H, *J* = 8.2 Hz), 5.59 (d, 1H, *J* = 10.0 Hz), 5.27 (m, 1H), 4.57 (dd, 1H, *J* = 10.0, 5.4 Hz), 4.13 (t, 1H, *J* = 10.0 Hz), 3.88 (s, 3H), 3.83 (s, 3H), 1.42 (s, 3H), 1.38 (s, 3H);
¹³C-NMR (CDCl₃, 100 MHz) δ 153.3, 150.3, 149.2, 148.2, 144.7, 129.7, 126.4, 123.7, 116.6, 116.2, 111.8, 110.6, 109.8, 109.6, 105.2, 100.9, 76.1, 71.1, 67.9, 56.3, 55.9, 28.0, 27.6;
HRMS (FAB) Calcd for C₂₃H₂₂O₅ (M⁺) : 378.1467, Found: 378.1474.

### Example 16: Preparation of (7aR,13aS)-9,10-Dimethoxy-3,3-dimethyl-13,13a-dihydro-3H-chromeno[3,4-b]pyrano[2,3-h]chromen-7(7aH)-one oxime (23)

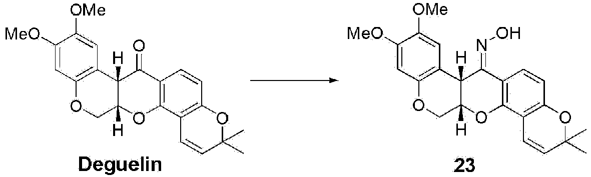

Hydroxylamine hydrochloride (14 mg, 0.19 mmol) was added to anhydrous pyridine (1.0 mL) containing deguelin (25 mg, 0.063 mmol) prepared in Comparative Example 1. The reaction temperature was increased to 70 °C. While monitoring with TLC, the reaction mixture was stirred until the reaction was completed. The mixture was cooled down with water (0.5 mL), followed by extraction with CH₂Cl₂. The organic layer of the extract was washed with 2 N-HCl aqueous solution, water, and brine stepwise. The solvent was eliminated under reduced pressure and the residue was dried over MgSO₄, filtered, and concentrated. Then, the obtained residue was purified by flash column chromatography (EtOAc:n-hexane=1:2) to give the compound 23 of Example 16 as a white solid (26 mg).
¹H-NMR (CDCl₃, 500 MHz) δ 8.26 (br, 1H), 7.59 (d, 1H, *J* = 8.7 Hz), 6.62 (m, 2H), 6.41 (s, 1H), 6.37 (d, 1H, *J* = 8.7 Hz), 5.49 (d, 1H, *J* = 10.0 Hz), 4.84 (d, 1H, *J* = 3.2 Hz), 4.61 (dd, 1H, *J* = 12.0, 2.4 Hz), 4.48 (m, 1H), 4.24 (d, 1H, *J* = 12.0 Hz), 3.79 (s, 3H), 3.72 (s, 3H), 1.39 (s, 3H), 1.34 (s, 3H);
¹³C-NMR (CDCl₃, 125 MHz) δ155.7, 151.6, 151.3, 149.3, 147.7, 143.6, 128.6, 124.5, 116.2, 112.2, 110.7, 109.8, 108.2, 106.2, 100.6, 76.5, 69.6, 66.8, 56.4, 55.8, 31.6, 28.1, 27.8;
HRMS (FAB) Calcd for C₂₃H₂₃NO₆ (M⁺): 409.1525, Found: 409.1513.

### Example 17: Preparation of (7aR,13aS)-9,10-Dimethoxy-3,3-dimethyl-13,13a-dihydro-3H-chromeno[3,4-b]pyrano[2,3-h]chro men-7(7aH)-one O-methyloxime (24)

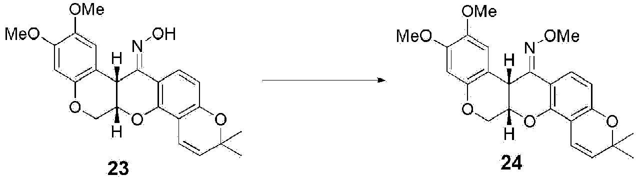

Iodomethane (1.5 equivalent) was added to anhydrous THF solution containing the compound 23 (1 equivalent) prepared in Example 16 at 0°C, to which t-BuOK solution (1 M in THF solution, 1 equivalent) was added drop by drop at 0°C. While monitoring with TLC, the reaction mixture was stirred at 0°C until the reaction was completed. The mixture was cooled down with saturated NH₄Cl aqueous solution, followed by extraction with EtOAc. The organic layer of the extract was dried over MgSO₄, filtered, and concentrated under reduced pressure. Then, the obtained residue was purified by flash column chromatography (EtOAc:n-hexane=1:4) to give the compound 24 of Example 17 as a white solid (yield: 57%, 12 mg).
¹H-NMR (CDCl₃, 500 MHz) δ 7.68 (d, 1H, *J* = 8.7 Hz), 6.61 (d, 1H, *J* = 10.0 Hz), 6.51 (s, 1H), 6.38 (m, 2H), 5.48 (d, 1H, *J* = 10.0 Hz), 4.71 (d, 1H, *J* = 3.3 Hz), 4.59 (dd, 1H, *J* = 12.0, 2.4 Hz), 4.45 (m, 1H), 4.21 (d, 1H, *J* = 12.0 Hz), 4.03 (s, 3H), 3.77 (s, 3H), 3.73 (s, 3H), 1.39 (s, 3H), 1.33 (s, 3H);
¹³C-NMR (CDCl₃, 125 MHz) δ155.6, 151.2, 150.0, 149.2, 147.6, 143.5, 128.5, 124.7, 116.3, 111.9, 110.6, 109.7, 108.4, 106.3, 100.5, 76.5, 69.6, 66.9, 61.9, 56.3, 55.8, 32.3, 28.1, 27.8;
HRMS (FAB) Calcd for C₂₄H₂₅NO₆ (M⁺) : 423.1682, Found: 423.1677.

### Example 18: Preparation of (7aR,13aS)-9,10-Dimethoxy-3,3-dimethyl-13,13a-dihydro-3H-chromeno[3,4-b]pyrano[2,3-h]chro men-7(7aH)-one O-benzyloxime (25)

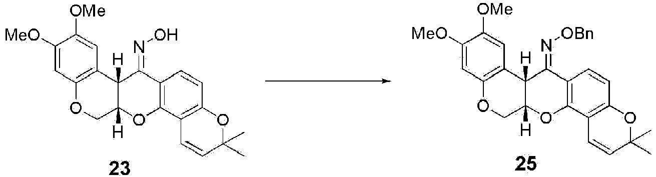

Benzylbromide (1.5 equivalent) was added to anhydrous THF solution containing the compound 23 (1 equivalent) prepared in Example 16 at 0°C, to which t-BuOK solution (1 M in THF solution, 1 equivalent) was added drop by drop at 0°C. While monitoring with TLC, the reaction mixture was stirred at 0°C until the reaction was completed. The mixture was cooled down with saturated NH₄Cl aqueous solution, followed by extraction with EtOAc. The organic layer of the extract was dried over MgSO₄, filtered, and concentrated under reduced pressure. Then, the obtained residue was purified by flash column chromatography (EtOAc:n-hexane=1:5) to give the compound 25 of Example 18 as a white solid (yield: 33%, 11 mg).
¹H-NMR (CDCl₃, 500 MHz) δ 7.71 (d, 1H, *J* = 8.7 Hz), 7.46 (d, 2H, *J* = 7.1 Hz), 7.31 (m, 3H), 6.62 (d, 1H, *J* = 10.0 Hz), 6.46 (s, 1H), 6.37 (m, 2H), 5.48 (d, 1H, *J* = 10.0 Hz), 5.27 (AB quartet, 2H, *J* = 50.7, 12.0 Hz), 4.76 (d, 1H, *J* = 3.0 Hz), 4.58 (dd, 1H, *J* = 12.0, 2.2 Hz), 4.44 (m, 1H), 4.20 (d, 1H, *J* = 12.0 Hz), 3.78 (s, 3H), 3.48(s, 3H), 1.40 (s, 3H), 1.33 (s, 3H);
¹³C-NMR (CDCl₃, 125 MHz) δ 155.6, 151.2, 150.1, 149.1, 147.5, 143.5, 137.5, 128.7, 128.7, 128.5, 128.4, 128.4, 128.1, 124.6, 116.3, 111.7, 110.6, 109.7, 108.5, 106.4, 100.5, 76.5, 76.5, 69.6, 66.8, 56.0, 55.7, 32.3, 28.1, 27.8;
HRMS (FAB) Calcd for C₃₀H₂₉NO₆ (M⁺) : 499.1995, Found: 499.1999.

### Example 20: Preparation of (7aS,13aS)-1,2-Dihydroxy-9,10-dimethoxy-3,3-dimethyl-2,3,13,13a-tetrahydro-1H-chromeno[3,4-b] pyrano[2,3-h]chromen-7(7aH)-one (27)

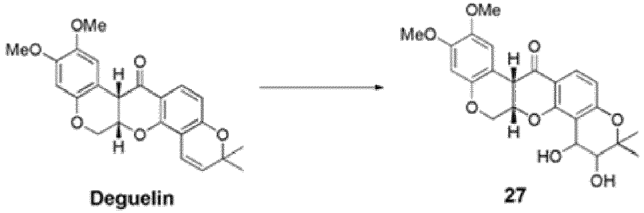

N-methylmopholine N-oxide (NMO, 18 mg, 0.15 mmol) and O_{S}O₄ (0.1 M in toluene, 0.020 mL, 0.002 mmol) were added to the mixed solution of acetone:water (4.0 mL, 4:1) containing deguelin (20 mg, 0.051 mmol) prepared in Comparative Example 1, followed by stirring at 0°C for 10 minutes. The mixture was warmed up at room temperature, followed by stirring for 3 days. The mixture was cooled down with sulfite aqueous solution at 0°C, filtered with celite pad, and washed with EtOAc. The organic layer was extracted with EtOAc, dried over MgSO₄, filtered, and concentrated under reduced pressure. Then, the obtained residue was purified by flash column chromatography (EtOAc:n-hexane=2:1 ∼ EtOAc 100%) to give the compound 27 of Example 20 as a light-yellow solid (yield: 47%, diastereomer mixture, 10 mg).
(1) isomer A: ¹H-NMR (CDCl₃, 400 MHz) δ 7.76 (d, 1H, *J* = 8.8 Hz), 6.48 (m, 3H), 4.98 (d, 1H, *J =* 4.7 Hz), 4.46 (m, 3H), 4.42 (s, 1H), 3.80 (s, 3H), 3.76 (t, 1H, *J* = 4.7 Hz), 3.71 (s, 3H), 3.45 (br, 1H), 3.34 (d, 1H, *J* = 4.5 Hz), 1.40 (s, 3H), 1.31 (s, 3H); ¹³C-NMR (CDCl₃, 100 MHz) δ 190.7, 160.6, 160.4, 151.2, 148.2, 144.3, 128.7, 113.0, 110.5, 110.0, 109.1, 108.6, 101.1, 79.1, 76.3, 70.4, 67.3, 63.7, 61.9, 56.3, 55.9, 24.3, 22.5;
(2) isomer B: ¹H-NMR (CDCl₃, 400 MHz) δ 7.77 (d, 1H, *J* = 8.8 Hz), 6.47 (m, 3H), 4.97 (d, 1H, *J* = 4.7 Hz), 4.59 (m, 3H), 4.43 (s, 1H), 3.80 (s, 3H), 3.73 (m, 1H), 3.70 (s, 3H), 3.53 (br, 1H), 3.30 (d, 1H, *J* = 4.5 Hz), 1.43 (s, 3H), 1.27 (s, 3H); ¹³C-NMR (CDCl₃, 100 MHz) δ 190.8, 160.8, 160.6, 151.2, 148.2, 144.1, 128.8, 113.2, 110.8, 109.7, 109.2, 108.4, 101.0, 79.0, 76.7, 70.3, 67.2, 63.8, 62.0, 56.3, 55.8, 24.5, 22.5.

### Example 21: Preparation of 2,3,9-Trimethoxy-8-(3-methyl-but-2-enyl)-6a,12a-dihydro-6H-chromeno[3,4-b]chromen-12-one (28)

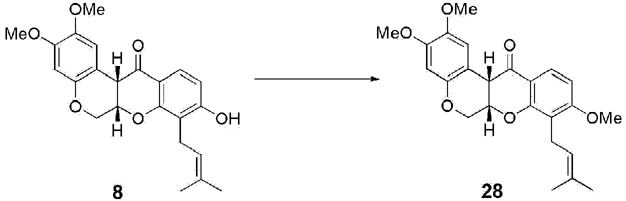

Cesium carbonate (1.5 equivalent) was added to anhydrous acetonitrile solution containing the compound 8 (1 equivalent) prepared in Example 1 and iodomethane (2 equivalent). While monitoring with TLC, the reaction mixture was stirred at 0°C until the reaction was completed. The mixture was cooled down with water, followed by extraction with EtOAc. The organic layer of the extract was washed with brine, dried over MgSO₄, and concentrated under reduced pressure. Then, the obtained residue was purified by flash column chromatography (EtOAc:n-hexane=1:4) to give the compound 28 of Example 21 as a white solid (yield: 41%, 16 mg).
¹H-NMR (CDCl₃, 500 MHz) δ 7.80 (d, 1H, *J* = 8.8 Hz), 6.74 (s, 1H), 6.56 (d, 1H, *J* = 8.9 Hz), 6.41 (s, 1H), 5.11 (m, 1H), 4.87 (m, 1H), 4.58 (dd, 1H, *J* = 11.9, 3.3 Hz), 4.15 (d, 1H, *J* = 11.9 Hz), 3.83 (s, 3H), 3.81 (d, 1H, *J* = 4.1 Hz), 3.77 (s, 3H), 3.74 (s, 3H), 3.28 (m, 2H), 1.74 (s, 3H), 1.61 (s, 3H);
¹³C-NMR (CDCl₃, 125 MHz) δ 190.0, 163.6, 159.2, 149.3, 147.4, 143.6, 131.8, 126.9, 121.6, 117.4, 113.1, 110.3, 105.1, 104.6, 100.7, 71.8, 66.2, 56.2, 55.8, 55.8, 44.3, 25.7, 21.9, 17.6;
HRMS (FAB) Calcd for C₂₄H₂₆O₆ (M⁺): 410.1732, Found: 410.1735.

### Example 22: Preparation of 9-Allyloxy-2,3-dimethoxy-8-(3-methyl-but-2-enyl)-6a,12a-dihydro-6H-chromeno[3,4-b]chromen-12-one (29)

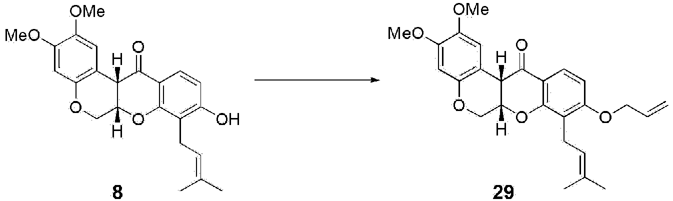

Cesium carbonate (1.5 equivalent) was added to anhydrous acetonitrile solution containing the compound 8 (1 equivalent) prepared in Example 1 and aryliodide (2 equivalent). While monitoring with TLC, the reaction mixture was stirred at 0°C until the reaction was completed. The mixture was cooled down with water, followed by extraction with EtOAc. The organic layer of the extract was washed with brine, dried over MgSO₄, and concentrated under reduced pressure. Then, the obtained residue was purified by flash column chromatography (EtOAc:n-hexane=1:6) to give the compound 29 of Example 22 as a light-yellow solid (yield: 39%, 12 mg).
¹H-NMR (CDCl₃, 400 MHz) δ 7.78 (d, 1H, *J* = 8.9 Hz), 6.75 (s, 1H), 6.53 (d, 1H, *J* = 8.9 Hz), 6.42 (s, 1H), 5.98 (m, 1H), 5.30 (m, 2H), 5.15 (m, 1H), 4.88 (m, 1H), 4.58 (m, 3H), 4.16 (d, 1H, *J* = 11.8 Hz), 3.81 (d, 1H, *J* = 2.0 Hz), 3.78 (s, 3H), 3.74 (s, 3H), 3.33 (m, 2H), 1.74 (s, 3H), 1.62 (s, 3H);
¹³C-NMR (CDCl₃, 100 MHz) δ 190.0, 162.6, 159.4, 149.4, 147.5, 143.7, 132.5, 131.7, 126.8, 121.6, 117.8, 117.6, 113.2, 110.5, 106.2, 104.7, 100.8, 72.0, 69.0, 66.3, 56.3, 55.8, 44.4, 25.7, 22.1, 17.8;
HRMS (FAB) Calcd for C₂₆H₂₈O₆ (M⁺) : 436.1886, Found: 436.1883.

### Example 23: Preparation of 9-Benzyloxy-2,3-dimethoxy-8-(3-methyl-but-2-enyl)-6a,12a-dihydro-6H-chromeno[3,4-b]chromen-12-one (30)

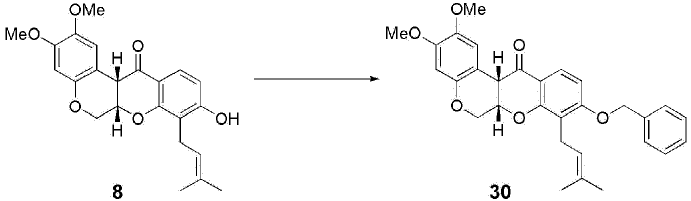

Cesium carbonate (1.5 equivalent) was added to anhydrous acetonitrile solution containing the compound 8 (1 equivalent) prepared in Example 1 and benzylbromide (2 equivalent). While monitoring with TLC, the reaction mixture was stirred at 0°C until the reaction was completed. The mixture was cooled down with water, followed by extraction with EtOAc. The organic layer of the extract was washed with brine, dried over MgSO₄, and concentrated under reduced pressure. Then, the obtained residue was purified by flash column chromatography (EtOAc:n-hexane=1:6) to give the compound 30 of Example 23 as a white solid (yield: 25%, 12 mg).
¹H-NMR (CDCl₃, 400 MHz) δ 7.78 (d, 1H, *J =* 8.8 Hz), 7.31 (m, 5H), 6.74 (s, 1H), 6.60 (d, 1H, *J* = 8.8 Hz), 6.41 (s, 1H), 5.15 (m, 3H), 4.48 (m, 1H), 4.61 (dd, 1H, *J* = 11.9, 3.0 Hz), 4.16 (d, 1H, *J* = 11.9 Hz), 3.81 (d, 1H, *J* = 4.1 Hz), 3.78 (s, 3H), 3.73 (s, 3H), 3.35 (m, 2H), 1.66 (s, 3H), 1.61 (s, 3H);
¹³C-NMR (CDCl₃, 75 MHz) δ 190.0, 162.7, 159.3, 149.4, 147.5, 143.6, 136.3, 131.8, 129.7, 128.6, 128.0, 127.1, 126.9, 122.8, 121.7, 117.9, 113.4, 110.5, 106.3, 104.7, 100.8, 72.0, 70.3, 66.3, 56.3, 55.8, 44.4, 25.7, 22.2, 17.7;
HRMS (FAB) Calcd for C₃₀H₃₀O₆ (M+H⁺) : 487.2121, Found: 487.2113.

### Example 24: Preparation of acetic acid 2,3-dimethoxy-8-(3-methyl-but-2-enyl)-12-oxo-6,6a,12,12a-tetrahydrochromeno [3,4-b]chromen-9-yl ester (31)

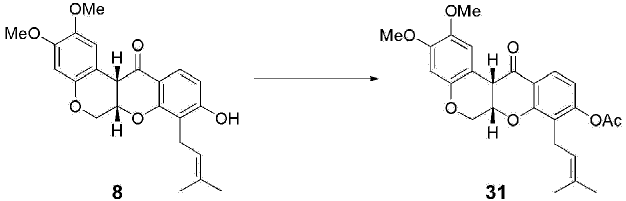

Triethylamine (0.01 mL, 0.07 mmol) and acetic anhydride (0.05 mL, 0.53 mmol) were added to CH₂Cl₂ solution (1.0 mL) containing the compound 8 (25 mg, 0.063 mmol) prepared in Example 1 and DMAP (catalytic amount) at 0°C under argon atmosphere. The mixture was stirred at room temperature for 10 minutes. The mixture was cooled down with saturated NH₄Cl aqueous solution, followed by extraction with CH₂Cl₂. The organic layer of the extract was washed with brine, dried over MgSO₄, and concentrated under reduced pressure. Then, the obtained residue was purified by flash column chromatography (EtOAc:n-hexane=1:4) to give the compound 31 of Example 24 as a light-yellow oil (yield: 44%, 14 mg).
¹H-NMR (CDCl₃, 400 MHz) δ 7.80 (d, 1H, *J* = 8.6 Hz), 6.70 (d, 1H, *J* = 8.6 Hz), 6.68 (s, 1H), 6.42 (s, 1H), 5.02 (m, 1H), 4.93 (m, 1H), 4.60 (dd, 1H, *J* = 12.0, 3.2 Hz), 4.17 (d, 1H, *J* = 12.0 Hz), 3.87 (d, 1H, *J* = 4.0 Hz), 3.78 (s, 3H), 3.74 (s, 3H), 3.24 (m, 2H), 2.27 (s, 3H), 1.72 (s, 3H), 1. 62 (s, 3H);
¹³C-NMR (CDCl₃, 75 MHz) δ 190.1, 168.6, 159.8, 154.9, 149.7, 147.6, 143.9, 132.5, 125.9, 123.0, 120.7, 116.4, 110.5, 104.0, 100.9, 72.3, 66.1, 56.3, 55.8, 44.4, 29.6, 25.6, 23.0, 20.8, 17.7;
HRMS (FAB) Calcd for C₂₅H₂₆O₇ (M⁺) : 438.1679, Found: 438.1681.

### Example 25: Preparation of (3S)-3-(3,4-Dimethoxyphenyl)-8,8-dimethyl-2,3-dihydro-4H,8H-pyrano[2,3-f]chromen-4-one (37)

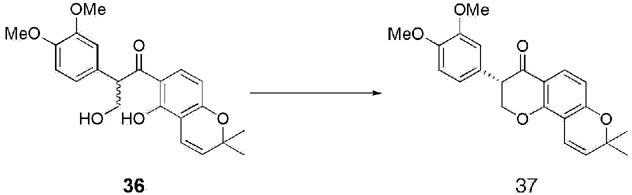

Anhydrous THF solution (0.5 mL) containing triphenylphosphine (65 mg, 0.25 mmol) and diisoppropylazodicarboxylate (0.024 mL, 0.12 mmol) was added to anhydrous THF (1.0 mL) containing the compound 36 (10 mg, 0.026 mmol) prepared in Manufacturing Example 5 under nitrogen atmosphere. While monitoring with TLC, the reaction mixture was stirred until the reaction was completed. The reaction mixture was concentrated under reduced pressure and the obtained residue was purified by flash column chromatography (EtOAc:n-hexane=1:4) to give the compound 37 of Example 25 as a white solid (yield: 58%, 5.5 mg).
¹H NMR (CDCl₃, 500 MHz) δ 7.74 (d, 1H, *J* = 8.7 Hz), 6.82 (s, 2H), 6.77 (s, 1H), 6.61 (d, 1H, *J* = 10.0 Hz), 6.47 (d, 1H, *J* = 8.7 Hz), 5.58 (d, 1H, *J* = 10.0 Hz), 4.63 (m, 2H), 3.83 (s, 3H), 3.83 (s, 3H), 1.44 (s, 6H);
¹³C NMR (CDCl₃, 125 MHz) δ 190.9, 159.5, 157.6, 149.1, 148.5, 128.9, 128.6, 127.8, 120.6, 115.6, 114.7, 111.8, 111.4, 111.2, 109.1, 77.5, 55.8, 51.3, 29.6, 28.2, 28.2, 21.7;
HRMS (FAB) Calcd for C₂₂H₂₃O₅ (M+H⁺) : 367.1545, Found: 367.1552.

### Example 26: Preparation of (6,7-Dimethoxychroman-4-yl)(2,2-dimethyl-2H-chromen-6-yl)methanone (45)

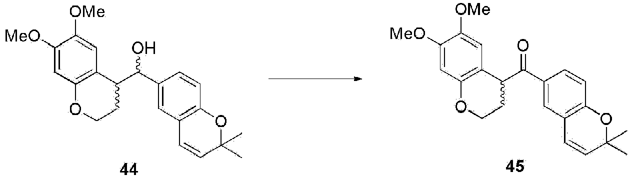

Dess-Martin periodinane (3.0 equivalent) was added to CH₂Cl₂ (0.03 M) solution containing the compound 44 (1.0 equivalent) obtained in Manufacturing Example 12, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:6) to give the compound 45 of Example 26 (yield: 55%, 9.9 mg).
¹H NMR (CDCl₃, 500 MHz) δ 7.79 (dd, 1H, *J* = 8.5, 2.1 Hz), 7.66 (d, 1H, *J* = 1.9 Hz), 6.81 (d, 1H, *J* = 8.4 Hz), 6.42 (s, 1H), 6.34 (m, 2H), 5.66 (d, 1H, *J* = 9.9 Hz), 4.61 (t, 1H, *J* = 5.8 Hz), 4.17 (m, 2H), 3.81 (s, 3H), 3.65 (s, 3H), 2.21 (m, 2H), 1.45 (s, 6H);
¹³C NMR (CDCl₃, 125 MHz) δ 199.6, 157.7, 149.4, 149.2, 143.3, 131.3, 130.5, 129.0, 127.4, 121.5, 120.9, 116.3, 112.4, 110.6, 101.1, 77.7, 63.4, 56.4, 55.8, 41.6, 28.4, 28.4, 26.5;
HRMS (FAB) Calcd for C₂₃H₂₄O₅ (M⁺): 380.1624, Found: 380.1621.

### Example 27: Preparation of 2-(3,4-Dimethoxyphenyl)-1-(2,2-dimethyl-2H-chromen-6-yl)ethanone (53)

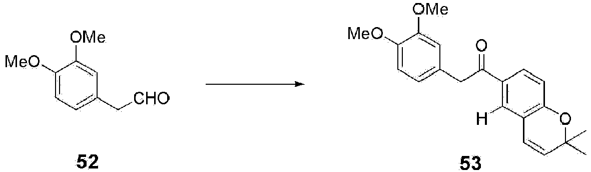

n-BuLi (1.4 equivalent) was added to anhydrous THF solution containing aryl bromide 6-bromo-2,2-dimethyl-2*H*-chromene (1.5 equivalent) drop by drop at -78 °C, which was stirred at -78 °C to generate aryl anions. The mixture was stirred at -78 °C for 20 minutes, to which the compound 52 (1.0 equivalent) prepared in Manufacturing Example 13 was added, followed by stirring for 30 minutes with raising the reaction temperature to room temperature. The reaction mixture was treated with saturated NH₄Cl aqueous solution, followed by extraction with EtOAc. The extract was washed with brine and then dried over MgSO₄. The residue obtained after evaporating the solvent was purified by flash column chromatography (EtOAc:n-hexane=1:2) to give the secondary alcohol.

Dess-Martin periodinane (3.0 equivalent) was added to CH₂Cl₂ (0.03 M) solution containing the secondary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:3) to give the compound 53 of Example 27 as a light-yellow solid (yield: 46%, 37 mg).
¹H NMR (CDCl₃, 400 MHz) δ 7.76 (dd, 1H, *J =* 8.4, 2.2 Hz), 7.62 (d, 1H, *J* = 2.2 Hz), 6.76 (m, 4H), 6.29 (d, 1H, *J* = 9.8 Hz), 5.61 (d, 1H, *J* = 9.8 Hz), 4.10 (s, 2H), 3.80 (s, 3H), 3.79 (s, 3H), 1.40 (s, 6H);
¹³C NMR (CDCl₃, 100 MHz) δ 196.3, 157.3, 148.8, 147.7, 131.0, 130.4, 129.4, 127.3, 127.1, 121.5, 121.4, 120.6, 116.0, 112.3, 111.2, 77.4, 55.7, 55.7, 44.5, 28.2 28.2;
HRMS (FAB) Calcd for C₂₁H₂₃O₄ (M+H⁺): 339.1596, Found: 339.1595.

### Example 28: Preparation of 2-(3,4-Dimethoxyphenyl)-1-(5-methoxy-2,2-dimethyl-2H-chromen-6-yl)ethanone (54)

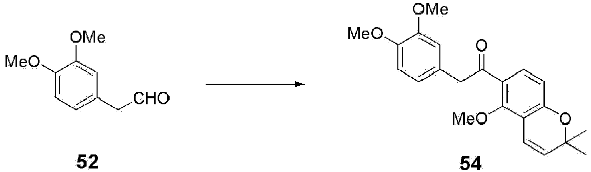

n-BuLi (1.4 equivalent) was added to anhydrous THF solution containing the compound 51 (1.5 equivalent) prepared in Manufacturing Example 27 drop by drop at -78 °C, which was stirred at -78 °C to generate aryl anions. The mixture was stirred at - 78 °C for 20 minutes, to which the compound 52 (1.0 equivalent) prepared in Manufacturing Example 13 was added, followed by stirring for 30 minutes with raising the reaction temperature to room temperature. The reaction mixture was treated with saturated NH₄Cl aqueous solution, followed by extraction with EtOAc. The extract was washed with brine and then dried over MgSO₄. The residue obtained after evaporating the solvent was purified by flash column chromatography (EtOAc:n-hexane=1:2) to give the secondary alcohol.

Dess-Martin periodinane (3.0 equivalent) was added to CH₂Cl₂ (0.03 M) solution containing the secondary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:4) to give the compound 54 of Example 28 as a light-yellow solid (yield: 48%, 80 mg).
¹H NMR (CDCl₃, 300 MHz) δ 7.43 (d, 1H, *J* = 8.4 Hz), 6.72 (s, 3H), 6.54 (d, 1H, *J* = 9.9 Hz), 6.52 (d, 1H, *J* = 8.4 Hz), 5.62 (d, 1H, *J* = 9.9 Hz), 4.12 (s, 2H), 3.77(s, 6H), 3.69(s, 3H), 1.37(s, 6H);
¹³C NMR (CDCl₃, 75 MHz) δ 198.7, 157.7, 156.3, 148.8, 147.8, 131.1, 130.5, 127.6, 124.8, 121.7, 116.5, 114.8, 112.8, 112.6, 111.2, 63.2, 55.8, 55.8, 50.3, 50.2, 28.1, 28.0;
HRMS (FAB) Calcd for C₂₂H₂₅O₅ (M+H⁺) : 369.1702, Found: 369.1705.

### Example 29: Preparation of 2-(3,4-Dimethoxyphenyl)-1-(5-methoxy-2,2-dimethyl-2H-chromen-6-yl)propan-1-one (56)

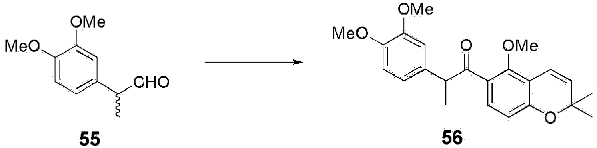

*n*-BuLi (1.4 equivalent) was added to anhydrous THF solution containing the compound 51 (1.5 equivalent) prepared in Manufacturing Example 27 drop by drop at -78 °C, which was stirred at -78 °C to generate aryl anions. The mixture was stirred at - 78 °C for 20 minutes, to which the compound 55 (1.0 equivalent) prepared in Manufacturing Example 14 was added, followed by stirring for 30 minutes with raising the reaction temperature to room temperature. The reaction mixture was treated with saturated NH₄Cl aqueous solution, followed by extraction with EtOAc. The extract was washed with brine and then dried over MgSO₄. The residue obtained after evaporating the solvent was purified by flash column chromatography (EtOAc:n-hexane=1:2) to give the secondary alcohol.

Dess-Martin periodinane (3.0 equivalent) was added to CH₂Cl₂ (0.03 M) solution containing the secondary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:4) to give the compound 56 of Example 29 as a light-yellow solid (yield: 64%, 11 mg).
¹H NMR (CDCl₃, 500 MHz) δ 7.27 (d, 1H, *J* = 8.5 Hz), 6.76 (m, 3H), 6.55 (d, 1H, *J* = 10.0 Hz), 6.48 (d, 1H, *J* = 8.5 Hz), 5.62 (d, 1H, *J* = 10.0 Hz), 4.60 (q, 1H, *J* = 6.9 Hz), 3.81 (s, 3H), 3.80 (s, 3H), 3.65 (s, 3H), 1.48 (d, 3H, *J* = 6.9 Hz), 1.39 (s, 6H);
¹³C NMR (CDCl₃, 75 MHz) δ 202.5, 157.1, 155.7, 149.0, 147.9, 133.8, 130.7, 130.5, 125.5, 120.2, 116.5, 112.3, 111.2, 77.2, 76.7, 63.3, 55.8, 55.7, 50.0, 43.1, 28.0, 28.0, 19.0;
HRMS (FAB) Calcd for C₂₃H₂₇O₅ (M+H⁺) : 383.1858, Found: 383.1853.

### Example 30: Preparation of 2-(3,4-Dimethoxyphenyl)-1-(5-methoxy-2,2-dimethyl-2H-chromen-6-yl)-2-methylpropan-1-one (57)

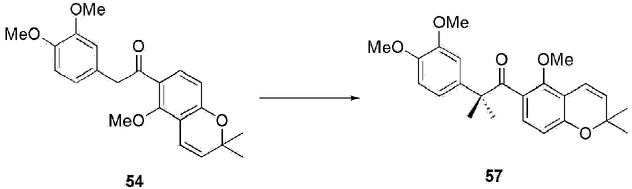

Anhydrous THF solution (1.0 mL) containing the compound 54 (15 mg, 0.041 mmol) obtained in Example 28 was stirred, to which sodium hydride (5.0 mg, 0.12 mmol, 60% in mineral oil) and iodomethane (0.02 mL, 0.16 mmol) were added at room temperature under argon atmosphere. Upon completion of the reaction, the mixture was extracted with EtOAc (2.0 mL×3). The organic layer of the extract was washed with brine, dried over MgSO₄, and concentrated under reduced pressure. Then, the obtained residue was purified by flash column chromatography (EtOAc:n-hexane=1:4) to give the compound 57 of Example 30 as a light-yellow solid (yield: 88%, 14 mg).
¹H NMR (CDCl₃, 400 MHz) δ 6.87 (m, 3H), 6.52 (d, 1H, *J* = 9.9 Hz), 6.21 (q, 2H, *J* = 5.4 Hz), 5.61 (d, 1H, *J* = 9.9 Hz), 3.88 (s, 3H), 3.83 (s, 3H), 3.70 (s, 3H), 1.50(s, 6H), 1.37(s, 6H);
¹³C NMR (CDCl₃, 100 MHz) δ 206.6, 155.1, 154.1, 148.9, 147.9, 136.2, 130.5, 127.8, 126.7, 118.2, 116.6, 115.0, 111.1, 111.0, 109.8, 77.2, 76.2, 63.6, 55.8, 55.8, 51.6, 27.9, 27.8, 26.3;
HRMS (FAB) Calcd for C₂₄H₂₉O₅ (M+H⁺): 397.2015, Found: 397.2015.

### Example 31: Preparation of 2-(3,4-Dimethoxyphenyl)-1-(5-methoxy-2,2-dimethyl-2H-chromen-6-yl)prop-2-en-1-one (58)

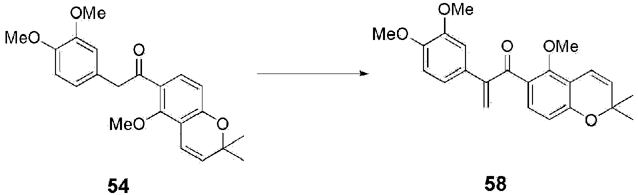

Potassium carbonate (15 mg, 0.11 mmol) was added to anhydrous DMF solution (1 mL) containing the compound 54 (20 mg, 0.054 mmol) prepared in Example 28 at room temperature under nitrogen atmosphere, followed by stirring at room temperature for 40 minutes. Then, paraformaldehyde (3 mg, 0.082 mmol) was added thereto. The mixture was stirred for 4 hours, followed by extraction with EtOAc (2.0 mL×2). The organic extract was washed with saturated NH₄Cl aqueous solution and brine, dried over MgSO₄, and concentrated under reduced pressure. The obtained residue was purified by flash column chromatography (EtOAc:n-hexane=1:4) to give the compound 58 of Example 31 as a colorless oil (yield: 87%, 18 mg).
¹H NMR (CDCl₃, 300 MHz) δ 7.30 (d, 1H, *J* = 8.6 Hz), 6.95 (m, 2H), 6.78 (d, 1H, *J* = 8.0 Hz), 6.51 (m, 2H), 5.90 (s, 1H), 5.60 (d, 1H, *J* = 10.0 Hz), 5.55 (s, 1H), 3.81 (s, 3H), 3.80 (s, 3H), 3.70 (s, 3H), 1.38 (s, 6H);
¹³C NMR (CDCl₃, 100 MHz) δ 196.5, 157.3, 156.2, 149.4, 149.1, 148.6, 131.7, 130.4, 129.7, 125.0, 121.7, 120.2, 116.5, 114.8, 111.8, 110.9, 110.7, 77.2, 76.8, 63.1, 55.8, 28.0, 27.9;
HRMS (FAB) Calcd for C₂₃H₂₅O₅ (M+H⁺) : 381.1702, Found: 381.1709.

### Example 32: Preparation of 1-(3,4-Dimethoxyphenyl)cyclopropyl)(5-methoxy-2,2-dimethyl-2H-chromen-6-yl)methanone (59)

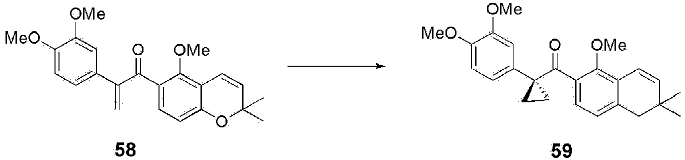

Sodium hydride (1.2 mg, 0.029 mmol, 60% in mineral oil) and trimethylsulfanium iodide (6.5 mg, 0.029 mmol) were added to anhydrous DMSO (0.5 mL) at room temperature under nitrogen atmosphere. The mixture was stirred for 40 minutes, to which anhydrous DMSO (0.5 mL) containing the compound 58 (10 mg, 0.026 mmol) prepared in Example 31 was added. The reaction mixture was stirred for 1 hour, cooled down with saturated NH₄Cl aqueous solution, and extracted with EtOAc (×3). The organic layer was washed with saturated NH₄Cl aqueous solution and brine, dried over MgSO₄, and concentrated under reduced pressure. The obtained residue was purified by flash column chromatography (EtOAc:n-hexane=1:4) to give the compound 59 of Example 32 as a light-yellow solid (yield: 91%, 9 mg).
¹H NMR (CDCl₃, 500 MHz) δ 6.98 (d, 1H, *J* = 8.4 Hz), 6.78 (m, 2H), 6.67 (d, 1H, *J* = 8.4 Hz), 6.48 (d, 1H, *J* = 10.0 Hz), 6.35 (d, 1H, *J* = 8.4 Hz), 5.58 (d, 1H, *J* = 10.0 Hz), 3.78 (s, 3H), 3.75(s, 3H), 3.73(s, 3H), 1.67 (q, 2H, *J* = 4.0 Hz), 1.35 (s, 6H), 1.28 (q, 2H, *J =* 4.0 Hz);
¹³C NMR (CDCl₃, 125 MHz) δ 203.6, 155.4, 153.9, 148.4, 147.8, 133.0, 130.4, 128.7, 126.3, 121.4, 116.5, 114.7, 113.6, 111.4, 110.6, 76.2, 63.1, 55.7, 55.7, 37.1, 27.8, 27.8, 17.7, 17.7;
HRMS (FAB) Calcd for C₂₄H₂₆O₅ (M⁺): 394.1780, Found: 394.1774.

### Example 33: Preparation of (S)-2-(3,4-Dimethoxyphenyl)-1-(5-methoxy-2,2-dimethyl-2H-chromen-6-yl)propan-1-one (69)

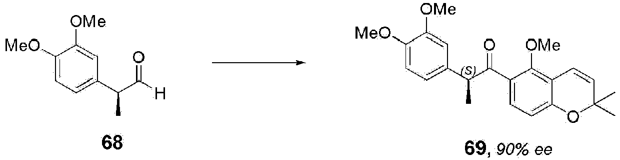

CeCl₃ · 7H₂O (189 mg, 0.52 mmol) was loaded in 1-neck flask, which was heated slowly until the temperature reached 135 ∼ 140 °C under reduced pressure (0.1 Torr) with stirring for 2 hours. When the flask was hot, argon gas was injected. Then, the flask was cooled down in an ice water bath. The reaction mixture was stirred vigorously, during which THF (1.0 mL) was added at once. The ice water bath was removed and the suspension was stirred overnight at room temperature under argon atmosphere. The flask was cooled down to -78 °C. n-BuLi (0.13 mL, 0.13 mmol) was added to anhydrous THF solution (1.0 mL) containing the compound 51 (61 mg, 0.23 mmol) prepared in Manufacturing Example 27 at -78 °C, followed by stirring for 20 minutes. The generated aryl anions were added to the reaction mixture. After stirring the mixture at -78 °C for 1.5 hours, the compound 68 (20 mg, 0.10 mmol) prepared in Manufacturing Example 19 was added to the mixture, followed by stirring for 30 minutes. The reaction mixture was treated with saturated NH₄Cl aqueous solution. The generated product was extracted with EtOAc, washed with brine, NaHCO₃, and brine stepwise, and dried over anhydrous MgSO₄. The solvent was evaporated and the residue was purified by flash column chromatography (EtOAc:n-hexane=1:3) to give the secondary alcohol as a diastereomer mixture (yield: 80%, 32 mg).
isomer A: ¹H-NMR (Acetone-d₆, 300 MHz) δ 7.22 (d, 1H, *J* = 8.4 Hz), 6.73 (m, 3H), 6.50 (m, 2H), 5.69 (d, 1H, *J* = 9.9 Hz), 4.95 (m, 1H), 3.70 (s, 6H), 3.61 (s, 3H), 3.07 (quin, 1H, *J*=6.8 Hz), 1,35 (s, 6H), 1.28 (d, 3H, *J* = 6.9 Hz);
isomer B: ¹H-NMR (Acetone-d₆, 500 MHz) δ 7.02 (d, 1H, *J* = 8.4 Hz), 6.75 (m, 3H), 6.55 (d, 1H, *J* = 10.0 Hz), 6.43 (d, 1H, *J* = 8.5 Hz), 5.70 (d, 1H, *J* = 9.9 Hz), 4.93 (dd, 1H, *J* = 7.5, 4.0 Hz), 3.72 (s, 3H), 3.72 (s, 3H), 3.71 (s, 3H), 2.95 (quin, 1H, *J* = 7.2 Hz), 1,34 (d, 6H, *J* = 11.8 Hz), 1.06 (d, 3H, *J* = 7.2 Hz).

The secondary alcohol (32 mg, 0.083 mmol) obtained above was dissolved in CH₂Cl₂ (2.0 mL), to which molecular sieve (41 mg) and N-methylmorpholine-N-oxide (NMO, 15 mg, 0.13 mmol) were added. The mixture was stirred for 10 minutes, to which tetrapropylammonium perruthenate (TPAP, 3 mg, 0.0083 mmol) was added. Upon completion of the reaction, the mixture was diluted with CH₂Cl₂ and washed with 10% sodium sulfite aqueous solution, brine, and lastly saturated copper(II) sulfate solution. The organic layer was dried over MgSO₄, filtered, and concentrated under reduced pressure. The obtained residue was purified by flash column chromatography (EtOAc:n-hexane=1:4) to give the compound 69 of Example 33 (yield: 84%, 27 mg).
¹H NMR (CDCl₃, 500 MHz) δ 7.27 (d, 1H, *J* = 8.5 Hz), 6.76 (m, 3H), 6.55 (d, 1H, *J* = 10.0 Hz), 6.48 (d, 1H, *J* = 8.5 Hz), 5.62 (d, 1H, *J* = 10.0 Hz), 4.60 (q, 1H, *J* = 6.9 Hz), 3.81 (s, 3H), 3.80 (s, 3H), 3.65 (s, 3H), 1.48 (d, 3H, *J* = 6.9 Hz), 1.39 (s, 6H);
¹³C NMR (CDCl₃, 75 MHz) δ 202.5, 157.1, 155.7, 149.0, 147.9, 133.8, 130.7, 130.5, 125.5, 120.2, 116.5, 112.3, 111.2, 77.2, 76.7, 63.3, 55.8, 55.7, 50.0, 43.1, 28.0, 28.0, 19.0;
HRMS (FAB) Calcd for C₂₃H₂₇O₅ (M+H⁺): 383.1858, Found: 383.1853.

### Example 34: Preparation of (R)-2-(3,4-Dimethoxyphenyl)-1-(5-methoxy-2,2-dimethyl-2H-chromen-6-yl)propan-1-one (72)

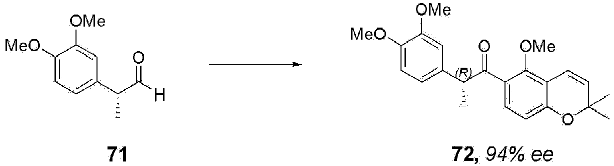

CeCl₃·7H₂O (125 mg, 0.34 mmol) was loaded in 1-neck flask, which was heated slowly until the temperature reached 135 ∼140 °C under reduced pressure (0.1 Torr) with stirring for 2 hours. When the flask was hot, argon gas was injected. Then, the flask was cooled down in an ice water bath. The reaction mixture was stirred vigorously, during which THF (1.0 mL) was added at once. The ice water bath was removed and the suspension was stirred overnight at room temperature under argon atmosphere. The flask was cooled down to -78 °C. n-BuLi (0.084 mL, 0.13 mmol) was added to anhydrous THF solution (1.0 mL) containing the compound 51 (36 mg, 0.14 mmol) prepared in Manufacturing Example 27 at -78 °C, followed by stirring for 20 minutes. The generated aryl anions were added to the reaction mixture. After stirring the mixture at -78 °C for 1.5 hours, the compound 71 (13 mg, 0.067 mmol) prepared in Manufacturing Example 21 was added to the mixture, followed by stirring for 30 minutes. The reaction mixture was treated with saturated NH₄Cl aqueous solution. The generated product was extracted with EtOAc, washed with brine, NaHCO₃, and brine stepwise, and dried over anhydrous MgSO₄. The solvent was evaporated and the residue was purified by flash column chromatography (EtOAc:n-hexane=1:3) to give the secondary alcohol as a diastereomer mixture (yield: 48%, 12 mg).
isomer A: ¹H-NMR (Acetone-d₆, 300 MHz) δ 7.22 (d, 1H, *J* = 8.4 Hz), 6.73 (m, 3H), 6.50 (m, 2H), 5.69 (d, 1H, *J* = 9.9 Hz), 4.95 (m, 1H), 3.70 (s, 6H), 3.61 (s, 3H), 3.07 (quin, 1H, J=6.8 Hz), 1,35 (s, 6H), 1.28 (d, 3H, *J* = 6.9 Hz);
isomer B: ¹H-NMR (Acetone-d₆, 500 MHz) δ 7.02 (d, 1H, *J* = 8.4 Hz), 6.75 (m, 3H), 6.55 (d, 1H, *J* = 10.0 Hz), 6.43 (d, 1H, *J* = 8.5 Hz), 5.70 (d, 1H, *J* = 9.9 Hz), 4.93 (dd, 1H, *J* = 7.5, 4.0 Hz), 3.72 (s, 3H), 3.72 (s, 3H), 3.71 (s, 3H), 2.95 (quin, 1H, *J* = 7.2 Hz), 1,34 (d, 6H, *J* = 11.8 Hz), 1.06 (d, 3H, *J* = 7.2 Hz).

The secondary alcohol (14 mg, 0.036 6 mmol) obtained above was dissolved in CH₂Cl₂ (1.0 mL), to which molecular sieve (18 mg) and *N*-methylmorpholine-*N*-oxide (NMO, 7.0 mg, 0.055 mmol) were added. The mixture was stirred for 10 minutes, to which tetrapropylammonium perruthenate (TPAP, 1.3 mg, 0.0036 6 mmol) was added. Upon completion of the reaction, the mixture was diluted with CH₂Cl₂ and washed with 10% sodium sulfite aqueous solution, brine, and lastly saturated copper(II) sulfate solution. The organic layer was dried over MgSO₄, filtered, and concentrated under reduced pressure. The obtained residue was purified by flash column chromatography (EtOAc:n-hexane=1:4) to give the compound 72 of Example 34 (yield: 71%, 10 mg).
¹H NMR (CDCl₃, 500 MHz) δ 7.27 (d, 1H, *J* = 8.5 Hz), 6.76 (m, 3H), 6.55 (d, 1H, *J* = 10.0 Hz), 6.48 (d, 1H, *J* = 8.5 Hz), 5.62 (d, 1H, *J* = 10.0 Hz), 4.60 (q, 1H, *J* = 6.9 Hz), 3.81 (s, 3H), 3.80 (s, 3H), 3.65 (s, 3H), 1.48 (d, 3H, *J* = 6.9 Hz), 1.39 (s, 6H);
¹³C NMR (CDCl₃, 75 MHz) δ 202.5, 157.1, 155.7, 149.0, 147.9, 133.8, 130.7, 130.5, 125.5, 120.2, 116.5, 112.3, 111.2, 77.2, 76.7, 63.3, 55.8, 55.7, 50.0, 43.1, 28.0, 28.0, 19.0;
HRMS (FAB) Calcd for C₂₃H₂₇O₅ (M+H⁺): 383.1858, Found: 383.1849.

### Example 35: Preparation of 3-(3,4-Dimethoxyphenyl)-8,8-dimethyl-4H,8H-pyrano[2,3-f]chromen-4-one (80)

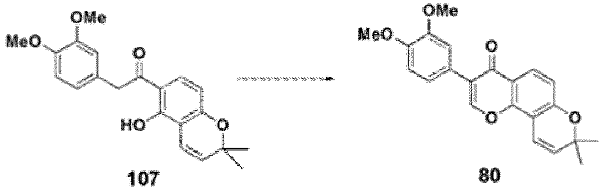

Dimethylaminodimethoxymethane (6.7 *µ*ℓ, 0.051 mmol) was added to anhydrous toluene solution (1.0 mL) containing the compound 107 (15 mg, 0.042 mmol) prepared in Manufacturing Example 32 at room temperature, followed by reflux for 2 hours. The solvent was eliminated under reduced pressure and the obtained residue was purified by flash column chromatography (Et OAc:n-hexane=1:3) to give the compound 80 of Example 35 (yield: 100%, 20 mg).
¹H-NMR (CDCl₃, 300 MHz) δ 8.00(d, 1H, *J* = 8.8 Hz), 7.90 (s, 1H), 7.14 (d, 1H, *J* = 2.0 Hz), 6.98 (dd, 1H, *J* = 8.3, 2.0 Hz), 6.86 (d, 1H, *J* = 8.3 Hz), 6.80 (d, 1H, *J* = 9.0 Hz), 6.75 (d, 1H, *J* = 10.0 Hz), 5.66 (d, 1H, *J* = 10.0 Hz), 3.86 (s, 3H), 3.84 (s, 3H), 1.43 (s, 6H);
¹³C-NMR (CDCl₃, 100 MHz) δ175.8, 157.2, 152.2, 151.8, 149.0, 148.7, 130.8, 126.6, 124.6, 124.6, 120.9, 118.2, 115.2, 114.8, 112.5, 111.1, 109.1, 77.6, 55.9, 55.8, 28.0, 28.0;
HRMS (FAB) Calcd for C₂₂H₂₁O₅ (M+H⁺): 365.1389, Found: 365.1394.

### Example 36: Preparation of 6,7-Dimethoxy-2H-chromen-4-yl(2,2-dimethyl-2H-chromen-6-yl)methanone (81)

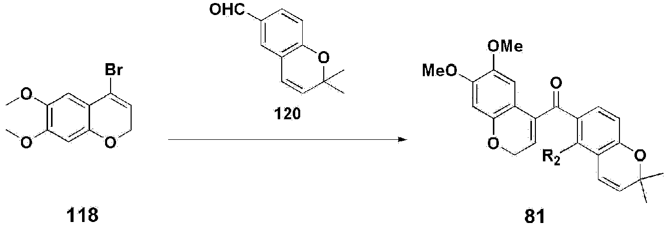

The compound 118 (49 mg, 0.179 mmol) prepared in Manufacturing Example 38 was added to anhydrous THF solution (2.0 mL) under nitrogen atmosphere, to which n-BuLi (0.12 mL, 0.20 mmol, 1.6 M in hexane) was added drop by drop at -78°C. The reaction mixture was stirred at -78°C for 20 minutes, to which anhydrous THF solution (1.0 mL) containing the compound 120 (78 mg, 0.357 mmol) prepared in Manufacturing Example 37 was added drop by drop by using a cannula. The prepared light-yellow solution was stirred at -78°C for 20 minutes, followed by extraction with EtOAc (×2). The organic layer was washed with brine, dried over anhydrous sodium sulfate, and concentrated under reduced pressure using a rotary evaporator. The obtained non-purified residue was purified by flash column chromatography (Et OAc:n-hexane=1:10) to give the intermediate secondary alcohol as a light-yellow solid (yield: 61%, 45 mg).
¹H NMR (CDCl₃, 300 MHz) δ7.15 (dd, 1H, *J* = 8.3, 2.2 Hz), 7.03 (d, 1H, *J* = 2.2Hz), 6.75 (d, 1H, *J* = 8.3 Hz), 6.62 (s, 1H), 6.41 (s, 1H), 6.26 (d, 1H, *J* = 9.9 Hz), 5.83 (t, 1H, *J* = 4.1 Hz), 5.59 (d, 1H, *J* = 9.9 Hz), 5.52 (br, 1H), 4.77 (m, 2H), 3.79 (s, 3H), 3.62 (s, 3H), 1.39(s, 6H).

Dess-Martin periodinane (3.0 equivalent) was added to CH₂Cl₂ (0.03 M) solution containing the secondary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (Et OAc:n-hexane=1:5) to give the compound 81 (yield: 87%, 39 mg).
¹H NMR (CDCl₃, 300 MHz) δ 7.70 (dd, 1H, *J* = 8.4, 2.2 Hz), 7.59 (d, 1H, *J* = 2.2 Hz), 6.91 (s, 1H), 6.77 (d, 1H, *J* = 8.4 Hz), 6.48 (s, 1H), 6.32 (d, 1H, *J* = 9.9 Hz), 5.96 (t, 1H, *J* = 4.0 Hz), 5.65 (d, 1H, *J* = 9.9 Hz), 4.77 (d, 2H, *J* = 4.0 Hz), 3.84 (s, 3H), 3.72 (s, 3H), 1.44 (s, 6H);
¹³C NMR (CDCl₃, 100 MHz) δ 193.7, 157.8, 160.0, 148.4, 143.4, 134.7, 132.1, 131.2, 129.7, 128.5, 124.1, 121.4, 120,6, 116.1, 112.0, 108.3, 100.4, 77.7, 64.6, 56.1, 55.9, 28.3, 28.3;
HRMS (FAB) Calcd for C₂₃H₂₂O₅ (M+H⁺) : 378.1467, Found: 378.1475.

### Example 37: Preparation of 6,7-Dimethoxy-2,2-dimethyl-2H-chromen-4-yl)(4-methoxy-2,2-dimethyl-2H-chromen-6-yl)methanone (82)

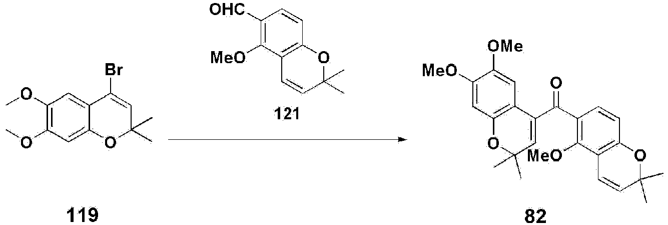

The compound 82 was prepared as a colorless solid by the same manner as described in Example 36 except that the compound 119 was used instead of the compound 118, the compound 121 was used instead of the compound 120, and flash column chromatography (EtOAc:n-hexane=1:4) was used for the purification (yield: 44%, 29 mg) .
Secondary alcohol: ¹H NMR (CDCl₃, 300 MHz) δ 7.01 (d, 1H, *J* = 8.2 Hz), 6.59 9 (s, 1H), 6.56 6 (d, 1H, *J* = 10.0 Hz), 6.50 (d, 1H, *J* = 8.2 Hz), 6.38 (s, 1H), 5.91 (br, 1H), 5.74 (s, 1H), 5.63 (d, 1H, *J* = 10.0 Hz), 3.88 (s, 3H), 3.77 (s, 3H), 3.62 (s, 3H), 1.45 (s, 3H), 1.43 (s, 3H), 1.39 (s, 6H);
Compound 82: ¹H NMR (CDCl₃, 300 MHz) δ 7.31 (d, *J* = 8.4 Hz, 1H), 7.21 (s, 1H), 6.60 (d, *J* = 9.5 Hz, 1H), 6.57 (d, *J* = 7.9 Hz, 1H), 6.45 (s, 1H), 5.88 (s, 1H), 5.65 (d, *J =* 10 Hz, 1H), 3.83 (s, 3H), 3.75 (s, 3H), 3.72 (s, 3H), 1.43 (s, 6H), 1.42 (s, 6H); ¹³C NMR (CDCl₃, 75 MHz) δ 194.3, 157.4, 156.3, 150.1, 147.3, 143.1, 136.1, 133.2, 131.8, 130.4, 124.7, 116.5, 114.8, 111.8, 110.7, 108.8, 108.7, 101.1, 77.4, 76.8, 75.4, 63.1, 56.3, 56.8, 28.0, 26.6; HRMS (FAB) Calcd for C₂₆H₂₉O₆ (M+H⁺): 437.1964, Found: 437.1973.

### Example 38: Preparation of 6,7-Dimethoxy-2,2-dimethyl-2H-chromen-4-yl)(2,2-dimethyl-2H-chromen-6-yl)methanone (83)

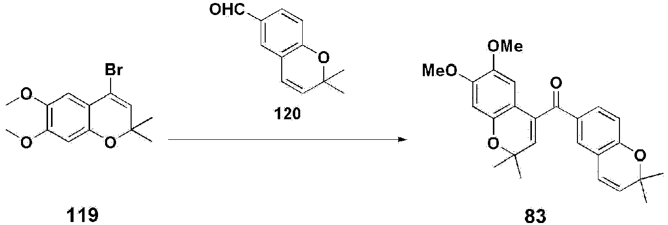

The compound 83 was prepared as a colorless solid by the same manner as described in Example 36 except that the compound 119 was used instead of the compound 118, and flash column chromatography (EtOAc:n-hexane=1:4) was used for the purification (yield: 51%, 35 mg).
Secondary alcohol: ¹H NMR (CDCl₃, 300 MHz) δ 7.09 (dd, 1H, *J* = 8.3 Hz), 6.98 (d, 1H, *J* = 2.0 Hz), 6.50 (s, 1H), 6.35 (s, 1H), 6.21 (d, 1H, 0 *J* = 9.9 Hz), 5.65 (s, 1H), 5.54 (d, 1H, *J* = 9.9 Hz), 5.46 (br, 1H), 3.73 (s, 3H), 3.54 (s, 3H), 1.39 (s, 6H), 1.34 (s, 6H);
Compound 83: ¹H NMR (CDCl₃, 300 MHz) δ 7.68 (dd, 1H, *J* = 8.4, 2.2 Hz), 7.57 (d, 1H, *J* = 2.2 Hz), 6.86 (s, 1H), 6.76 (d, 1H, *J* = 8.4 Hz), 6.46 (s, 1H), 6.32 (d, 1H, *J* = 9.9 Hz), 5.74 (s, 1H), 5.65 (d, 1H, *J* = 9.9 Hz), 3.84 (s, 3H), 3.71 (s, 3H), 1.47 (s, 6H), 1.44 (s, 6H) ; ¹³C NMR (CDCl₃, 75 MHz) δ 194. 0, 157. 8, 150.4, 147.3, 143.2, 132.5, 132.3, 132.1, 131.2, 130.1, 128.5, 121.6, 120.7, 116.1, 108.7, 101.2, 77.7, 77.2, 75.5, 56.4, 55.9, 50.3, 50.3, 28.4, 27.0; HRMS (FAB) Calcd for C₂₅H₂₆O₅ (M+H⁺) : 406.1780, Found: 406.1775.

### Example 39: Preparation of 6,7-Dimethoxy-2H-chromen-4-yl)(4-methoxy-2,2-dimethyl-2H-chromen-6-yl)methanone (84)

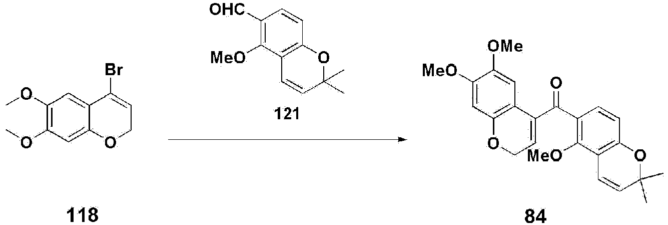

The compound 84 was prepared as a colorless solid by the same manner as described in Example 36 except that the compound 121 was used instead of the compound 120, and flash column chromatography (EtOAc:n-hexane=1:5) was used for the purification (yield: 41%, 22 mg).
Secondary alcohol: ¹H NMR (CDCl₃, 300 MHz) δ 7.01 (d, 1H, *J* = 8.3 Hz), 6.60 (s, 1H), 6.48 (s, 1H), 6.34 (s, 1H), 5.83 (m, 2H), 5.59 (d, 1H, *J* = 9.9 Hz), 4.73 (d, 1H, *J* = 3.8 Hz), 3.81 (s, 3H), 3.73 (s, 3H), 3.60 (s, 3H), 1.34 (s, 6H);
Compound 84: ¹H NMR (CDCl₃, 300 MHz) δ 7.31(d, *J* = 8.6 Hz, 1H), 7.27(s, 1H), 6.59(d, *J* = 10.1 Hz, 1H), 6.56 (d, *J* = 8.0 Hz, 1H), 6.47(s, 1H), 6.11 (t, *J* = 4.0 Hz, 1H), 5.65(d, *J* = 10 Hz, 1H), 4.78(d, *J* = 4.2 Hz, 2H), 3.84(s, 3H), 3.77(s, 3H), 3.72(s, 3H), 1.43(s, 6H): ¹³C NMR (CDCl₃, 100 MHz) δ 193.3, 157.3, 156.1, 149.8, 148.6, 143.3, 135.4, 131.6, 130.5, 128.2, 124.3, 116.4, 114.8, 111.8, 111.7, 108.5, 100.3, 77.2, 64.7, 63.1, 56.1, 55.8, 27.9, 27.9; LRMS (FAB) *m*/*z* 409 (M+H⁺) .

### Example 40: Preparation of 2-(3,4-Dimethoxyphenyl)-1-(5-hydroxy-2,2-dimethyl-2H-chromen-6-yl)-2-(phenylsulfonyl)ethanone (86)

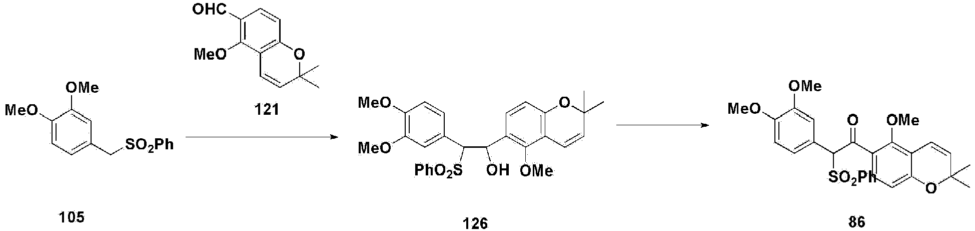

n-BuLi (0.26 mL, 0.444 mmol, 1.6 M in hexane) was added drop by drop to anhydrous THF solution (5.0 mL) containing the compound 105 (0.407 mmol) prepared in Manufacturing Example 28 at -78°C, which was stirred at the same temperature for 1 hour. Anhydrous THF (3.0 mL) containing the compound 121 (0.448 mmol) obtained in Manufacturing Example 38 was added slowly to the above reaction mixture at -78°C. One hour later, water was added to the reaction mixture to terminate the reaction, followed by extraction with EtOAc (×3). The extract was washed with brine, dried over anhydrous sodium sulfate, and concentrated under reduced pressure. The obtained non-purified residue (compound 126) was used for the next step without being through purification process.

Dess-Martin periodinane (286 mg, 0.660 mmol) was added to anhydrous CH₂Cl₂ (5.0 mL) solution containing the compound 126 (183 mg, 0.339 mmol) obtained above, followed by stirring for 1 hour. The reaction mixture was treated with saturated sodium carbonate solution:saturated sodium thiosulfate solution (1:1, 4 mL), followed by stirring for 30 minutes. The reaction mixture was poured in water, followed by extraction with CH₂Cl₂ (×3). The organic layer was dried over MgSO₄, filtered and concentrated under reduced pressure. Then, the obtained residue was purified by flash column chromatography (EtOAc:n-hexane=1:2) to give the compound 86 (yield: 63%, 137 mg).
¹H NMR (CDCl₃, 300 MHz) δ 7.53 (m, 3H), 7.35 (m, 3H), 6.80 (m, 2H), 6.68 (d, *J* = 8.2 Hz, 1H), 6.50 (d, *J* = 8.6 Hz, 1H), 6.45 (d, *J* = 10.1 Hz, 1H), 6.35 (s, 1H), 5.61 (d, *J* = 10.1 Hz, 1H), 3.79 (s, 3H), 3.61 (s, 3H), 3.53 (s, 3H), 1.38(s, 3H), 1.35(s, 3H);
¹³C NMR (CDCl₃, 100 MHz) δ 206.6, 155.1, 154.1, 148.9, 147.9, 136.2, 130.5, 127.8, 126.7, 118.2, 116.6, 115.0, 111.1, 111.0, 109.8, 77.2, 76.2, 63.6, 55.8, 55.8, 51.6, 27.9, 27.8, 26.3;
HRMS (FAB) Calcd for C₂₄H₂₉O₅ (M+H⁺) : 397.2015, Found: 397.2015.

### Example 41: Preparation of (3,4-Dimethoxyphenyl)(2,2-dimethyl-2H-chromen-6-yl)methanone (87)

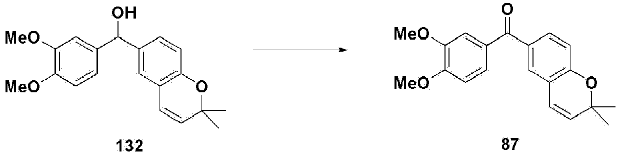

Dess-Martin periodinane (3.0 equivalent) was added to CH₂Cl₂ (0.03 M) solution containing the compound 132 (1.0 equivalent) obtained in Manufacturing Example 40, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (Et OAc:n-hexane=1:8) to give the compound 87 as a colorless solid (yield: 81%, 86 mg).
¹H NMR (CDCl₃, 300 MHz) δ 7.56 (d, 1H, *J* = 8.4, 2.2 Hz), 7.47 (d, 1H, *J* = 2.2 Hz), 7.41 (d, 1H, *J* = 1.8 Hz), 7.34 (d, 1H, *J* = 8.2, 2.0 Hz), 6.88 (d, 1H, *J* = 8.4 Hz), 6.79 (d, 1H, *J* = 8.4 Hz), 6.34 (d, 1H, *J* = 9.9 Hz), 5.65 (d, 1H, *J* = 9.9 Hz),3.94 (s, 3H), 3.92 (s, 3H), 1.45 (s, 6H) ;
¹³C NMR (CDCl₃, 100 MHz) δ 194.3, 156.7, 152.5, 148.8, 131.8, 131.1, 130.7, 130.7, 128.5, 124.7, 121.6, 120.5, 115.7, 112.1, 109.7, 77.4, 56.0, 28.3, 28.2.;
LRMS (FAB) *m*/*z* 325 (M+H⁺).

### Example 42: Preparation of (E)-1-(3,4-Dimethoxyphenyl)-3-(2,2-dimethyl-2H-chromen-6-yl)prop-2-en-1-one (88)

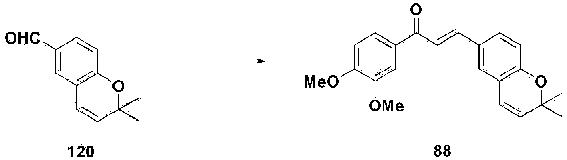

Potassium hydroxide aqueous solution (1.5 equivalent) was added to ethyl alcohol (0.1 M) containing the compound 120 (2.0 equivalent) obtained in Manufacturing Example 37 and 3',4'-dimethoxyphenylacetophenone (1.0 equivalent) at room temperature, followed by reflux for 5 hours. The mixture was cooled down to room temperature. The solvent was eliminated under reduced pressure using a rotary evaporator and the obtained non-purified residue was extracted with EtOAc (×2). The organic layer was washed with brine, dried over anhydrous MgSO₄, and concentrated under reduced pressure using a rotary evaporator. The obtained residue was purified by flash column chromatography (EtOAc:n-hexane=1:5) to give the compound 88 as a light-yellow solid (yield: 59%, 29 mg).
¹H NMR (CDCl₃, 300 MHz) δ 7.62 (m, 3H), 7.35 (m, 2H), 7.21 (d, 1H, *J* = 2.0 Hz), 6.86 (d, 1H, *J* = 8.4 Hz), 6.74 (d, 1H, *J* = 8.4 Hz), 6.29 (d, 1H, *J* = 9.9 Hz), 5.61 (d, 1H, *J* = 9.7 Hz), 3.90 (s, 3H), 3.90 (s, 3H), 1.39 (s, 6H);
¹³C NMR (CDCl₃, 100 MHz) δ 188.6, 155.3, 153.0, 149.1, 143.9, 131.6, 131.3, 129.7, 127.7, 123.2, 121.6, 121.3, 119.1, 116.8, 116.0, 110.7, 109.9, 77.1, 56.1, 56.0, 28.2, 28.1.

### Example 43: Preparation of (E)-3-(3,4-Dimethoxyphenyl)-1-(5-hydroxy-2,2-dimethyl-2H-chromen-6-yl)prop-2-en-1-one (89)

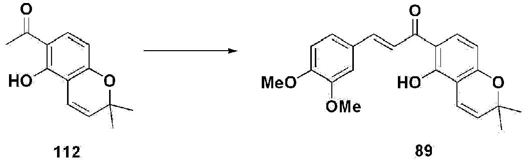

Potassium hydroxide aqueous solution (1.0 mL, 0.5 mM) was added to ethyl alcohol (4.0 mL) containing the compound 112 (100 mg, 0.46 mmol) obtained in Manufacturing Example 41 and 3,4-dimethoxybenzaldehyde (154 4 mg, 0.92 mmol) at room temperature, followed by reflux for 5 hours. The mixture was cooled down to room temperature and concentrated under reduced pressure. The non-purified residue was extracted with EtOAc (10 mL) and water (5.0 mL). The organic layer was washed with brine, dried over anhydrous MgSO₄, and concentrated under reduced pressure. The obtained residue was purified by flash column chromatography (EtOAc:n-hexane=1:4) to give the compound 89 as a white solid (yield: 57%, 95 mg).
¹H-NMR (CDCl₃, 400 MHz) 13.77 (s, 1H), 7.78 (d, 1H, *J* = 15.3 Hz), 7.68 (d, 1H, *J* = 8.8 Hz), 7.37 (d, 1H, *J* = 15.3 Hz), 7.18 (d, 1H, *J* = 8.1 Hz), 7.10 (s, 1H), 6.84 (d, 1H, *J* = 8.3 Hz), 6.71 (d, 1H, *J* = 10.0 Hz), 6.33 (d, 1H, *J* = 8.8 Hz), 5.54 (d, 1H, *J* = 10.0 Hz), 3.90 (s, 3H), 3.88 (s, 3H), 1.42 (s, 6H);
¹³C-NMR (CDCl₃, 100 MHz) δ 191.7, 160.8, 159.5, 151.4, 149.1, 144.3, 130.4, 127.9, 127.6, 123.2, 117.8, 115.8, 113.9, 111.0, 110.2, 110.0, 109.3, 108.8, 108.0, 77.6, 55.8, 28.2.

### Example 44: Preparation of (E)-3-(3,4-Dimethoxyphenyl)-1-(2,2-dimethyl-2H-chromen-6-yl)prop-2-en-1-one (90)

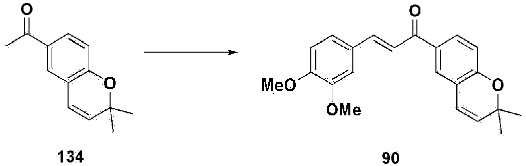

Potassium hydroxide aqueous solution (1.5 equivalent) was added to ethyl alcohol (0.1 M) containing the compound 134 (2.0 equivalent) obtained in Manufacturing Example 42 and 3,4-dimethoxybenzaldehyde (1.0 equivalent) at room temperature, followed by reflux for 5 hours. The mixture was cooled down to room temperature. The solvent was eliminated under reduced pressure using a rotary evaporator and the obtained non-purified residue was extracted with EtOAc (×2). The organic layer was washed with brine, dried over anhydrous MgSO₄, and concentrated under reduced pressure using a rotary evaporator. The obtained residue was purified by flash column chromatography (EtOAc:n-hexane=1:4) to give the compound 90 as a light-yellow solid (yield: 57%, 23 mg).
¹H NMR (CDCl₃, 300 MHz) δ 7.87 (dd, 1H, *J* = 8.4, 2.0 Hz), 7.77 (m, 2H), 7.41 (d, 1H, *J* = 15.5 Hz), 7.24 (m, 2H), 6.92 (d, 1H, *J* = 8.4 Hz), 6.87 (d, 1H, *J* = 8.4 Hz), 6.43 (d, 1H, *J* = 9.9 Hz), 5.71 (d, 1H, *J* = 9.9 Hz), 3.98 (s, 3H), 3.95 (s, 3H), 1.49 (s, 6H);
¹³C NMR (CDCl₃, 100 MHz) δ 190.7, 188.5, 157.1, 151.1, 149.1, 143.9, 131.2, 131.0, 130.1, 127.9, 127.0, 122.8, 121.6, 120.7, 119.6, 116.0, 111.0, 110.0, 77.4, 55.8, 28.2, 28.1;
HRMS (FAB) Calcd for C₂₂H₂₃O₄ (M+H⁺) : 351.1596, Found: 351.1590.

### Example 45: Preparation of (E)-1-(5-hydroxy-2,2-dimethyl-2H-chromen-6-yl)-3-(2,4,5-trimethoxyphenyl)prop-2-en-1-one (91)

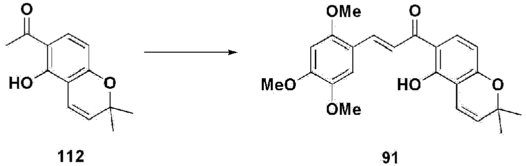

The compound 91 was prepared by the same manner as described in Example 43 except that 2,4,5-trimethoxybenzaldehyde was used instead of 3,4-dimethoxybenzaldehyde (yield: 61%, 29 mg).
¹HNMR (CDCl₃, 300MHz) δ 7.87 (dd, 1H, *J* = 8.4, 2.0 Hz), 7.77 (m, 2H), 7.41 (d, 1H, *J* = 15.5 Hz), 7.24 (m, 2H), 6.92 (d, 1H, *J* = 8.4 Hz), 6.87 (d, 1H, *J* = 8.4 Hz), 6.43 (d, 1H, *J* = 9.9 Hz), 5.71 (d, 1H, *J* = 9.9 Hz), 3.98 (s, 3H), 3.95 (s, 3H), 1.49 (s, 6H);
¹³C-NMR (CDCl₃, 100 MHz) δ 190.7, 188.5, 157.1, 151.1, 149.1, 143.9, 131.2, 131.0, 130.1, 127.9, 127.0, 122.8, 121.6, 120.7, 119.6, 116.0, 111.0, 110.0, 77.4, 55.8, 28.2, 28.1;
HRMS (FAB) Calcd for C₂₂H₂₃O₄ (M+H⁺) : 351.1596, Found: 351.1590.

### Example 46: Preparation of (E)-3-(3,4-Dimethoxyphenyl)-1-(5-methoxy-2,2-dimethyl-2H-chromen-6-yl)prop-2-en-1-one (92)

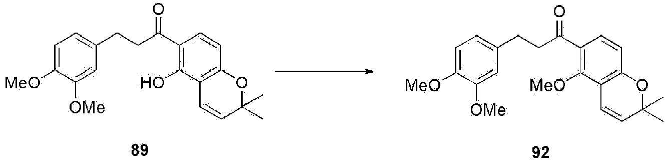

Acetone (0.25 M) mixture containing phenol (1 equivalent), potassium carbonate (3 equivalent) and iodomethane (1.5 equivalent) was refluxed for 4 hours, which was further stirred at room temperature overnight. The mixture was concentrated, treated with water, and extracted with CH₂Cl₂ (×3). The extract was washed with water and dried over anhydrous MgSO₄. The solvent was eliminated under reduced pressure and the obtained non-purified residue was purified by flash column chromatography (EtOAc:n-hexane=1:5) to give the compound 92 as a light-yellow solid (yield: 81%, 18 mg).
¹H NMR (CDCl₃, 300 MHz) δ 7.60 (d, 1H, *J* = 14.5 Hz), 7.46 (d, 1H, *J* = 8.4 Hz), 7.30 (d, 1H, *J* = 14.5 Hz), 7.12 (m, 2H), 6.82 (d, 1H, *J* = 8.4 Hz), 6.58 (m, 2H), 6.63 (d, 1H, *J* = 10.0 Hz), 3.86 (s, 3H), 3.85 (s, 3H), 1.39 (s, 6H);
HRMS (FAB) Calcd for C₂₃H₂₅O₅ (M+H⁺) : 381.1702, Found: 381.1698.

### Example 47: Preparation of (E)-1-(5-methoxy-2,2-dimethyl-2H-chromen-6-yl)-3-(2,4,5-trimethoxyphenyl)prop-2-en-1-one (93)

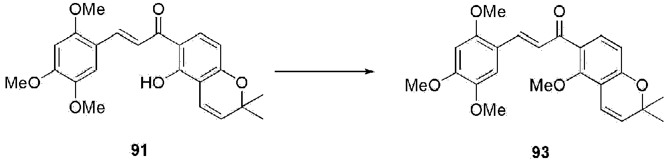

The compound 93 was prepared by the same manner as described in Example 46 except that the compound 91 was used instead of the compound 89 (yield: 75%, 20 mg).
¹H NMR (CDCl₃, 300 MHz) δ 7.96(d, 1H, *J* = 15.9 Hz), 7.44 (d, 1H, *J* = 8.4 Hz), 7.32 (d, 1H, *J* = 15.9 Hz), 7.05 (s, 1H), 6.57 (m, 1H), 6.44 (s, 1H), 5.62 (d, 1H, *J* = 9.8 Hz), 3.87 (s, 3H), 3.81 (s, 3H), 3.81 (s, 3H), 3.69 (s, 3H), 1.39 (s, 6H);
¹³C-NMR(CDCl₃, 100 MHz) δ 191.5, 156.9, 156.0, 154.4, 152.3, 143.2, 138.6, 131.1, 130.4, 126.0, 124.1, 116.6, 115.7, 114.7, 112.4, 110.9, 96.9, 76.7, 63.2, 56.5, 56.4, 56.3, 28.0, 28.0;
HRMS(FAB) Calcd for C₂₄H₂₇O₆ (M+H⁺): 411.1808, Found:411.1813.

### Example 48: Preparation of 2-(3,4-Dimethoxyphenyl)-8,8-dimethyl-4H,8H-pyrano[2,3-f]chromen-4-one (94)

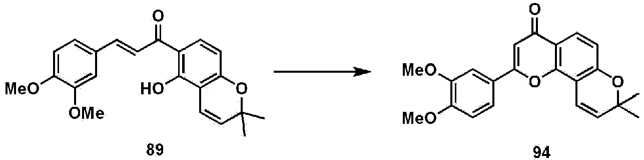

Iodide (catalytic amount) was added to anhydrous DMSO (5.0 mL) containing the compound 89 (50 mg, 0.14 mmol) prepared in Example 43 at room temperature. While monitoring with TLC, the reaction mixture was refluxed until the reaction was completed. The mixture was cooled down to room temperature. Then, the mixture was poured in 20% sodium thiosulfate aqueous solution (10 mL), followed by extraction with EtOAc (10 mL). The organic layer was washed with water and brine, dried over anhydrous sodium sulfate, and concentrated under reduced pressure using a rotary evaporator. Then, the obtained residue was purified by flash column chromatography (CH2Cl2:MeOH=1:10) to give the compound 94 as a white solid (yield: 28%, 14 mg).
¹H-NMR (CDCl₃, 300 MHz) δ 7.92 (d, 1H, *J* = 8.6 Hz), 7.48 (dd, 1H, *J* = 8.4, 2.0 Hz), 7.29 (d, 1H, *J* = 2.0 Hz), 6.93 (d, 1H, *J* = 8.4 Hz), 6.81 (m, 2H), 6.66 (s, 1H), 5.70 (d, 1H, *J* = 9.8 Hz), 3.91 (s, 3H), 3.90 (s, 3H), 1.45 (s, 6H);
HRMS (FAB) Calcd for C₂₂H₂₁O₅ (M+H⁺) : 365.1389, Found: 365.1387.

### Example 49: Preparation of 2-(3,4-Dimethoxyphenyl)-N-(5-methoxy-2,2-dimethyl-2H-chromen-6-yl)acet-amide (95)

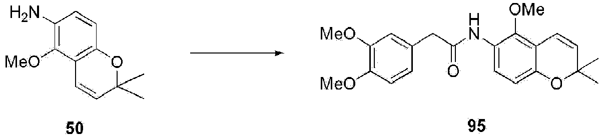

*N*,*N*-diisopropylethylamine (2.2 equivalent) was added to anhydrous dichloromethane (0.3 M) mixture containing 3',4'-dimethoxyphenylacetic acid (1.0 equivalent), PyBOP (1.0 equivalent) and the compound 50 (1.2 equivalent) obtained in Manufacturing Example 26. The mixture was stirred overnight under nitrogen atmosphere. The solvent was evaporated and the obtained residue was dissolved in EtOAc. The reaction mixture was extracted with 5% HCl aqueous solution, washed with brine, and extracted again with saturated sodium hydrogen carbonate aqueous solution, dried over anhydrous MgSO₄, and filtered. The solvent was evaporated and the obtained brown oil residue was purified by flash column chromatography (EtOAc:n-hexane=1:2) to give the compound 95 as a yellow solid (yield: 91%, 31 mg).
¹H NMR (CDCl₃, 400 MHz) δ 7.93 (d, 1H, *J* = 8.8 Hz), 7.52 (br, 1H), 6.82 (m, 3H), 6.49 (d, 1H, *J* = 8.8 Hz), 6.37 (d, 1H, *J* = 9.9 Hz), 5.57 (d, 1H, *J* = 9.9 Hz), 3.82 (s, 3H), 3.81 (s, 3H), 3.62 (s, 2H), 3.35 (s, 3H), 1.31 (s, 6H);
¹³C NMR (CDCl₃, 100 MHz) δ 168.8, 149.5, 149.3, 148.3, 144.8, 131.2, 127.0, 124.2, 121.5, 120.3, 116.2, 114.1, 112.3, 112.0, 111.5, 75.4, 61.4, 55.7, 55.7, 44.3, 27.3, 27.2;
HRMS (FAB) Calcd for C₂₂H₂₅NO₅ (M+H⁺): 384.1811, Found: 384.1806.

### Example 50: Preparation of N-(3,4-Dimethoxybenzyl)-2,2-dimethyl-2H-chromene-6-carboxamide (96)

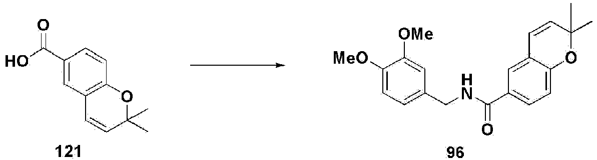

The compound 96 was prepared as a yellow solid by the same manner as described in Example 49 except that N,N-diisopropylethylamine (2.2 equivalent) was added to anhydrous dichloromethane (0.3 M) mixture containing 3,4-dimethoxybenzylamine (1.0 equivalent), PyBOP (1.0 equivalent) and the compound 121 (1.2 equivalent) prepared in Manufacturing Example 38 (yield: 79%, 41 mg).
¹H NMR (CDCl_{3,} 400 MHz) δ 7.51 (dd, 1H, *J* = 8.3, 2.0 Hz), 7.43 (d, 1H, *J* = 2.0 Hz), 6.80 (m, 3H), 6.71 (d, 1H, *J* = 8.3 Hz), 6.46 (m, 2H), 5.60 (d, 1H, *J* = 9.8 Hz), 4.49 (d, 1H, *J* = 5.5 Hz), 3.81 (s, 3H), 3.80 (s, 3H), 1.39 (s, 6H);
¹³C NMR (CDCl₃, 100 MHz) δ 166.7, 155.8, 149.0, 148.3, 131.2, 130.9, 127.8, 126.6, 125.5, 121.6, 120.8, 120.1, 116.0, 111.2, 111.1, 77.6, 55.8, 55.7, 43.8, 28.1, 28.1;
HRMS (FAB) Calcd for C₂₁H₂₃NO₄ (M+H⁺) : 354.1705, Found: 354.1703.

### Example 51: Preparation of N-(3,4-Dimethoxyphenyl)-2,2-dimethyl-2H-chromene-6-carboxamide (97)

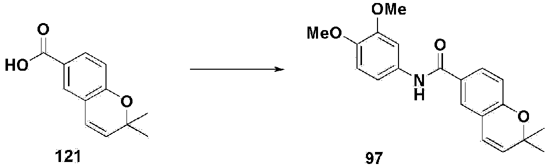

The compound 97 was prepared as a yellow solid by the same manner as described in Example 49 except that N,N-diisopropylethylamine (2.2 equivalent) was added to anhydrous dichloromethane (0.3 M) mixture containing 3,4-dimethoxyaniline (1.0 equivalent), PyBOP (1.0 equivalent) and the compound 121 (1.2 equivalent) prepared in Manufacturing Example 38, and flash column chromatography (EtOAc:n-hexane=1:3) was used for the purification (yield: 87%, 29 mg).
¹H NMR (CDCl₃, 400 MHz) δ 7.89 (s, 1H), 7.53 (dd, 1H, *J* = 8.8, 2.2 Hz), 7.50 (d, 1H, *J* = 2.3 Hz), 7.42 (d, 1H, *J* = 2.2 Hz), 6. 96 (dd, 1H, *J* = 8.6, 2.3 Hz), 6.76 (m, 2H), 6.28 (d, 1H, *J* = 9.8 Hz), 5.63 (d, 1H, *J* = 9.8 Hz), 3.82 (s, 6H), 1.42 (s, 6H);
¹³C NMR (CDCl₃, 100 MHz) δ 184.5, 165.2, 156.0, 148.9, 145.7, 131.7, 131.4, 127.9, 127.0, 125.6, 121.5, 121.0, 116.2, 112.1, 111.2, 105.9, 77.2, 56.0, 56.0, 55.7, 28.1, 28.1;
HRMS (FAB) Calcd for C₂₀H₂₁NO₄ (M+H⁺) : 340.1549, Found: 340.1542.

### Example 52: Preparation of N-(2,2-Dimethyl-2H-chromen-6-yl)-3,4-dimethoxybenzamide (98)

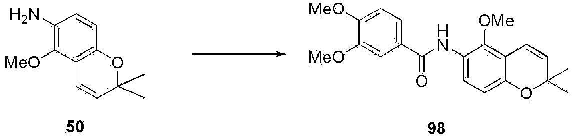

N,N-diisopropylethylamine (2.2 equivalent) was added to anhydrous dichloromethane (0.3 M) mixture containing 3',4'-dimethoxybenzoic acid (1.0 equivalent), PyBOP (1.0 equivalent) and the compound 50 (1.2 equivalent) obtained in Manufacturing Example 26. The mixture was stirred overnight under nitrogen atmosphere. The solvent was evaporated and the obtained residue was dissolved in EtOAc. The reaction mixture was extracted with 5% HCl aqueous solution, washed with brine, and extracted again with saturated sodium hydrogen carbonate aqueous solution, dried over anhydrous MgSO₄, and filtered. The solvent was evaporated and the obtained brown oil residue was purified by flash column chromatography (EtOAc:n-hexane=1:2) to give the compound 98 as a yellow solid (yield: 60%, 17 mg).
¹H NMR (CDCl₃, 500 MHz) δ 8.16 (s, 1H), 8.08 (d, 1H, *J* = 8.8 Hz), 7.49 (s, 1H), 7.36 (dd, 1H, *J* = 7.8. 1.8 Hz), 6.89 (d, 1H, *J* = 7.8 Hz), 6.61 (d, 1H, *J* = 8.8 Hz), 6.53 (d, 1H, *J* = 9.9 Hz), 6.57 (d, 1H, *J* = 9.9 Hz), 3.93 (s, 3H), 3.91 (s, 3H), 3.78 (s, 1H), 1.40 (s, 6H);
¹³C NMR (CDCl₃, 125 MHz) δ 164.7, 151.9, 149.8, 149.1, 145.4, 131.4, 127.7, 124.6, 121.2, 119.1, 116.5, 114.3, 112.4, 110.7, 110.3, 75.6, 62.0, 56.0, 27.5, 27.5;
LRMS (FAB) *m*/*z* 370 (M+H⁺).

### Example 53: Preparation of (R)-2-(3,4-Dimethoxyphenyl)-1-(2,2-dimethyl-2H-chromen-6-yl)propan-1-one (99)

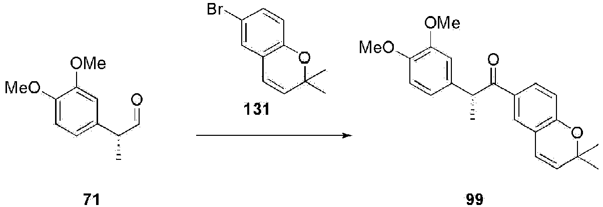

n-BuLi (1.4 equivalent) was added to anhydrous THF solution containing the compound 131 (aryl bromide, 1.5 equivalent) drop by drop at -78°C, which was stirred at -78°C to generate aryl anions. The mixture was stirred at -78°C for 20 minutes, to which the compound 71 (1.0 equivalent) prepared in Manufacturing Example 21 was added, followed by stirring for 30 minutes. The reaction mixture was treated with saturated NH₄Cl aqueous solution, followed by extraction with EtOAc. The extract was washed with brine and then dried over MgSO₄. The residue obtained after evaporating the solvent was purified by flash column chromatography (EtOAc:n-hexane=1:4) to give the secondary alcohol (yield: 48%, 19 mg).

Dess-Martin periodinane (3.0 equivalent) was added to CH₂Cl₂ (0.03 M) solution containing the secondary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:5) to give the compound 99 (yield: 85%, 16 mg).
¹H NMR (CDCl₃, 500 MHz) δ 7.73 (dd, 1H, *J* = 8.4, 1.7 Hz), 7.60 (d, 1H, *J* = 1.6 Hz), 6.78 (m, 3H), 6.68 (d, 1H, *J* = 8.5 Hz), 6.27 (d, 1H, *J* = 9.9 Hz), 5.59 (d, 1H, *J* = 9.9 Hz), 4.54 (q, 1H, *J* = 6.8 Hz), 3.82 (s, 3H), 3.80 (s, 3H), 1.46 (d, 3H, *J* = 6.8 Hz), 1.39 (s, 6H);
¹³C NMR (CDCl₃, 125 MHz) δ 198.9, 157.1, 149.2, 147.8, 134.3, 130.9, 130.5, 129.4, 127.3, 121.6, 120.5, 119.9, 115.9, 111.4, 110.5, 77.4, 55.8, 55.7, 46.8, 28.3, 28.3, 19.5;
HRMS (FAB) Calcd for C₂₂H₂₄O₄ (M+H⁺): 353.1753, Found: 353.1760.

### Example 54: Preparation of (S)-2-(3,4-Dimethoxyphenyl)-1-(2,2-dimethyl-2H-chromen-6-yl)propan-1-one (100)

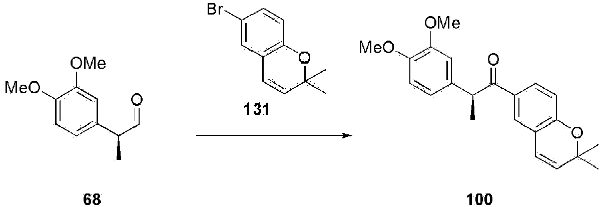

n-BuLi (1.4 equivalent) was added to anhydrous THF solution containing the compound 131 (aryl bromide, 1.5 equivalent) drop by drop at -78°C, which was stirred at -78°C to generate aryl anions. The mixture was stirred at -78°C for 20 minutes, to which the compound 68 (1.0 equivalent) prepared in Manufacturing Example 19 was added, followed by stirring for 30 minutes. The reaction mixture was treated with saturated NH₄Cl aqueous solution, followed by extraction with EtOAc. The extract was washed with brine and then dried over MgSO₄. The residue obtained after evaporating the solvent was purified by flash column chromatography (EtOAc:n-hexane=1:4) to give the secondary alcohol (yield: 59%, 13 mg).

Dess-Martin periodinane (3.0 equivalent) was added to CH₂Cl₂ (0.03 M) solution containing the secondary alcohol (1.0 equivalent) obtained above, followed by stirring for 1 hour. The reaction mixture was diluted with CH₂Cl₂, to which sodium thiosulfate (10%) was added. The mixture was stirred at room temperature for 10 minutes until the two layers were separated. The obtained organic layer was washed with saturated NaHCO₃ aqueous solution and dried over MgSO₄. The organic layer was filtered and concentrated under reduced pressure. Then, the obtained residue was purified by flash column chromatography (EtOAc:n-hexane=1:5) to give the compound 100 (yield: 85%, 9.8 mg, 76% ee).
¹H NMR (CDCl_{3,} 500 MHz) δ 7.74 (dd, 1H, *J* = 8.4, 1.7 Hz), 7.60 (d, 1H, *J* = 1.6 Hz), 6.78 (m, 3H), 6.68 (d, 1H, *J* = 8.5 Hz), 6.28 (d, 1H, *J* = 9.9 Hz), 5.59 (d, 1H, *J* = 9.9 Hz), 4.54 (q, 1H, *J* = 6.8 Hz), 3.82 (s, 3H), 3.80 (s, 3H), 1.46 (d, 3H, *J* = 6.8 Hz), 1.40 (s, 32H), 1.39 (s, H);
¹³C NMR (CDCl₃, 125 MHz) δ 198.9, 157.1, 149.2, 147.8, 134.3, 130.9, 130.5, 129.4, 127.4, 121.7, 120.5, 119.9, 116.0, 111.4, 110.5, 77.5, 55.8, 55.7, 46.8, 28.3, 28.3, 19.6;
HRMS (FAB) Calcd for C₂₂H₂₄O₄ (M+H⁺) : 353.1753, Found: 353.1759.

### Example 55 : Preparation of (7S,7aR, 3aS)-9,10-Dimethoxy-3,3-dimethyl-7-(prop-2-en-oxy)-7,7a,13,13a-tetrahydro-3H-chromeno[3,4-b]pyrano[2,3-h]chromene (101)

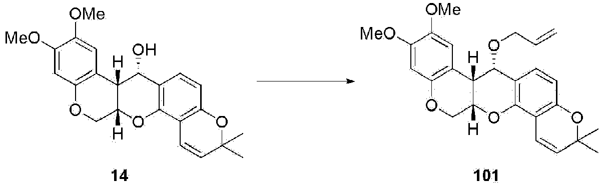

3-iodopropene (1.5 equivalent) was added to anhydrous THF solution containing the compound 14 (1 equivalent) prepared in Example 7 at 0°C, to which t-BuOK solution (1 M in THF solution, 1 equivalent) was added drop by drop at 0°C. While monitoring with TLC, the reaction mixture was stirred at 0°C until the reaction was completed. The mixture was cooled down with saturated NH₄Cl aqueous solution, followed by extraction with EtOAc. The organic layer of the extract was dried over MgSO₄, filtered, and concentrated under reduced pressure. Then, the obtained residue was purified by flash column chromatography (EtOAc:n-hexane=1:5) to give the compound 101 as a colorless solid (yield: 68%, 15 mg).
¹H NMR (CDCl_{3,} 500 MHz) δ 7.93 (d, 1H, *J* = 8.2 Hz), 6.79 (s, 1H), 6.64 (d, 1H, *J* = 9.9 Hz), 6.41 (s, 1H), 6.36 (d, 1H, *J* = 8.2 Hz), 5.66 (m, 1H), 5.54 (d, 1H, *J* = 9.9 Hz), 5.56 (m, 2H), 4.78 (quin, 1H, *J* = 5.3Hz), 4.57 (m, 2H), 4.23 (dd, 1H, *J* = 9.4, 3.8 Hz) 3.90 (m, 1H), 3.81 (s, 3H), 1.39 (s, 3H);
¹³C NMR (CDCl₃, 125 MHz) δ 154.1, 149.1, 149.0, 148.5, 143.4, 134.7, 129.0, 128.9, 116.5, 116.4, 113.9, 111.6, 109.9, 109.9, 108.5, 100.3, 75.9, 73.8, 70.0, 69.6, 65.8, 56.5, 55.7, 37.0, 27.9, 27.8.

### Comparative Example 1: Preparation of (7aS,13aS)-9,10-Dimethoxy-3,3-dimethyl-13,13a-dihydro-3H-chromeno[3,4-b]pyrano[2,3-h]chromen-7(7aH)-one (Deguelin)

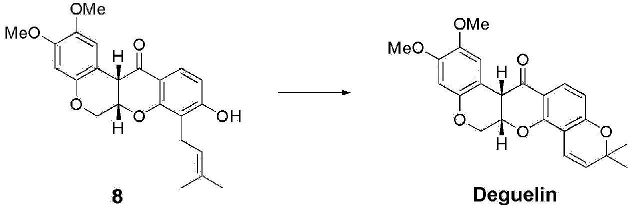

Phenylselenyl chloride (68 mg, 0.35 mmol) was added to anhydrous CH₂Cl₂ solution (4.0 mL) containing the compound 8 (128 mg, 0.32 mmol) prepared in Example 1 at -30°C under argon atmosphere, followed by stirring for 10 minutes with maintaining the temperature at -30°C. The temperature was raised to room temperature with stirring for 2 hours, and then additional stirring was performed for 1 more hour. The solvent was eliminated from the reaction mixture under reduced pressure and the obtained residue was dissolved in THF (4.0 mL), to which hydrogen peroxide (30% in water, 0.06 mL) was added 0°C. The reaction mixture was stirred until the temperature of the mixture reached to room temperature, during which the reaction was monitored with TLC. EtOAc (8.0 mL) and water (4.0 mL) were added thereto. The organic layer was separated, washed with 5% NaHCO₃ aqueous solution and brine, dried over MgSO₄, filtered, and then concentrated. The obtained non-purified residue was purified by flash column chromatography (EtOAc:n-hexane=1:2) to give deguelin as a light-yellow solid (yield: 61%, 78 mg).
¹H-NMR (CDCl₃, 400 MHz) δ 7.72 (d, 1H, *J* = 8.7 Hz), 6.77 (s, 1H), 6.62 (d, 1H, *J* = 10.0 Hz), 6.43 (s, 1H), 6.43 (d, 1H, *J* = 8.7 Hz), 5.53 (d, 1H, *J* = 10.0 Hz), 4.89 (m, 1H), 4.61 (dd, 1H, *J* = 12.0, 3.1 Hz), 4.17 (d, 1H, *J* = 12.0 Hz), 3.82 (d, 1H, *J* = 4.1 Hz), 3.78 (s, 3H), 3.75 (s, 3H), 1.43 (s, 3H), 1.36 (s, 3 H) ;
¹³C-NMR (CDCl₃, 100 MHz) δ 189.2, 160.0, 156.9, 149.4, 147.4, 143.8, 128.6, 128.5, 115.7, 112.7, 111.4, 110.4, 109.1, 104.7, 100.9, 77.6, 72.4, 66.2, 56.3, 55.8, 44.3, 28.4, 28.1;
HRMS (FAB) Calcd for C₂₃H₂₃O₆ (M+H⁺): 395.1495, Found: 395.1495.

### Experimental Example 1: Evaluation of cancer cell growth inhibition

To investigate the inhibitory effect of the compounds of Examples of the present invention on cancer cell growth, the following experiment was performed. Particularly, H1299 NSCLC (non-small cell lung cancer) cells were distributed in a 96-well plate at the density of 5 x 10³ cells/well, followed by culture in a 37°C, 5% CO₂ incubator for 24 hours. The compound of each example was dissolved in DMSO (10 nM, 100 nM, 1 uM, 10 uM), which was treated to each well. 48 hours later, cell growth was measured by using MTS reagent (3-(4,5-dimethylthiazole-2-yl)-5-(3-carboxymethoxyphenyl)-2-(4-sulfophenyl)-2H-tetrazolium). The experiment was performed in triplicate and the standard error of the analysis result was corrected. The results are presented in Table 2.

**[Table 2]**

| Compound | IC₅₀ (µM) |
|---|---|
| Comparative Example 1 (Deguelin) | 0.11 |
| Example 1 (Compound 8) | NA |
| Example 2 (Compound 9) | 9.8 |
| Example 3 (Compound 10) | 0.87 |
| Example 4 (Compound 11) | 0.97 |
| Example 5 (Compound 12) | 10.1 |
| Example 7 (Compound 13) | 4.2 |
| Example 9 (Compound 16) | 9.8 |
| Example 10 (Compound 17) | 10.5 |
| Example 11 (Compound 18) | NA |
| Example 12 (Compound 19) | NA |
| Example 13 (Compound 20) | 0.68 |
| Example 15 (Compound 22) | NA |
| Example 16 (Compound 23) | 0.3 |
| Example 17 (Compound 24) | 4.3 |
| Example 18 (Compound 25) | NA |
| Example 20 (Compound 27) | NA |
| Example 21 (Compound 28) | 5.2 |
| Example 22 (Compound 29) | NA |
| Example 23 (Compound 30) | NA |
| Example 24 (Compound 31) | NA |
| Example 25 (Compound 37) | 4.5 |
| Example 26 (Compound 45) | 3.8 |
| Example 27 (Compound 53) | 1.0 |
| Example 28 (Compound (54) | 0.14 |
| Example 29 (Compound 56) | 0.73 |
| Example 30 (Compound 57) | 3.2 |
| Example 32 (Compound 59) | 102 |
| Example 33 (Compound 69) | 0.49 |
| Example 34 (Compound 72) | 1.3 |

As shown in Table 2, when the compounds of the present invention were treated to H1299 NSCLC cells, the mean value of cell growth IC₅₀ was approximately 3 ∼ 5 µM. In particular, IC₅₀ of the compound 54 of Example 28 was 0.14 µM, indicating that the compound had similar cancer cell growth inhibiting effect to the conventional anticancer agent deguelin of Comparative Example 1.

Therefore, the compounds of the present invention can be effectively used as an anticancer agent since they have excellent cancer cell growth inhibiting effect.

### Experimental Example 2: Evaluation of HIF-1α inhibiting effect

The inhibitory effect of the compounds of the invention that had confirmed previously to have a strong cell growth inhibitory activity in Experimental Example 1 on the accumulation of HIF-1α was investigated in the lung cancer cell line H1299 NSCLC. Particularly, to confirm whether or not the compounds prepared in Examples of the present invention could inhibit HIF-1α generation induced under hypoxic condition dose-dependently, the compounds prepared in Examples and deguelin prepared in Comparative Example 1 were treated to the cells at different concentrations and then HIF-1α generation was investigated by Western blotting.

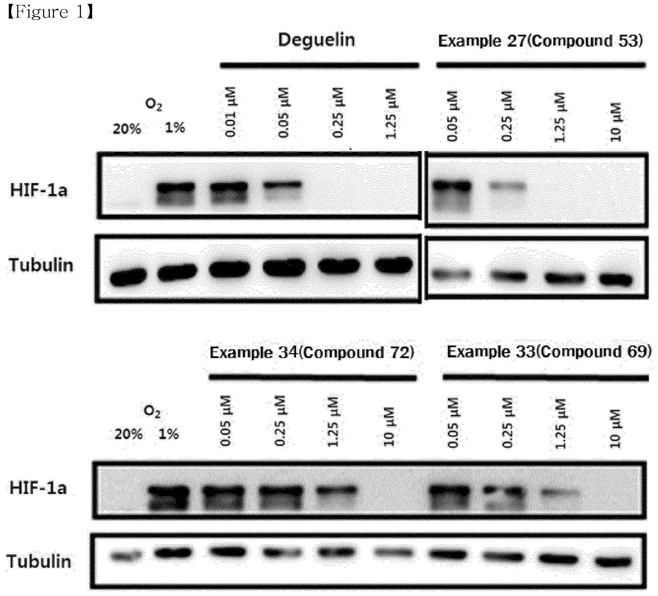

First, H1299 NSCLC (non-small cell lung cancer) cells were distributed in a 96-well plate at the density of 5 x 10³ cells/well, followed by culture for 24 hours. The cells were pre-treated under hypoxic condition (oxygen 1%, nitrogen 94%, carbon dioxide 5%) for 12 hours to induce the accumulation of HIF-1α. The compound of formula 1 was dissolved in DMSO, which was treated to H1299 NSCLC cells at the concentrations of 0 ∼ 10 µM. The cells were cultured under hypoxic condition for 16 hours. Then, the nucleus extract was prepared by using RIPA buffer. At this time, to compare the HIF-1 target gene expression according to hypoxia, the control group was cultured under 20% oxygen condition. 30 *µ*g of the nucleus extract was separated by SDS-PAGE (sodium dodecyl sulfate-polyacrylamide gel electrophoresis), which was transferred onto polyvinylidene fluoride membrane. HIF-1α was quantified by using HIF-1α antibody (BD Pharmingen) and HRP (horseradish peroxidase) conjugated secondary antibody. Tublin was used as the internal control gene. The results are presented in Figure 1.

Figure 1 is a diagram illustrating the result of Western blotting performed to measure the activity of the compounds of the present invention to inhibit the accumulation of HIF-1α.

As shown in Figure 1, the compounds prepared in Examples of the present invention were confirmed to inhibit HIF-1α generation dose-dependently without damaging tublin generation under hypoxic condition.

Therefore, it was confirmed that the compounds of the present invention inhibit the accumulation of HIF-1α characterized by aggravating cancer, so that they can be effectively used as an active ingredient of an anticancer agent.

### Experimental Example 3: Evaluation of angiogenesis inhibiting effect

To investigate the inhibitory effect of the compounds of the present invention on VEGF (Vascular endothelial growth factor A), the angiogenesis factor, angiogenesis in the transgenic zebra fish was examined.

After cross-breeding zebra fish, the generated zebra fish embryos (Albino;*Tg(fli1:EGFP)^{b4;y1},* Zebra fish International Resource Center, University of Oregon, Eugene) were cultured in 28.5°C amniotic fluid (Artificial sea salt was dissolved in distilled water at the concentration of 0.2 g/L.) for 6 hours. 10 zebra fish embryos cultured above were grouped together, which were distributed in a 24-well plate, followed by culture along with 500 *µ*ℓ of the amniotic fluid. The compound of each example was treated thereto at the concentrations of 50 nM, 250 nM, and 1.25 µM. 48 hours later, during which the experiment was kept going on, the amniotic fluid (including each compound) was replaced. 72 hours later, angiogenesis in the subintestinal vessel plexus (SIV) of each embryo was examined and photographed under DM5000B fluorescent microscope (Leica Microsystems, Wetzlar GmbH, Germany).

Figure 2 is a photograph illustrating the effect of the compound 53 of the present invention on angiogenesis.

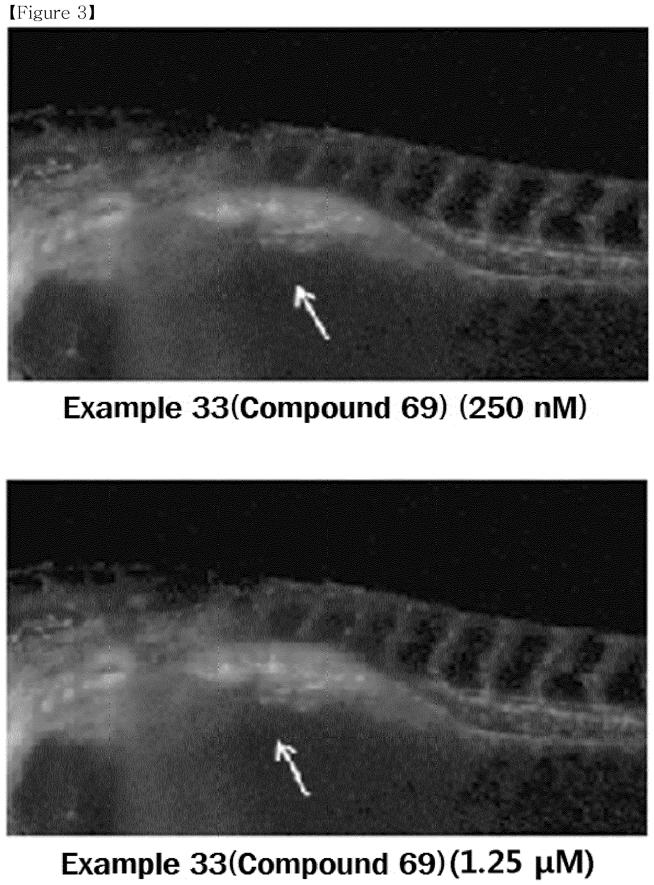

Figure 3 is a photograph illustrating the effect of the compound 69 of the present invention on angiogenesis.

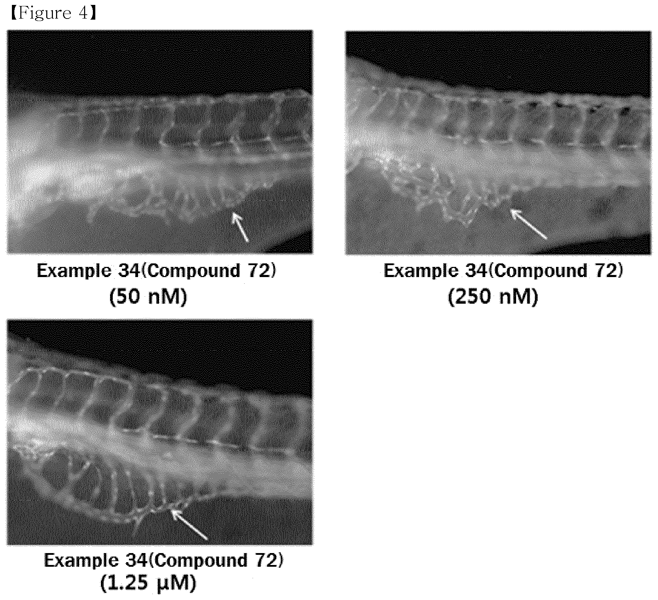

Figure 4 is a photograph illustrating the effect of the compound 72 of the present invention on angiogenesis.

As shown in Figures 2, 3, and 4, when the embryo was treated with the compound of the present invention, angiogenesis was reduced by the compound dose-dependently, compared with the other embryo non-treated with the compound.

Therefore, the compounds of the present invention can inhibit angiogenesis, that is they can inhibit the activity of VEGF, the angiogenesis factor, so that they can be effectively used as an active ingredient of an anticancer agent.

### Experimental Example 4: Evaluation of angiogenesis inhibiting effect

Following experiment was performed to investigate the angiogenesis inhibiting effect of deguelin and the compound of each example under hypoxic condition by inhibiting VEGF generation.

### Preparation stage: Preparation of cell culture fluid

First, HCT116, the human colorectal cancer cell line, was cultured in a hypoxic chamber (1% O₂) as the *in vitro* hypoxic model. The cells were treated with deguelin, compound 69 (Example 33) and compound 54 (Example 28) at the concentrations of 1 ∼ 100 nM, followed by culture for 8 hours. Next, the culture fluid was collected and proceeded to centrifugation at 4°C for 10 minutes (6,000 x g). Cell culture fluid was obtained from the supernatant (conditioned media (CM)). The obtained cell culture fluid was treated to human umbilical vein endothelial cells (HUVECs) which were cultured separately. Then changes of angiogenesis were investigated by the following experiments.

### <4-1> Evaluation of vascular endothelial cell proliferation inhibiting effect

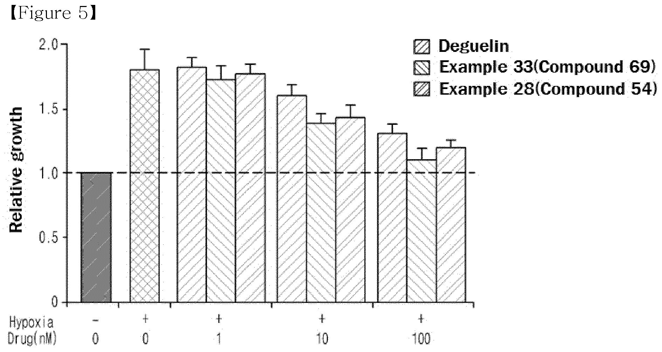

The cell culture fluid prepared in the above preparation stage was treated to umbilical vein endothelial cells (HUVECs) for 18 hours. Cell proliferation was confirmed by [³H]-thymidine incorporation assay. The result is presented in Figure 5.

Figure 5 is a graph illustrating the inhibitory effect of the compound of the present invention on the proliferation of vascular endothelial cells.

As shown in Figure 5, compared with the control (deguelin), the compound 69 of Example 33 and the compound 54 of Example 28 inhibited the proliferation of vascular endothelial cells dose-dependently, and the inhibition effect was greater than that of deguelin.

### <4-2> Evaluation of vascular endothelial cell migration inhibiting effect

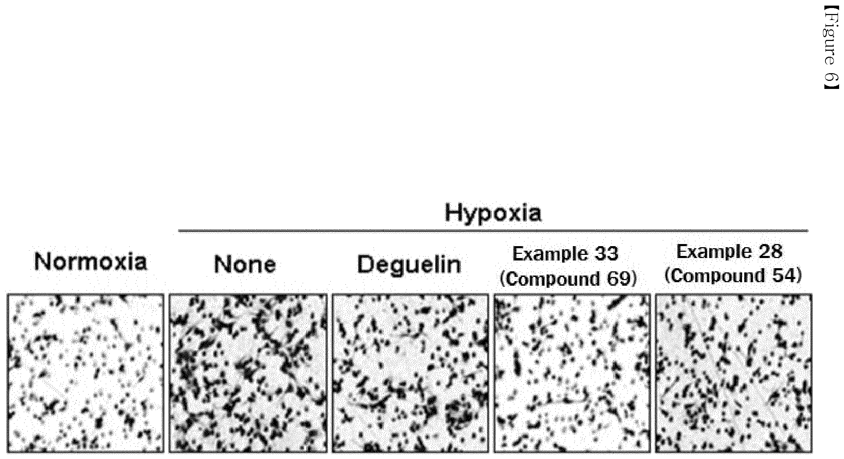

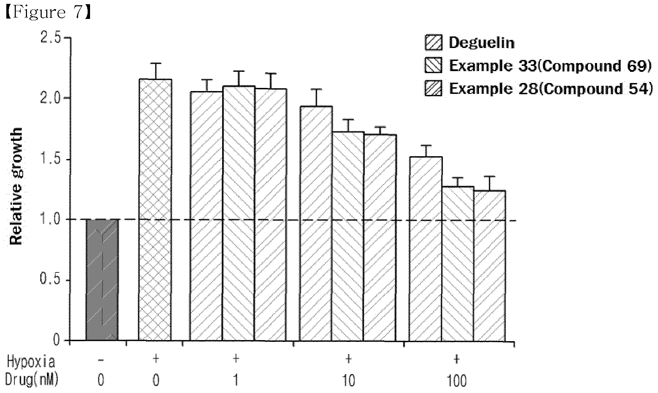

The cell culture fluid obtained in the above preparation stage was loaded in the lower chamber of trans-well plate (6.5 mm diameter filter) and umbilical vein endothelial cells (HUVECs) were plated carefully in the upper chamber, followed by culture at 37°C for 4 hours. The cells migrated down the filter were confirmed by H&E staining. The results are presented in Figure 6 and Figure 7.

Figure 6 is a diagram illustrating the inhibitory effect of the compound of the present invention on the migration of vascular endothelial cells.

Figure 7 is a graph illustrating the inhibitory effect of the compound of the present invention on the migration of vascular endothelial cells.

As shown in Figure 6 and Figure 7, the cell culture fluid treated with the compound 69 (Example 33) and the compound 54 (Example 28) at the concentration of 100 nM showed the inhibitory effect on the migration of vascular endothelial cells, and such effect was greater than that of the control, deguelin. In the meantime, the inhibitory effect of the above compounds on vascular endothelial cell migration was dose-dependent.

### <4-3> Evaluation of vascular endothelial cell tube formation inhibiting effect

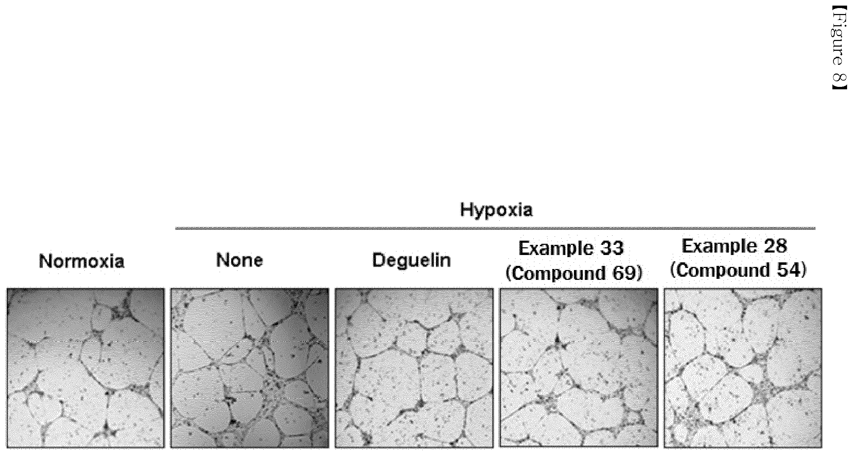

Umbilical vein endothelial cells (HUVECs) were treated with the cell culture fluid obtained in the above preparation stage. 30 hours later, tube formation was observed. As a result, tube formation was induced by the control deguelin and the compounds of the present invention. The results are presented in Figure 8.

Figure 8 is a diagram illustrating the inhibitory effect of the compound of the present invention on the tube formation of vascular endothelial cells.

As shown in Figure 8, the inhibitory effect of the compound 69 (Example 33) and the compound 54 (Example 28) on the tube formation was greater than that of the control deguelin.

Therefore, it was confirmed that the compounds of the present invention have excellent anti-angiogenesis effect, compared with that of the conventional anticancer agent deguelin, so that they can be effectively used as an active ingredient of an anticancer agent.

### Experimental Example 5: Cytotoxicity test

To investigate the cytotoxicity of the control deguelin, the compound 69 (Example 33), and the compound 54 (Example 28) to the normal cells, it was examined how these compounds affect the proliferation of human bronchial epithelial cells (HBEC-1 and BEAS-2B).

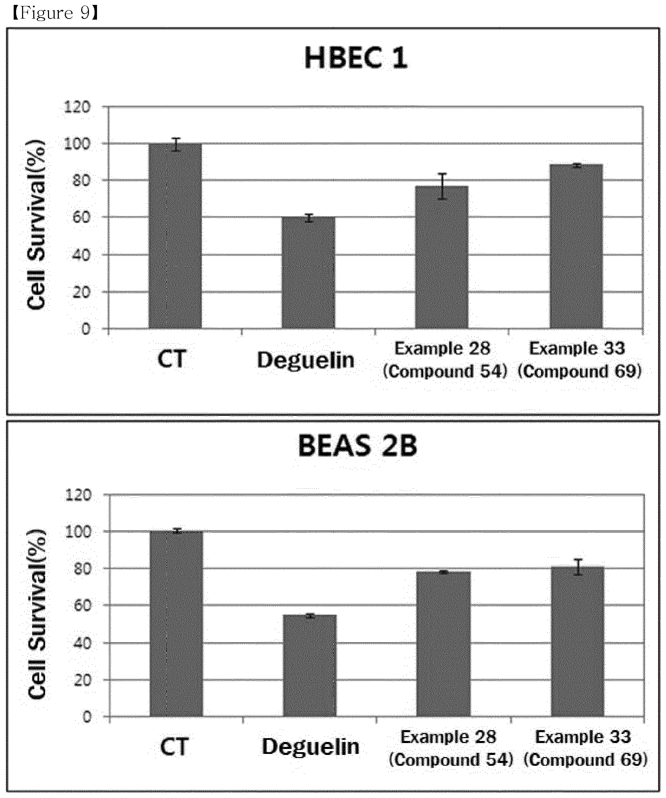

Particularly, HBEC-1 and BEAS-2B cells were seeded on a 96-well culture plate (5,000 cells/well), followed by culture for 24 hours. The plate was treated with 0.1% DMSO (comparative control (CT)), 1 µM of deguelin (control), or 1 µM of the compound 69 (Example 33) and the compound 54 (Example 28) (experimental group), followed by further culture for 3 days. Cytotoxicity was evaluated by MTT assay. The results are presented in Figure 9.

Figure 9 is a graph illustrating the cell survival rate (%) obtained by MTT assay performed to investigate the cytotoxicity of the compound of the present invention.

As shown in Figure 9, when deguelin was treated, the cell survival rate was approximately 60%. In the meantime, when the compound 69 of Example 33 and the compound 54 of Example 28 were treated, the cell survival rate was approximately 80%. The above results indicate that the compounds of the present invention have the cancer cell specific cytotoxicity.

Therefore, it was confirmed that the compounds of the present invention have significantly lower cytotoxicity to normal cells than the conventional anticancer agent deguelin, so that they can be effectively used as an active ingredient of an anticancer agent.

The Manufacturing Examples of the composition for the present invention are described hereinafter.

### Manufacturing Example 1: Preparation of powders

| | |
|---|---|
| Compound of formula 1 or formula 2 | 2 g |
| Lactose | 1 g |

Powders were prepared by mixing all the above components, which were filled in airtight packs according to the conventional method for preparing powders.

### Manufacturing Example 2: Preparation of tablets

| | |
|---|---|
| Compound of formula 1 or formula 2 | 10 mg |
| Corn starch | 10 mg |
| Lactose | 10 mg |
| Magnesium stearate | 2 mg |

Tablets were prepared by mixing all the above components by the conventional method for preparing tablets.

### Manufacturing Example 3: Preparation of capsules

| | |
|---|---|
| Compound of formula 1 or formula 2 | 10 mg |
| Corn starch | 10 mg |
| Lactose | 10 mg |
| Magnesium stearate | 2 mg |

Capsules were prepared by mixing all the above components, which were filled in gelatin capsules according to the conventional method for preparing capsules.

### Manufacturing Example 4: Preparation of injectable solutions

| | |
|---|---|
| Compound of formula 1 or formula 2 | 10 *µ*g/mℓ |
| Weak HC1 BP | until pH 3.5 |
| Injectable NaCl BP | up to 1 mℓ |

The compound of formula 1 or formula 2 of the present invention was dissolved in proper volume of injectable NaCl BP. pH of the prepared solution was regulated as 3.5 by using weak HCl BP. The volume was adjusted by using injectable NaCl BP. The solution was well mixed and filled in 5 m*ℓ* type I transparent glass ampoules. The ampoules were sealed by melting the glass of opening, followed by autoclave at 120°C for at least 15 minutes for sterilization.

### INDUSTRIAL APPLICABILITY

The compounds represented by formula 2 of the present invention suppress the expression of Hsp90 so that they can inhibit the accumulation of HIF-1α, the Hsp90 client protein, and also efficiently inhibit the activation of VEGF. In addition, these compounds display low cytotoxicity, so that they can be effectively used as an active ingredient of a diabetic retinopathy treating agent, and an anti-arthritic agent.

Those skilled in the art will also appreciate that equivalent embodiments do not depart from the scope of the invention as set forth in the appended Claims.

## Claims

1. A compound represented by formula 2 or the pharmaceutically acceptable salt thereof: 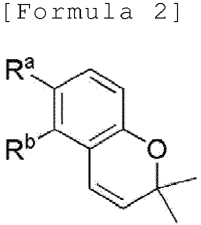 (In formula 2, R^{a} is 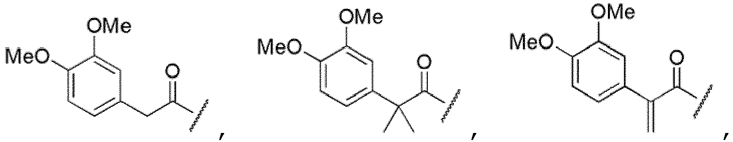 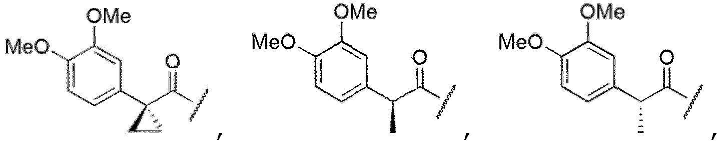 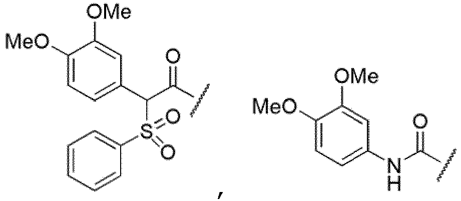 or 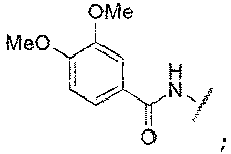 and
R^{b} is -H, -OH or C₁₋₃ alkoxy;
for use in the prevention or treatment of diabetic retinopathy or rheumatoid arthritis.

2. The compound for use according to claim 1, wherein:
R^{b} is -H, -OH or C₁₋₂ alkoxy.

3. The compound for use according to claim 1, wherein:
R^{b} is -H, -OH or methoxy.

4. A compound for use in the prevention or treatment of diabetic retinopathy or rheumatoid arthritis, wherein the compound is selected from the group consisting of the following compounds:
2-(3,4-dimethoxyphenyl)-1-(2,2-dimethyl-2*H-*chromen-6-yl)ethanone,
2-(3,4-dimethoxyphenyl)-1-(5-methoxy-2,2-dimethyl-2*H*-chromen-6-yl)ethanone,
2-(3,4-dimethoxyphenyl-1-(5-methoxy-2,2-dimethyl-2*H*-chromen-6-yl)propan-1-one
2-(3,4-dimethoxyphenyl)-1-(5-methoxy-2,2-dimethyl-2*H*-chromen-6-yl)-2-methylpropan-1-one,
2-(3,4-dimethoxyphenyl)-1-(5-methoxy-2,2-dimethyl-2*H*-chromen-6-yl)prop-2-en-1-one,
1-(3,4-dimethoxyphenyl)cyclopropyl)(5-methoxy-2,2-dimethyl-2*H*-chromen-6-yl)methanone,
(*S*)-2-(3,4-dimethoxyphenyl)-1-(5-methoxy-2,2-dimethyl-2*H*-chromen-6-yl)propan-1-one,
(*R*)-2-(3,4-dimethoxyphenyl)-1-(5-methoxy-2,2-dimethyl-2*H*-chromen-6-yl)propan-1-one,
2-(3,4-dimethoxyphenyl)-1-(5-hydroxy-2,2-dimethyl-2*H*-chromen-6-yl)-2-(phenylsulfonyl)ethanone,
(3,4-dimethoxyphenyl)(2,2-dimethyl-2*H*-chromen-6-yl)methanone,
(*E*)-1-(3,4-dimethoxyphenyl)-3-(2,2-dimethyl-2*H-*chromen-6-yl)prop-2-en-1-one,
(*E*)-3-(3,4-dimethoxyphenyl)-1-(5-hydroxy-2,2-dimethyl-2*H*-chromen-6-yl)prop-2-en-1-one,
(*E*)-3-(3,4-dimethoxyphenyl)-1-(2,2-dimethyl-2*H-*chromen-6-yl)prop-2-en-1-one,
(*E*)-1-(5-hydroxy-2,2-dimethyl-2*H*-chromen-6-yl)-3-(2,4,5-trimethoxyphenyl)prop-2-en-1-one,
(*E*)-3-(3,4-dimethoxyphenyl)-1-(5-methoxy-2,2-dimethyl-2*H*-chromen-6-yl)prop-2-en-1-one,
(*E*)-1-(5-methoxy-2,2-dimethyl-2*H*-chromen-6-yl)-3-(2,4,5-trimethoxyphenyl)prop-2-en-1-one,
2-(3,4-dimethoxyphenyl)-*N*-(5-methoxy-2,2-dimethyl-2*H*-chromen-6-yl)acet-amide,
*N*-(3,4-dimethoxybenzyl)-2,2-dimethyl-2*H*-chromen-6-carboxamide,
*N*-(3,4-dimethoxyphenyl)-2,2-dimethyl-2*H*-chromen-6-carboxamide,
*N*-(2,2-dimethyl-2H-chromen-6-yl)-3,4-dimethoxybenzamide,
(*R*)-2-(3,4-dimethoxyphenyl)-1-(2,2-dimethyl-2*H-*chromen-6-yl)propan-1-one, and
(*S*)-2-(3,4-dimethoxyphenyl)-1-(2,2-dimethyl-2*H-*chromen-6-yl)propan-1-one.

5. A compound represented by formula 2 or the pharmaceutically acceptable salt thereof: 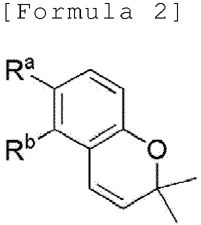 (In formula 2, R^{a} is 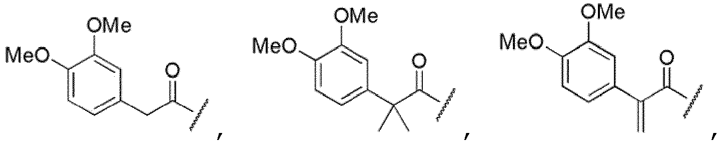 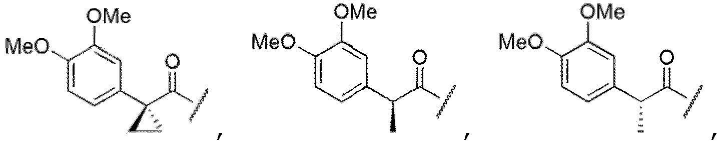 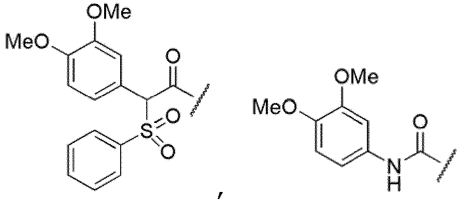 or 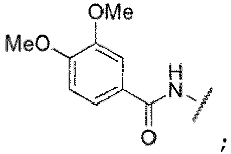 and
R^{b} is C₁₋₃ alkoxy.

6. The compound represented by formula 2 or the pharmaceutically acceptable salt thereof according to claim 5, wherein:
R^{b} is C₁₋₂ alkoxy.

7. The compound represented by formula 2 or the pharmaceutically acceptable salt thereof according to claim 5, wherein:
R^{b} is methoxy.

8. A compound, or the pharmaceutically acceptable salt thereof, selected from the group consisting of the following compounds:
2-(3,4-dimethoxyphenyl)-1-(5-methoxy-2,2-dimethyl-2H-chromen-6-yl)ethanone,
2-(3,4-dimethoxyphenyl-1-(5-methoxy-2,2-dimethyl-2*H*-chromen-6-yl)propan-1-one
2-(3,4-dimethoxyphenyl)-1-(5-methoxy-2,2-dimethyl-2*H*-chromen-6-yl)-2-methylpropan-1-one,
2-(3,4-dimethoxyphenyl)-1-(5-methoxy-2,2-dimethyl-2*H*-chromen-6-yl)prop-2-en-1-one,
1-(3,4-dimethoxyphenyl)cyclopropyl)(5-methoxy-2,2-dimethyl-2H-chromen-6-yl)methanone,
(*S*)-2-(3,4-dimethoxyphenyl)-1-(5-methoxy-2,2-dimethyl-2H-chromen-6-yl)propan-1-one,
(R)-2-(3,4-dimethoxyphenyl)-1-(5-methoxy-2,2-dimethyl-2*H*-chromen-6-yl)propan-1-one,
2-(3,4-dimethoxyphenyl)-1-(5-hydroxy-2,2-dimethyl-2H-chromen-6-yl)-2-(phenylsulfonyl)ethanone,
(*E*)-1-(3,4-dimethoxyphenyl)-3-(2,2-dimethyl-2*H-*chromen-6-yl)prop-2-en-1-one,
(*E*)-3-(3,4-dimethoxyphenyl)-1-(2,2-dimethyl-2*H-*chromen-6-yl)prop-2-en-1-one,
(*E*)-3-(3,4-dimethoxyphenyl)-1-(5-methoxy-2,2-dimethyl-2*H*-chromen-6-yl)prop-2-en-1-one,
(*E*)-1-(5-methoxy-2,2-dimethyl-2H-chromen-6-yl)-3-(2,4,5-trimethoxyphenyl)prop-2-en-1-one,
2-(3,4-dimethoxyphenyl)-N-(5-methoxy-2,2-dimethyl-2H-chromen-6-yl)acet-amide,
N-(2,2-dimethyl-2H-chromen-6-yl)-3,4-dimethoxybenzamide,
(*R*)-2-(3,4-dimethoxyphenyl)-1-(2,2-dimethyl-2*H-*chromen-6-yl)propan-1-one, and
(*S*)-2-(3,4-dimethoxyphenyl)-1-(2,2-dimethyl-2*H-*chromen-6-yl)propan-1-one.

9. A pharmaceutical composition comprising the compound represented by formula 2 of claim 5, or the pharmaceutically acceptable salt thereof, or a compound according to claim 8, or the pharmaceutically acceptable salt thereof, as an active ingredient.

10. A compound represented by formula 2 of claim 5, or the pharmaceutically acceptable salt thereof, or a compound according to claim 8, or the pharmaceutically acceptable salt thereof, for use as a medicament.

## Patentansprüche

1. Verbindung, die durch Formel 2 dargestellt wird, oder das pharmazeutisch geeignete Salz davon: 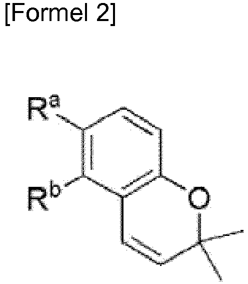 (In Formel 2 ist R^{a} 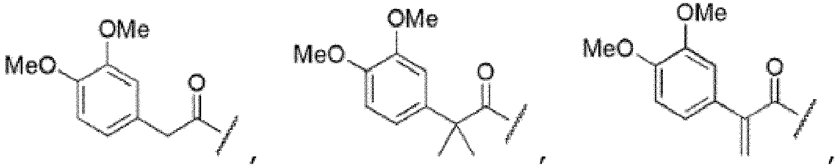 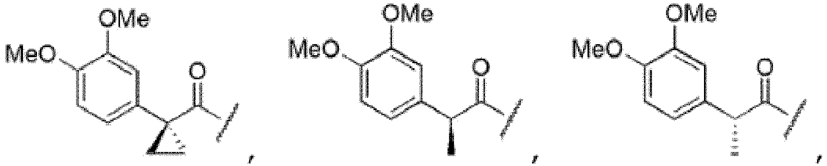 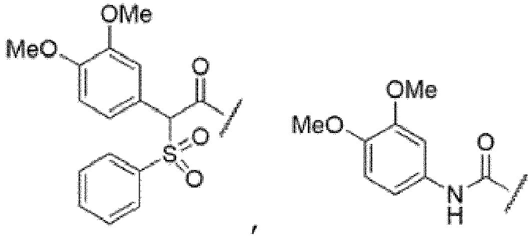 oder 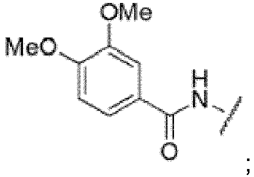 und
R^{b} ist -H, -OH oder C₁₋₃-Alkoxy);
für die Verwendung bei der Verhinderung oder Behandlung von diabetischer Retinopathie oder rheumatoider Arthritis.

2. Verbindung zur Verwendung nach Anspruch 1, wobei:
R^{b} -H, -OH oder C₁₋₂-Alkoxy ist.

3. Verbindung zur Verwendung nach Anspruch 1, wobei:
R^{b} -H, -OH oder Methoxy ist.

4. Verbindung zur Verwendung bei der Verhinderung oder Behandlung von diabetischer Retinopathie oder rheumatoider Arthritis, wobei die Verbindung aus der Gruppe ausgewählt ist, die aus den folgenden Verbindungen besteht:
2-(3,4-Dimethoxyphenyl)-1-(2,2-dimethyl-2*H*-chromen-6-yl)ethanon,
2-(3,4-Dimethoxyphenyl)-1-(5-methoxy-2,2-dimethyl-2H-chromen-6-yl)ethanon,
2-(3,4-Dimethoxyphenyl-1-(5-methoxy-2,2-dimethyl-2*H*-chromen-6-yl)propan-1-on,
2-(3,4-Dimethoxyphenyl)-1-(5-methoxy-2,2-dimethyl-2*H*-chromen-6-yl)-2-methylpropan-1-on,
2-(3,4-Dimethoxyphenyl)-1-(5-methoxy-2,2-dimethyl-2*H*-chromen-6-yl)prop-2-en-1-on,
1-(3,4-Dimethoxyphenyl)cyclopropyl)(5-methoxy-2,2-dimethyl-2H-chromen-6-yl)methanon,
(S)-2-(3,4-Dimethoxyphenyl)-1-(5-methoxy-2,2-dimethyl-2H-chromen-6-yl)propan-1-on,
(*R*)-2-(3,4-Dimethoxyphenyl)-1-(5-methoxy-2,2-dimethyl-2*H*-chromen-6-yl)propan-1-on,
2-(3,4-Dimethoxyphenyl)-1-(5-hydroxy-2,2-dimethyl-2*H*-chromen-6-yl)-2-(phenylsulfonyl)ethanon,
(3,4-Dimethoxyphenyl)(2,2-dimethyl-2*H*-chromen-6-yl)methanon,
(*E*)-1-(3,4-Dimethoxyphenyl)-3-(2,2-dimethyl-2*H*-chromen-6-yl)prop-2-en-1-on,
(*E*)-3-(3,4-Dimethoxyphenyl)-1-(5-hydroxy-2,2-dimethyl-2*H*-chromen-6-yl)prop-2-en-1-on,
(*E*)-3-(3,4-Dimethoxyphenyl)-1-(2,2-dimethyl-2*H*-chromen-6-yl)prop-2-en-1-on,
(*E*)-1-(5-Hydroxy-2,2-dimethyl-2H-chromen-6-yl)-3-(2,4,5-trimethoxyphenyl)prop-2-en-1-on,
(*E*)-3-(3,4-Dimethoxyphenyl)-1-(5-methoxy-2,2-dimethyl-2*H*-chromen-6-yl)prop-2-en-1-on,
(*E*)-1-(5-Methoxy-2,2-dimethyl-2*H*-chromen-6-yl)-3-(2,4,5-trimethoxyphenyl)prop-2-en-1-on,
2-(3,4-Dimethoxyphenyl)-*N*-(5-methoxy-2,2-dimethyl-2*H*-chromen-6-yl)acet-amid,
*N*-(3,4-Dimethoxybenzyl)-2,2-dimethyl-2*H*-chromen-6-carboxamid,
*N*-(3,4-Dimethoxyphenyl)-2,2-dimethyl-2*H*-chromen-6-carboxamid,
*N*-(2,2-Dimethyl-2*H*-chromen-6-yl)-3,4-dimethoxybenzamid,
(*R*)-2-(3,4-Dimethoxyphenyl)-1-(2,2-dimethyl-2*H*-chromen-6-yl)propan-1-on und
(*S*)-2-(3,4-Dimethoxyphenyl)-1-(2,2-dimethyl-2*H*-chromen-6-yl)propan-1-on.

5. Verbindung, die durch Formel 2 dargestellt wird, oder das pharmazeutisch geeignete Salz davon: 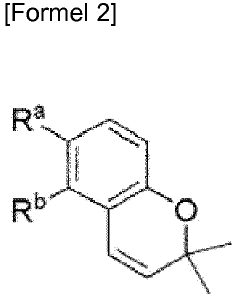 (In Formel 2 ist R^{a} 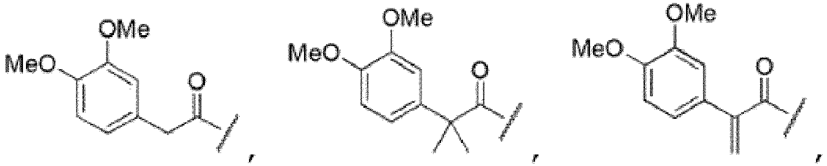 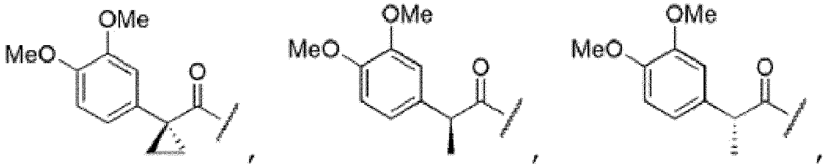 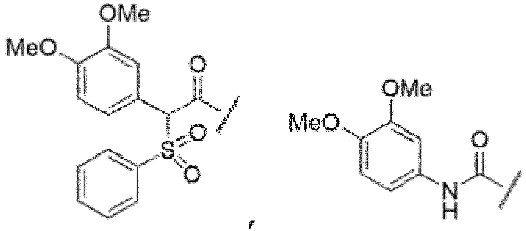 oder 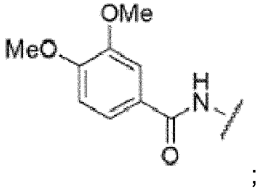 und
R^{b} ist C₁₋₃-Alkoxy.

6. Verbindung, die durch Formel 2 dargestellt wird, oder das pharmazeutisch geeignete Salz davon nach Anspruch 5, wobei:
R^{b} C₁₋₂-Alkoxy ist.

7. Verbindung, die durch Formel 2 dargestellt wird, oder das pharmazeutisch geeignete Salz davon nach Anspruch 5, wobei:
R^{b} Methoxy ist.

8. Verbindung oder das pharmazeutisch geeignete Salz davon, gewählt aus der Gruppe bestehend aus den folgenden Verbindungen:
2-(3,4-Dimethoxyphenyl)-1-(5-methoxy-2,2-dimethyl-2*H*-chromen-6-yl)ethanon,
2-(3,4-Dimethoxyphenyl-1-(5-methoxy-2,2-dimethyl-2*H*-chromen-6-yl)propan-1-on,
2-(3,4-Dimethoxyphenyl)-1-(5-methoxy-2,2-dimethyl-2*H*-chromen-6-yl)-2-methylpropan-1-on,
2-(3,4-Dimethoxyphenyl)-1-(5-methoxy-2,2-dimethyl-2*H*-chromen-6-yl)prop-2-en-1-on,
1-(3,4-Dimethoxyphenyl)cyclopropyl)(5-methoxy-2,2-dimethyl-2H-chromen-6-yl)methanon,
(*S*)-2-(3,4-Dimethoxyphenyl)-1-(5-methoxy-2,2-dimethyl-2*H*-chromen-6-yl)propan-1-on,
(R)-2-(3,4-Dimethoxyphenyl)-1-(5-methoxy-2,2-dimethyl-2H-chromen-6-yl)propan-1-on,
2-(3,4-Dimethoxyphenyl)-1-(5-hydroxy-2,2-dimethyl-2*H*-chromen-6-yl)-2-(phenylsulfonyl)ethanon,
(*E*)-1-(3,4-Dimethoxyphenyl)-3-(2,2-dimethyl-2*H*-chromen-6-yl)prop-2-en-1-on,
(*E*)-3-(3,4-Dimethoxyphenyl)-1-(2,2-dimethyl-2H-chromen-6-yl)prop-2-en-1-on,
(*E*)-3-(3,4-Dimethoxyphenyl)-1-(5-methoxy-2,2-dimethyl-2*H*-chromen-6-yl)prop-2-en-1-on,
(*E*)-1-(5-Methoxy-2,2-dimethyl-2*H*-chromen-6-yl)-3-(2,4,5-trimethoxyphenyl)prop-2-en-1-on,
2-(3,4-Dimethoxyphenyl)-*N*-(5-methoxy-2,2-dimethyl-2*H*-chromen-6-yl)acet-amid,
*N*-(2,2-dimethyl-2*H*-chromen-6-yl)-3,4-Dimethoxybenzamid
(*R*)-2-(3,4-Dimethoxyphenyl)-1-(2,2-dimethyl-2*H*-chromen-6-yl)propan-1-on und
(*S*)-2-(3,4-Dimethoxyphenyl)-1-(2,2-dimethyl-2*H*-chromen-6-yl)propan-1-on.

9. Pharmazeutische Zusammensetzung mit der durch Formel 2 von Anspruch 5 dargestellten Verbindung oder dem pharmazeutisch geeigneten Salz davon oder Verbindung nach Anspruch 8 oder das pharmazeutisch geeignete Salz davon als Wirkstoff.

10. Verbindung, die durch Formel 2 von Anspruch 5 dargestellt wird, oder das pharmazeutisch geeignete Salz davon oder Verbindung nach Anspruch 8 oder das pharmazeutisch geeignete Salz davon zur Verwendung als Medikament.

## Revendications

1. Composé représenté par la formule 2 ou son sel pharmaceutiquement acceptable : 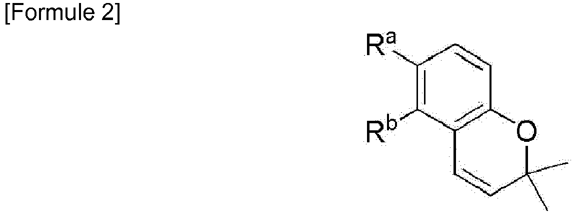 (dans la formule 2, R^{a} est 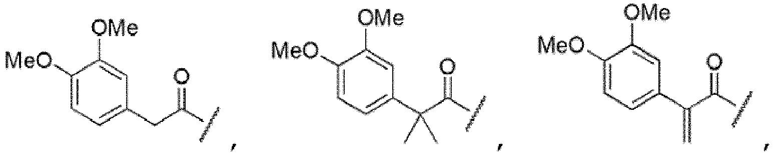 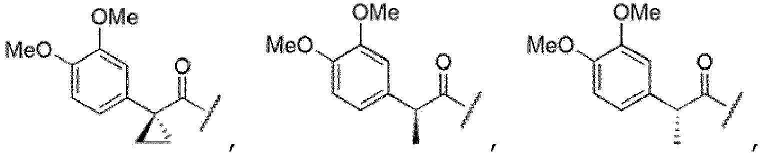 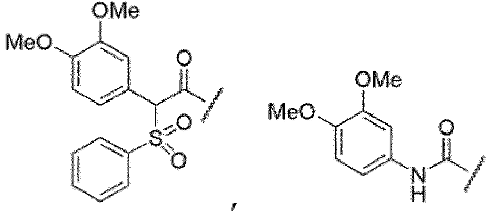 ou 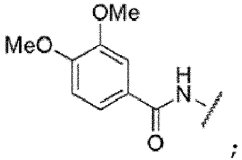 et R^{b} est -H, -OH ou un alcoxy en C₁ à C₃ ;
pour une utilisation dans la prévention ou le traitement de la rétinopathie diabétique ou de l'arthrite rhumatoïde.

2. Composé pour une utilisation selon la revendication 1, dans lequel R^{b} est - H, -OH ou un alcoxy en C₁ à C₂.

3. Composé pour une utilisation selon la revendication 1, dans lequel R^{b} est - H, -OH ou méthoxy.

4. Composé pour une utilisation dans la prévention ou le traitement de la rétinopathie diabétique ou de l'arthrite rhumatoïde, lequel composé est choisi dans le groupe constitué par les composés suivants :
2-(3,4-diméthoxyphényl)-1-(2,2-diméthyl-2*H*-chromén-6-yl)éthanone,
2-(3,4-diméthoxyphényl)-1-(5-méthoxy-2,2-diméthyl-2*H*-chromén-6-yl)éthanone,
2-(3,4-diméthoxyphényl-1-(5-méthoxy-2,2-diméthyl-2H-chromén-6-yl)propan-1-one,
2-(3,4-diméthoxyphényl)-1-(5-méthoxy-2,2-diméthyl-2*H*-chromén-6-yl)-2-méthylpropan-1-one,
2-(3,4-diméthoxyphényl)-1-(5-méthoxy-2,2-diméthyl-2H-chromén-6-yl)prop-2-én-1-one,
1-(3,4-diméthoxyphényl)cyclopropyl)(5-méthoxy-2,2-diméthyl-2H-chromén-6-yl)méthanone,
(S)-2-(3,4-diméthoxyphényl)-1-(5-méthoxy-2,2-diméthyl-2H-chromén-6-yl)propan-1-one,
(*R*)-2-(3,4-diméthoxyphényl)-1-(5-méthoxy-2,2-diméthyl-2*H*-chromén-6-yl)propan-1-one,
2-(3,4-diméthoxyphényl)-1-(5-hydroxy-2,2-diméthyl-2H-chromén-6-yl)-2-(phénylsulfonyl)éthanone,
(3,4-diméthoxyphényl)(2,2-diméthyl-2H-chromén-6-yl)méthanone,
(*E*)-1-(3,4-diméthoxyphényl)-3-(2,2-diméthyl-2*H*-chromén-6-yl)prop-2-én-1-one,
(E)-3-(3,4-diméthoxyphényl)-1-(5-hydroxy-2,2-diméthyl-2H-chromén-6-yl)prop-2-én-1-one,
(*E*)-3-(3,4-diméthoxyphényl)-1-(2,2-diméthyl-2*H*-chromén-6-yl)prop-2-én-1-one,
(*E*)-1-(5-hydroxy-2,2-diméthyl-2*H*-chromén-6-yl)-3-(2,4,5-triméthoxyphényl)prop-2-én-1-one,
(E)-3-(3,4-diméthoxyphényl)-1-(5-méthoxy-2,2-diméthyl-2H-chromén-6-yl)prop-2-én-1-one,
(*E*)-1-(5-méthoxy-2,2-diméthyl-2*H*-chromén-6-yl)-3-(2,4,5-triméthoxyphényl)prop-2-én-1-one,
2-(3,4-diméthoxyphényl)-*N*-(5-méthoxy-2,2-diméthyl-2*H*-chromén-6-yl)acétamide,
*N*-(3,4-diméthoxybenzyl)-2,2-diméthyl-2H-chromène-6-carboxamide,
*N*-(3,4-diméthoxyphényl)-2,2-diméthyl-2*H*-chromène-6-carboxamide,
*N*-(2,2-diméthyl-2*H*-chromén-6-yl)-3,4-diméthoxybenzamide,
(*R*)-2-(3,4-diméthoxyphényl)-1-(2,2-diméthyl-2*H*-chromén-6-yl)propan-1-one, et
(*S*)-2-(3,4-diméthoxyphényl)-1-(2,2-diméthyl-2*H*-chromén-6-yl)propan-1-one.

5. Composé représenté par la formule 2 ou son sel pharmaceutiquement acceptable : 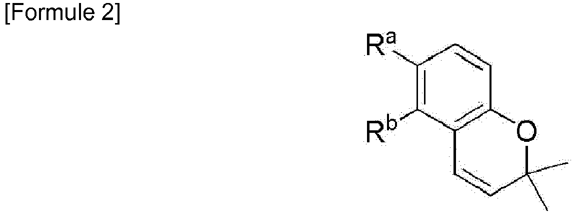 (dans la formule 2, R^{a} est 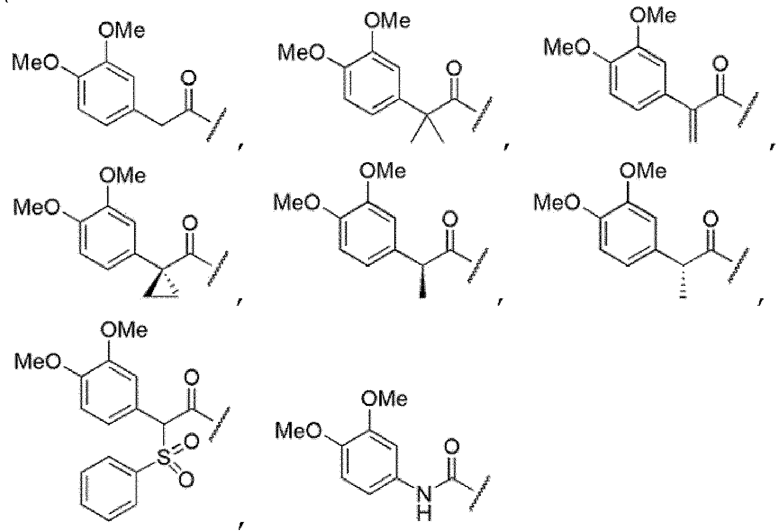 ou 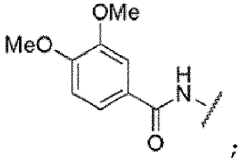 et R^{b} est un alcoxy en C₁ à C₃.

6. Composé représenté par la formule 2 ou son sel pharmaceutiquement acceptable selon la revendication 5, dans lequel R^{b} est un alcoxy en C₁ à C₂.

7. Composé représenté par la formule 2 ou son sel pharmaceutiquement acceptable selon la revendication 5, dans lequel R^{b} est méthoxy.

8. Composé ou son sel pharmaceutiquement acceptable choisi dans le groupe constitué par les composés suivants :
2-(3,4-diméthoxyphényl)-1-(5-méthoxy-2,2-diméthyl-2H-chromén-6-yl)éthanone,
2-(3,4-diméthoxyphényl-1-(5-méthoxy-2,2-diméthyl-2H-chromén-6-yl)propan-1-one,
2-(3,4-diméthoxyphényl)-1-(5-méthoxy-2,2-diméthyl-2*H*-chromén-6-yl)-2-méthylpropan-1-one,
2-(3,4-diméthoxyphényl)-1-(5-méthoxy-2,2-diméthyl-2H-chromén-6-yl)prop-2-én-1-one,
1-(3,4-diméthoxyphényl)cyclopropyl)(5-méthoxy-2,2-diméthyl-2H-chromén-6-yl)méthanone,
(S)-2-(3,4-diméthoxyphényl)-1-(5-méthoxy-2,2-diméthyl-2H-chromén-6-yl)propan-1-one,
(*R*)-2-(3,4-diméthoxyphényl)-1-(5-méthoxy-2,2-diméthyl-2*H*-chromén-6-yl)propan-1-one,
2-(3,4-diméthoxyphényl)-1-(5-hydroxy-2,2-diméthyl-2H-chromén-6-yl)-2-(phénylsulfonyl)éthanone,
(*E*)-1-(3,4-diméthoxyphényl)-3-(2,2-diméthyl-2*H*-chromén-6-yl)prop-2-én-1-one,
(*E*)-3-(3,4-diméthoxyphényl)-1-(2,2-diméthyl-2*H*-chromén-6-yl)prop-2-én-1-one,
(E)-3-(3,4-diméthoxyphényl)-1-(5-méthoxy-2,2-diméthyl-2H-chromén-6-yl)prop-2-én-1-one,
(*E*)-1-(5-méthoxy-2,2-diméthyl-2*H*-chromén-6-yl)-3-(2,4,5-triméthoxyphényl)prop-2-én-1-one,
2-(3,4-diméthoxyphényl)-*N*-(5-méthoxy-2,2-diméthyl-2*H*-chromén-6-yl)acétamide,
N-(2,2-dimethyl-2H-chromen-6-yl)-3,4-dimethoxybenzamide
(*R*)-2-(3,4-diméthoxyphényl)-1-(2,2-diméthyl-2*H*-chromén-6-yl)propan-1-one, et
(*S*)-2-(3,4-diméthoxyphényl)-1-(2,2-diméthyl-2*H*-chromén-6-yl)propan-1-one.

9. Composition pharmaceutique comprenant le composé représenté par la formule 2 de la revendication 5, ou son sel pharmaceutiquement acceptable, ou un composé selon la revendication 8, ou son sel pharmaceutiquement acceptable, en tant qu'agent actif.

10. Composé représenté par la formule 2 de la revendication 5, ou son sel pharmaceutiquement acceptable, ou composé selon la revendication 8, ou son sel pharmaceutiquement acceptable, pour une utilisation en tant que médicament.
